# EUROPEAN PATENT APPLICATION

(11) **EP 2 228 457 A1**
(43) Date of publication of application: **15.09.2010**
(21) Application number: 10161883.3
(22) Date of filing: 15.08.2008
(51) Int. Cl.: C12Q 1/68

(54) **Gene expression markers of recurrence risk in cancer patients after chemotherapy**

(30) Priority: 16.08.2007 US 956380 P; 05.09.2007 US 970188 P; 06.09.2007 US 970490 P
(62) Divisional of application: 08797949.8
(71) Applicant: Genomic Health, Inc., Redwood City, CA 94063 (US); Aventis Inc., Greenville, DE 19807 (US)
(72) Inventor: Shak, Steven, Hillsborough, CA 94010 (US); Baker, Joffre, Montara, CA 94037 (US); Yoshizawa, Carl, Redwood City, CA 94063 (US); Sparano, Joseph, Pleasantville, NY 10570 (US); Gray, Robert, Boston, MA 02115 (US)
(74) Representative: Tollett, Ian

(57) **Abstract**

The present invention relates to genes, the expression levels of which are correlated with likelihood of breast cancer recurrence in patients after tumor resection and chemotherapy.

## Description

### Field of the Invention

The present invention relates to genes, the expression levels of which are correlated with likelihood of breast cancer recurrence in patients after tumor resection and chemotherapy.

### Background of the Invention

The prognosis for breast cancer patients varies with various clinical parameters including tumor expression of estrogen receptor and presence of tumor cells in draining lymph nodes. Although the prognosis for estrogen receptor positive (ER⁺), lymph node negative (N⁻) patients is generally good, many of these patients elect to have chemotherapy. Of the patients who do receive chemotherapy, about 50% receive anthracycline + cyclophosphamide (AC) while about 30% receive a more aggressive combination of AC + taxane (ACT). Although chemotherapy is more effective in patients who are at higher risk of recurrence without it, there is a subset of patients who experience recurrence even after chemotherapy with AC or ACT.

The prognosis for ER⁺N⁺ patients is less favorable than for ER⁺N⁻ patients. Therefore, these patients more often elect chemotherapy, with about 10% receiving AC and about 80% receiving ACT. Chemotherapy is also less effective in this ER⁺N⁺ group, in that N+ patients have higher recurrence rates than N- after chemotherapy.

In both ER⁺N⁺ and ER⁺N⁻ breast cancer patients, the ability to predict the likelihood of recurrence after standard anthracycline-based chemotherapy (residual risk) would be extremely useful. Patients shown to have high residual risk could elect an alternative therapeutic regimen. Treatment choices could include a more intensive (than standard) course of anthracycline-based chemotherapy, a different drug or drug combination, a different treatment modality, such as radiation, or no treatment at all.

Improved ability to predict residual risk would also extremely useful in carrying out clinical trials. For example, a drug developer might want to test the efficacy of a drug candidate added in combination with AC chemotherapy. In the absence of a recurrence risk prediction, a large number of patients would be required for such a trial because many of the patients enrolled would have a high likelihood of a positive outcome without the added drug. By applying a test for recurrence risk, the population enrolled in a trial can be enriched for patients having a low likelihood of a positive outcome without the added drug. This reduces the enrollment required to demonstrate the efficacy of the drug and thus reduces the time and cost of executing the trial.

### Summary of the Invention

In one aspect, the invention concerns a method of predicting the clinical outcome for a patient receiving adjuvant anthracycline-based chemotherapy and having hormone receptor positive (HR+) breast cancer, the method comprising:
assaying an expression level of at least one RNA transcript listed in Tables 4A-B, or its expression product, in a biological sample comprising cancer cells obtained from the patient; and
determining a normalized expression level of the at least one RNA transcript, or its expression product,
wherein the normalized expression level of the at least one RNA transcript listed in Table 4A, or its expression product, correlates with a decreased likelihood of a positive clinical outcome; and
wherein the normalized expression level of the at least one RNA transcript listed in Table 4B, or its expression product, correlates with an increased likelihood of a positive clinical outcome.

In another aspect, the invention concerns a method of predicting the clinical outcome for a patient receiving adjuvant anthracycline-based chemotherapy and having hormone receptor negative (HR-) breast cancer, the method comprising:
assaying an expression level of at least one RNA transcript listed in Tables 5A-B, or its expression product, in a biological sample comprising cancer cells obtained from the patient; and
determining a normalized expression level of the at least one RNA transcript, or its expression product,
wherein the normalized expression level of the at least one RNA transcript listed in Table 5A, or its expression product, correlates with a decreased likelihood of a positive clinical outcome; and
wherein the normalized expression level of the at least one RNA transcript listed in Table 5B, or its expression product, correlates with an increased likelihood of a positive clinical outcome.

In yet another aspect, the invention concerns method of predicting the clinical outcome for a patient receiving adjuvant anthracycline-based chemotherapy and having hormone receptor positive (HR+), human epidermal growth factor receptor 2 negative (HER2-) breast cancer, the method comprising:
assaying an expression level of the at least one RNA transcript listed in Tables 6A-B, or its expression product, in a biological sample comprising cancer cells obtained from the patient; and
determining a normalized expression level of the at least one RNA transcript, or its expression product,
wherein the normalized expression level of the at least one RNA transcript listed in Table 6A, or its expression product, correlates with a decreased likelihood of a positive clinical outcome; and
wherein the normalized expression level of the at least one RNA transcript listed in Table 6B, or its expression product, correlates with an increased likelihood of a positive clinical outcome.

In a further aspect, the invention concerns a method of predicting the clinical outcome for a patient receiving adjuvant anthracycline-based chemotherapy and having hormone receptor negative (HR), human epidermal growth factor receptor 2 negative (HER2-) breast cancer, the method comprising:
assaying an expression level of at least one RNA transcript listed in Tables 7A-B, or its expression product, in a biological sample comprising cancer cells obtained from the patient; and
determining a normalized expression level of the at least one RNA transcript, or its expression product,
wherein the normalized expression level of the at least one RNA transcript listed in Table 7A, or its expression product, correlates with a decreased likelihood of a positive clinical outcome; and
wherein the normalized expression level of the at least one RNA transcript listed in Table 7B, or its expression product, correlates with an increased likelihood of a positive clinical outcome.

In a still further aspect, the invention concerns a method of predicting the clinical outcome for a patient receiving adjuvant anthracycline-based chemotherapy and having hormone receptor positive (HR+), human epidermal growth factor receptor 2 positive (HER2+) breast cancer, the method comprising:
assaying an expression level of at least one RNA transcript listed in Tables 8A-B, or its expression product, in a biological sample comprising cancer cells obtained from the patient; and
determining a normalized expression level of the at least one RNA transcript, or its expression product,
wherein the normalized expression level of the at least one RNA transcript listed in Table 8A, or its expression product, correlates with a decreased likelihood of a positive clinical outcome; and
wherein the normalized expression level of the at least one RNA transcript listed in Table 8B, or its expression product, correlates with an increased likelihood of a positive clinical outcome.

The invention further concerns a method of predicting the clinical outcome for a patient receiving adjuvant anthracycline-based chemotherapy and having hormone receptor negative (HR-), human epidermal growth factor receptor 2 positive (HER2+) breast cancer, the method comprising:
assaying an expression level of at least one RNA transcript listed in Tables 9A-B, or its expression product, in a biological sample comprising cancer cells obtained from the patient; and
determining a normalized expression level of the at least one RNA transcript, or its expression product,
wherein the normalized expression level of the at least one RNA transcript listed in Table 9A, or its expression product, correlates with a decreased likelihood of a positive clinical outcome; and
wherein the normalized expression level of the at least one RNA transcript listed in Table 9B, or its expression product, correlates with an increased likelihood of a positive clinical outcome.

In yet another aspet, the invention concerns a method of predicting the likelihood that a patient having hormone receptor positive (HR+) breast cancer will exhibit a clinical benefit in response to adjuvant treatment with an anthracycline-based chemotherapy, the method comprising:
assaying a biological sample obtained from a cancer tumor of the patient for an expression level of at least one RNA transcript listed in Tables 4A-B, 6A-B, and/or 8A-B, or its expression product,
determining a normalized expression level of the at least one RNA transcript in Tables 4A-B, 6A-B, and/or 8A-B, or its expression product,
wherein the normalized expression level of the at least one RNA transcript listed in Table 4A, 6A, and/or 8A, or its expression product, positively correlates with a clinical benefit in response to treatment with an anthracycline-based chemotherapy; and
wherein the normalized expression level of the at least one RNA transcript listed in Table 4B, 6B, and/or 8B, or its expression product, negatively correlates with a clinical benefit in response to treatment with an anthracycline-based chemotherapy.

In a different aspect, the invention concerns a method of predicting the likelihood that a patient having hormone receptor negative (HR-) breast cancer will exhibit a clinical benefit in response to adjuvant treatment with an anthracycline-based chemotherapy, the method comprising:
assaying a biological sample obtained from a cancer tumor of the patient for an expression level of at least one RNA transcript listed in Tables 5A-B, 7A-B, and/or 9A-B, or its expression product,
determining a normalized expression level of the at least one RNA transcript in Tables 5A-B, 7A-B, and/or 9A-B, or its expression product,
wherein the normalized expression level of the at least one RNA transcript listed in Table 5A, 7A, and/or 9A, or its expression product, positively correlates with a clinical benefit in response to treatment with an anthracycline-based chemotherapy; and
wherein the normalized expression level of the at least one RNA transcript listed in Table 5B, 7B, and/or 9B, or its expression product, negatively correlates with a clinical benefit in response to treatment with an anthracycline-based chemotherapy.

The clinical outcome of the method of the invention may be expressed, for example, in terms of Recurrence-Free Interval (RFI), Overall Survival (OS), Disease-Free Survival (DFS), or Distant Recurrence-Free Interval (DRFI).

In one aspect, the cancer is human epidermal growth factor receptor 2 (HER2) positive breast cancer.

In one aspect, the cancer is HER2 negative breast cancer.

For all aspects of the method of the invention, determining the expression level of at least one genes may be obtained, for example, by a method of gene expression profiling. The method of gene expression profiling may be, for example, a PCR-based method or digital gene expression.

For all aspects of the invention, the patient preferably is a human.

For all aspects of the invention, the method may further comprise creating a report based on the normalized expression level. The report may further contain a prediction regarding clinical outcome and/or recurrence. The report may further contain a treatment recommendation.

For all aspects of the invention, the determination of expression levels may occur more than one time. For all aspects of the invention, the determination of expression levels may occur before the patient is subjected to any therapy.

The prediction of clinical outcome may comprise an estimate of the likelihood of a particular clinical outcome for a subject or may comprise the classification of a subject into a risk group based on the estimate.

In another aspect, the invention concerns a kit comprising a set of gene specific probes and/or primers for quantifying the expression of one or more of the genes listed in any one of Tables 1, 2, 3, 4A-B, 5A-B, 6A-B, 7A-B, 8A-B, and 9A-B by quantitative RT-PCR.

In one embodiment, the kit further comprises one or more reagents for expression of RNA from tumor samples.

In another embodiment, the kit comprises one or more containers.

In yet another embodiment, the kit comprises one or more algorithms that yield prognostic or predictive information.

In a further embodiment, one or more of the containers present in the kit comprise pre-fabricated microarrays, a buffers, nucleotide triphosphates, reverse transcriptase, DNA polymerase, RNA polymerase, probes, or primers.

In a still further embodiment, the kit comprises a label and/or a package insert with instructions for use of its components.

In a further embodiment, the instructions comprise directions for use in the prediction or prognosis of breast cancer.

The invention further comprises a method of preparing a personalized genomics profile for a patient comprising the steps of: (a) determining the normalized expression levels of the RNA transcripts or the expression products of one or more genes listed in Tables 1, 2, 3, 4A-B, 5A-B, 6A-B, 7A-B, 8A-B, and 9A-B, in a cancer cell obtained from the patient; and (b) creating a report summarizing the data obtained by said gene expression analysis.

The method may further comprise the step of communicating the report to the patient or a physician of the patient.

The invention further concerns a report comprises the results of the gene expression analysis performed as described in any of the aspects and embodiments described above.

### Brief Description of the Drawings

Figure 1: E2197 Main Study Results - Disease-Free Survival
Figure 2: E2197 Main Study Results Overall Survival

### Detailed Description of the Invention

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

A "biological sample" encompasses a variety of sample types obtained from an individual. The definition encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., nucleic acids, polypeptides, etc. The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like. A "biological sample" includes a sample obtained from a patient's cancer cell, e.g., a sample comprising polynucleotides and/or polypeptides that is obtained from a patient's cancer cell (e.g., a cell lysate or other cell extract comprising polynucleotides and/or polypeptides); and a sample comprising cancer cells from a patient. A biological sample comprising a cancer cell from a patient can also include non-cancerous cells.

The terms "cancer," "neoplasm," and "tumor" are used interchangeably herein to refer to the physiological condition in mammal cells that is typically characterized by an aberrant growth phenotype and a significant loss of control of cell proliferation. In general, cells of interest for detection, analysis, classification, or treatment in the present application include precancerous (*e*.*g*., benign), malignant, pre-metastatic, metastatic, and non-metastatic cells.

The term "hormone receptor positive (IIR+) tumors" means tumors expressing either estrogen receptor (ER) or progesterone receptor (PR) as determined by standard methods (e.g., immunohistochemical staining of nuclei in the patients biological samples). The term "hormone receptor negative (HR-) tumors" means tumors expressing neither estrogen receptor (ER) nor progesterone receptor (PR) as determined by standard methods, including immunohistochemical staining. Such methods of immunohistochemical staining are routine and known to one of skill in the art.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

The term "prognosis" is used herein to refer to the prediction of the likelihood of cancer-attributable death or progression, including recurrence, metastatic spread, and drug resistance, of a neoplastic disease, such as breast cancer.

Prognostic factors are those variables related to the natural history of breast cancer, which influence the recurrence rates and outcome of patients once they have developed breast cancer. Clinical parameters that have been associated with a worse prognosis include, for example, lymph node involvement, and high grade tumors. Prognostic factors are frequently used to categorize patients into subgroups with different baseline recurrence risks.

The term "prediction" is used herein to refer to the likelihood that a patient will have a particular clinical outcome, whether positive or negative, following surgical removal of the primary tumor and treatment with anthracycline-based chemotherapy. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as chemotherapy or surgical intervention.

"Positive patient response" or "positive clinical outcome" can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of at least one symptoms associated with the tumor; (8) increase in the length of survival following treatment; and/or (9) decreased mortality at a given point of time following treatment. The term "positive clinical outcome" means an improvement in any measure of patient status, including those measures ordinarily used in the art, such as an increase in the duration of Recurrence-Free interval (RFI), an increase in the time of Overall Survival (OS), an increase in the time of Disease-Free Survival (DFS), an increase in the duration of Distant Recurrence-Free Interval (DRFI), and the like. An increase in the likelihood of positive clinical outcome corresponds to a decrease in the likelihood of cancer recurrence.

The term "residual risk" except when specified otherwise is used herein to refer to the probability or risk of cancer recurrence in breast cancer patients after surgical resection of their tumor and treatment with anthracycline-based chemotherapies.

The term "anthracycline-based chemotherapies" is used herein to refer to chemotherapies that comprise an anthracycline compound, for example doxorubicin, daunorubicin, epirubicin or idarubicin. Such anthracycline based chemotherapies may be combined with other chemotherapeutic compounds to form combination chemotherapies such as, without limitation, anthracycline + cyclophosphamide (AC), anthracycline + taxane (AT), or anthracycline +cyclophosphamide +taxane (ACT).

The term "long-term" survival is used herein to refer to survival for at least 3 years, more preferably for at least 5 years.

The term "Recurrence-Free Interval (RFI)" is used herein to refer to time in years to first breast cancer recurrence censoring for second primary cancer or death without evidence of recurrence.

The term "Overall Survival (OS)" is used herein to refer to time in years from surgery to death from any cause.

The term "Disease-Free Survival (DFS)" is used herein to refer to time in years to breast cancer recurrence or death from any cause.

The term "Distant Recurrence-Free Interval (DRFI)" is used herein to refer to the time (in years) from surgery to the first anatomically distant cancer recurrence, censoring for second primary cancer or death without evidence of recurrence.

The calculation of the measures listed above in practice may vary from study to study depending on the definition of events to be either censored or not considered.

The term "subject" or "patient" refers to a mammal being treated. In an embodiment the mammal is a human.

The term "microarray" refers to an ordered arrangement of hybridizable array elements, preferably polynucleotide probes, on a substrate.

The terms "gene product" and "expression product" are used interchangeably herein in reference to a gene, to refer to the RNA transcription products (transcripts) of the gene, including mRNA and the polypeptide translation products of such RNA transcripts, whether such product is modified post-translationally or not. The terms "gene product" and "expression product" are used interchangeably herein, in reference to an RNA, particularly an mRNA, to refer to the polypeptide translation products of such RNA, whether such product is modified post-translationally or not. A gene product can be, for example, an unspliced RNA, an mRNA, a splice variant mRNA, a polypeptide, a post-translationally modified polypeptide, a splice variant polypeptide, etc.

As used herein, the term "normalized expression level" refers to an expression level of a response indicator gene relative to the level of an expression product of a reference gene(s).

The term "polynucleotide," when used in singular or plural, generally refers to any polyribonucleotide or polydeoxyribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of at least one of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain at least one modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

The terms "differentially expressed gene," "differential gene expression" and their synonyms, which arc used interchangeably, refer to a gene whose expression is activated to a higher or lower level in a subject suffering from a disease, specifically cancer, such as breast cancer, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically cancer, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about two-fold, preferably at least about four-fold, more preferably at least about six-fold, most preferably at least about ten-fold difference between the expression of a given gene in normal and diseased subjects, or in various stages of disease development in a diseased subject.

The term "over-expression" with regard to an RNA transcript is used to refer to the level of the transcript determined by normalization to the level of reference mRNAs, which might be all measured transcripts in the specimen or a particular reference set of mRNAs such as housekeeping genes. The assay typically measures and incorporates the expression of certain normalizing genes, including well known housekeeping genes, such as GAPDH and Cyp1. Alternatively, normalization can be based on the mean or median signal (Ct) of all of the assayed genes or a large subset thereof (global normalization approach). On a gene-by-gene basis, measured normalized amount of a patient tumor mRNA is compared to the amount found in a cancer tissue reference set. The number (N) of cancer tissues in this reference set should be sufficiently high to ensure that different reference sets (as a whole) behave essentially the same way. If this condition is met, the identity of the individual cancer tissues present in a particular set will have no significant impact on the relative amounts of the genes assayed. Usually, the cancer tissue reference set consists of at least about 30, preferably at least about 40 different FPE cancer tissue specimens.

As used herein, "gene expression profiling" refers to research methods that measure mRNA made from many different genes in various cell types. For example, this method may be used to monitor the expression of thousands of genes simultaneously using microarray technology. Gene expression profiling may be used as a diagnostic test to help identify subgroups of tumor types, to help predict which patients may respond to treatment, and which patients may be at increased risk for cancer relapse.

The phrase "gene amplification" refers to a process by which multiple copies of a gene or gene fragment are formed in a particular cell or cell line. The duplicated region (a stretch of amplified DNA) is often referred to as "amplicon." Usually, the amount of the messenger RNA (mRNA) produced, i.e., the level of gene expression, also increases in the proportion of the number of copies made of the particular gene expressed.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The term "node negative" cancer, such as "node negative" breast cancer, is used herein to refer to cancer that has not spread to the lymph nodes.

The terms "splicing" and "RNA splicing" are used interchangeably and refer to RNA processing that removes introns and joins exons to produce mature mRNA with continuous coding sequence that moves into the cytoplasm of an eukaryotic cell.

In theory, the term "exon" refers to any segment of an interrupted gene that is represented in the mature RNA product (B. Lewin. Genes IV Cell Press, Cambridge Mass. 1990). In theory the term "intron" refers to any segment of DNA that is transcribed but removed from within the transcript by splicing together the exons on either side of it. Operationally, exon sequences occur in the mRNA sequence of a gene as defined by Ref.Seq ID numbers on the Entrez Gene database maintained by the National Center for Biotechnology Information. Operationally, intron sequences are the intervening sequences within the genomic DNA of a gene, bracketed by exon sequences and having GT and AG splice consensus sequences at their 5' and 3' boundaries.

The term "expression cluster" is used herein to refer to a group of genes which demonstrate similar expression patterns when studied within samples from a defined set of patients. As used herein, the genes within an expression cluster show similar expression patterns when studied within samples from patients with invasive breast cancer.

The terms "correlate" and "correlation" refer to the simultaneous change in value of two numerically valued variables. For example, correlation may indicate the strength and direction of a linear relationship between two variables indicating that they are not independent. The correlation between the two such variables could be positive or negative.

### B.1 General Description of the Invention

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

Disruptions in the normal functioning of various physiological processes, including proliferation, apoptosis, angiogenesis and invasion, have been implicated in the pathology in cancer. The relative contribution of dysfunctions in particular physiological processes to the pathology of particular cancer types is not well characterized. Any physiological process integrates the contributions of numerous gene products expressed by the various cells involved in the process. For example, tumor cell invasion of adjacent normal tissue and intravasation of the tumor cell into the circulatory system are effected by an array of proteins that mediate various cellular characteristics, including cohesion among tumor cells, adhesion of tumor cells to normal cells and connective tissue, ability of the tumor cell first to alter its morphology and then to migrate through surrounding tissues, and ability of the tumor cell to degrade surrounding connective tissue structures.

Multi-analyte gene expression tests can measure the expression level of at least one genes involved in each of several relevant physiologic processes or component cellular characteristics. In some instances the predictive power of the test, and therefore its utility, can be improved by using the expression values obtained for individual genes to calculate a score which is more highly associated with outcome than is the expression value of the individual genes. For example, the calculation of a quantitative score (recurrence score) that predicts the likelihood of recurrence in estrogen receptor-positive, node-negative breast cancer is describe in U.S. Publication No. 20050048542, published March 3, 2005, the entire disclosure of which is expressly incorporated by reference herein. The equation used to calculate such a recurrence score may group genes in order to maximize the predictive value of the recurrence score. The grouping of genes may be performed at least in part based on knowledge of their contribution to physiologic functions or component cellular characteristics such as discussed above. The formation of groups, in addition, can facilitate the mathematical weighting of the contribution of various expression values to the recurrence score. The weighting of a gene group representing a physiological process or component cellular characteristic can reflect the contribution of that process or characteristic to the pathology of the cancer and clinical outcome. Accordingly, in an important aspect, the present invention also provides specific groups of the prognostic genes identified herein, that together are more reliable and powerful predictors of outcome than the individual genes or random combinations of the genes identified.

Measurement of prognostic RNA transcript expression levels may be performed by using a software program executed by a suitable processor. Suitable software and processors are well known in the art and are commercially available. The program may be embodied in software stored on a tangible medium such as CD-ROM, a floppy disk, a hard drive, a DVD, or a memory associated with the processor, but persons of ordinary skill in the art will readily appreciate that the entire program or parts thereof could alternatively be executed by a device other than a processor, and/or embodied in firmware and/or dedicated hardware in a well known manner.

Following the measurement of the expression levels of the genes identified herein, or their expression products, and the determination that a subject is likely or not likely to respond to treatment with an anthracycline-based chemotherapy (e.g., anthracycline + cyclophosphamide (AC) or AC + taxane (ACT)), the assay results, findings, diagnoses, predictions and/or treatment recommendations are typically recorded and communicated to technicians, physicians and/or patients, for example. In certain embodiments, computers will be used to communicate such information to interested parties, such as, patients and/or the attending physicians. In some embodiments, the assays will be performed or the assay results analyzed in a country or jurisdiction which differs from the country or jurisdiction to which the results or diagnoses arc communicated.

In a preferred embodiment, a diagnosis, prediction and/or treatment recommendation based on the expression level in a test subject of at least one of the biomarkers herein is communicated to the subject as soon as possible after the assay is completed and the diagnosis and/or prediction is generated. The results and/or related information may be communicated to the subject by the subject's treating physician. Alternatively, the results may be communic ated directly to a test subject by any means of communication, including writing, electronic forms of communication, such as email, or telephone. Communication may be facilitated by use of a computer, such as in case of email communications. In certain embodiments, the communication containing results of a diagnostic test and/or conclusions drawn from and/or treatment recommendations based on the test, may be generated and delivered automatically to the subject using a combination of computer hardware and software which will be familiar to artisans skilled in telecommunications. One example of a healthcare-oriented communications system is described in U.S. Pat. No. 6,283,761; however, the present invention is not limited to methods which utilize this particular communications system. In certain embodiments of the methods of the invention, all or some of the method steps, including the assaying of samples, diagnosing of diseases, and communicating of assay results or diagnoses, may be carried out in diverse (e.g., foreign) jurisdictions.

The utility of a marker in predicting recurrence risk may not be unique to that marker. An alternative gene having expression values that are closely correlated with those of a known gene marker may be substituted for or used in addition to the known marker and have little impact on the overall predictive utility of the test. The correlated expression pattern of the two genes may result from involvement of both genes in a particular process and/or being under common regulatory control in breast tumor cells. The present invention specifically includes and contemplates the use of at least one such substitute genes in the methods of the present invention.

The markers of recurrence risk in breast cancer patients provided by the present invention have utility in the choice of treatment for patients diagnosed with breast cancer. While the rate of recurrence in early stage breast cancer is relatively low compared to recurrence rates in some other types of cancer, there is a subpopulation of these patients who have a relatively high recurrence rate (poor prognosis) if not treated with chemotherapy in addition to surgical resection of their tumors. Among these patients with poor prognosis are a smaller number of individuals who are unlikely to respond to chemotherapy, for example AC or ACT. The methods of this invention are useful for the identification of individuals with poor initial prognosis and low likelihood of response to standard chemotherapy which, taken together, result in high recurrence risk. In the absence of a recurrence risk prediction, these patients would likely receive and often fail to benefit from standard chemotherapy treatment. With an accurate test for prediction of recurrence risk, these patients may elect alternative treatment to standard chemotherapy and in doing so avoid the toxicity of standard chemotherapy and unnecessary delay in availing themselves of what may be a more effective treatment.

The markers and associated information provided by the present invention for predicting recurrence risk in breast cancer patients also have utility in screening patients for inclusion in clinical trials that test the efficacy of drug compounds. Experimental chemotherapy drugs are often tested in clinical trials by testing the experimental drug in combination with standard chemotherapeutic drugs and comparing the results achieved in this treatment group with the results achieved using standard chemotherapy alone. The presence in the trial of a significant subpopulation of patients who respond to the experimental treatment because it includes standard chemotherapy drugs already proven to be effective complicates the identification of patients who are responsive to the experimental drug and increases the number of patients that must be enrolled in the clinical trial to optimize the likelihood of demonstrating the efficacy of the experimental drug. A more efficient clinical trial could be designed if patients having a high degree of recurrence risk could be identified. The markers of this invention are useful for developing such a recurrence risk test, such that high recurrence risk could be used as an inclusion criteria for clinical trial enrollment.

In a particular embodiment, prognostic markers and associated information are used to design or produce a reagent that modulates the level or activity of the gene's transcript or its expression product. Said reagents may include but are not limited to an antisense RNA, a small inhibitory RNA, micro RNA, a ribozyme, a monoclonal or polyclonal antibody.

In various embodiments of the inventions, various technological approaches arc available for determination of expression levels of the disclosed genes, including, without limitation, RT-PCR, microarrays, serial analysis of gene expression (SAGE) and Gene Expression Analysis by Massively Parallel Signature Sequencing (MPSS), which will be discussed in detail below. In particular embodiments, the expression level of each gene may be determined in relation to various features of the expression products of the gene including exons, introns, protein epitopes and protein activity. In other embodiments, the expression level of a gene may be inferred from analysis of the structure of the gene, for example from the analysis of the methylation pattern of the gene's promoter(s).

### B.2 Gene Expression Profiling

Methods of gene expression profiling include methods based on hybridization analysis of polynucleotides, methods based on sequencing of polynucleotides, and proteomies-based methods. The most commonly used methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize sequence-specific duplexes, including DNA duplexes, RNA duplexes, and DNA RNA hybrid duplexes or DNA protein duplexes. Representative methods for sequencing-based gene expression analysis include Serial Analysis of Gene Expression (SAGE), and gene expression analysis by massively parallel signature sequencing (MPSS).

### a. Reverse Transcriptase PCR

Of the techniques listed above, the most sensitive and most flexible quantitative method is quantitative real time polymerase chain reaction (qRf-PCR), which can be used to determine mRNA levels in various samples. The results can be used to compare gene expression patterns between sample sets, for example in normal and tumor tissues or in patients with or without drug treatment.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE^{®}, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non extendible by Taq DNA polymerase enzyme, and is labeled with a reporter fluorescent dye and a quencher fluorescent dye. Any laser induced emission from the reporter dye is quenched by the quenching dye when the two dyes are located close together as they are on the probe. During the amplification reaction, the Taq DNA polymerase enzyme cleaves the probe in a template dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700TM Sequence Detection SystemTM (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700TM Sequence Detection SystemTM. The system consists of a thermocycler, laser, charge coupled device (CCD), camera and computer. The system amplifies samples in a 96 well format on a thermocycler. During amplification, laser induced fluorescent signal is collected in real time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for profiling gene expression using fixed, paraffin-embedded tissues as the RNA source, including mRNA isolation, purification, primer extension and amplification are given in various published journal articles (for example: T.E. Godfrey et al. J. Molec. Diagnostics 2: 84-91 (2000); K. Specht et al., Am. J. Pathol. 158: 419-29 (2001)). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR.

### b. MassARRAY System

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derived PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### c. Other PCR-based Methods

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### d. Microarrays

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology.

The development of microarray methods for large-scale analysis of gene expression makes it possible to search systematically for molecular markers of outcome predictions for a variety of chemotherapy treatments for a variety of tumor types.

### e. Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### f. Gene Expression Analysis by Massively Parallel Signature Sequencing (MPSS)

This method, described by Brenner el al., Nature Biotechnology 18:630-634 (2000), is a sequencing approach that combines non-gel-based signature sequencing *with in vitro* cloning of millions of templates on separate 5 µm diameter microbeads. First, a microbead library of DNA templates is constructed by *in vitro* cloning. This is followed by the assembly of a planar array of the template-containing microbeads in a flow cell at a high density (typically greater than 3 x 10⁶ microbeads/cm²). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

### g. Immunohistochemistry

Immunohistochemistry methods are also suitable for detecting the expression levels of the prognostic markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

### h. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. by mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

### i. Chromatin Structure Analysis

A number of methods for quantization of RNA transcripts (gene expression analysis) or their protein translation products are discussed herein. The expression level of genes may also be inferred from information regarding chromatin structure, such as for example the methylation status of gene promoters and other regulatory elements and the acetylation status of histones.

In particular, the methylation status of a promoter influences the level of expression of the gene regulated by that promoter. Aberrant methylation of particular gene promoters has been implicated in expression regulation, such as for example silencing of tumor suppressor genes. Thus, examination of the methylation status of a gene's promoter can be utilized as a surrogate for direct quantization of RNA levels.

Several approaches for measuring the methylation status of particular DNA elements have been devised, including methylation-specific PCR (Herman J.G. et al. (1996) Methylation-specific PCR: a novel PCR assay for methylation status of CpG islands. Proc. Natl Acad. Sci. USA. 93, 9821-9826.) and bisulfite DNA sequencing (Frommer M. et al. (1992) A genomic sequencing protocol that yields a positive display of 5-methylcytosine residues in individual DNA strands. Proc. Natl Acad. Sci. USA. 89, 1827-1831.). More recently, microarray-based technologies have been used to characterize promoter methylation status (Chen C.M. (2003) Methylation target array for rapid analysis of CpG island hypermethylation in multiple tissue genomes. Am. J. Pathol. 163, 37-45.).

### j. General Description of the mRNA Isolation, Purification and Amplification

The steps of a representative protocol for profiling gene expression using fixed, paraffin-embedded tissues as the RNA source, including mRNA isolation, purification, primer extension and amplification are provided in various published journal articles (for example: T.E. Godfrey et al,. J. Molec. Diagnostics 2: 84-91 (2000); K. Specht et al., Am. J. Pathol. 158: 419-29 (2001)). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and the RNA is reverse transcribed using gene specific promoters followed by RT-PCR. Finally, the data are analyzed to identify the best treatment option(s) available to the patient on the basis of the characteristic gene expression pattern identified in the tumor sample examined, dependent on the predicted likelihood of cancer recurrence.

### k. Breast Cancer Gene Set, Assayed Gene Subsequences, and Clinical Application of Gene Expression Data

An important aspect of the present invention is to use the measured expression of certain genes by breast cancer tissue to provide prognostic information. For this purpose it is necessary to correct for (normalize away) both differences in the amount of RNA assayed and variability in the quality of the RNA used. Therefore, the assay typically measures and incorporates the expression of certain normalizing genes, including well known housekeeping genes, such as GAPDH and Cyp1. Alternatively, normalization can be based on the mean or median signal (Ct) of all of the assayed genes or a large subset thereof (global normalization approach). On a gene-by-gene basis, measured normalized amount of a patient tumor mRNA is compared to the amount found in a breast cancer tissue reference set. The number (N) of breast cancer tissues in this reference set should be sufficiently high to ensure that different reference sets (as a whole) behave essentially the same way. If this condition is met, the identity of the individual breast cancer tissues present in a particular set will have no significant impact on the relative amounts of the genes assayed. Usually, the breast cancer tissue reference set consists of at least about 30, preferably at least about 40 different FPE breast cancer tissue specimens. Unless noted otherwise, normalized expression levels for each mRNA/tested tumor/patient will be expressed as a percentage of the expression level measured in the reference set. More specifically, the reference set of a sufficiently high number (e.g. 40) of tumors yields a distribution of normalized levels of each mRNA species. The level measured in a particular tumor sample to be analyzed falls at some percentile within this range, which can be determined by methods well known in the art. Below, unless noted otherwise, reference to expression levels of a gene assume normalized expression relative to the reference set although this is not always explicitly stated.

### /. Design of Intron-Based PCR Primers and Probes

According to one aspect of the present invention, PCR primers and probes are designed based upon intron sequences present in the gene to be amplified. Accordingly, the first step in the primer/probe design is the delineation of intron sequences within the genes. This can be done by publicly available software, such as the DNA BLAT software developed by Kent, W.J., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386).

The most important factors considered in PCR primer design include primer length, melting temperature (Tm), and G/C content, specificity, complementary primer sequences, and 3'-end sequence. In general, optimal PCR primers arc generally 17-30 bases in length, and contain about 20-80%, such as, for example, about 50-60% G+C bases. Tm's between 50 and 80 °C, e.g. about 50 to 70 °C are typically preferred.

For further guidelines for PCR primer and probe design see, e.g. Dieffenbach, C.W. et al., "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

### m. Kits of the Invention

The materials for use in the methods of the present invention are suited for preparation of kits produced in accordance with well known procedures. The invention thus provides kits comprising agents, which may include gene-specific or gene-selective probes and/or primers, for quantitating the expression of the disclosed genes for predicting prognostic outcome or response to treatment. Such kits may optionally contain reagents for the extraction of RNA from tumor samples, in particular fixed paraffin-embedded tissue samples and/or reagents for RNA amplification. In addition, the kits may optionally comprise the reagent(s) with an identifying description or label or instructions relating to their use in the methods of the present invention. The kits may comprise containers (including microtiter plates suitable for use in an automated implementation of the method), each with at least one of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and at least one probes and primers of the present invention (e.g., appropriate length poly(T) or random primers linked to a promoter reactive with the RNA polymerase). Mathematical algorithms used to estimate or quantify prognostic or predictive information are also properly potential components of kits.

The methods provided by the present invention may also be automated in whole or in part.

### n. Reports of the Intention

The methods of the present invention are suited for the preparation of reports summarizing the predictions resulting from the methods of the present invention. The invention thus provides for methods of creating reports and the reports resulting therefrom. The report may include a summary of the expression levels of the RNA transcripts or the expression products for certain genes in the cells obtained from the patients tumor tissue. The report may include a prediction that said subject has an increased likelihood of response to treatment with a particular chemotherapy or the report may include a prediction that the subject has a decreased likelihood of response to the chemotherapy. The report may include a recommendation for treatment modality such as surgery alone or surgery in combination with chemotherapy. The report may be presented in electronic format or on paper.

All aspects of the present invention may also be practiced such that a limited number of additional genes that are co-expressed with the disclosed genes, for example as evidenced by high Pearson correlation coefficients, are included in a prognostic or predictive test in addition to and/or in place of disclosed genes.

Having described the invention, the same will be more readily understood through reference to the following Example, which is provided by way of illustration, and is not intended to limit the invention in any way.

### EXAMPLE 1:

### Identifying Genomic Predictors of Recurrence After Adjuvant Chemotherapy

Clinical specimens were obtained from patients with operable breast cancer enrolled in clinical trial E2197 conducted by the East Coast Oncology Cooperative Group (ECOG). Goldstein and colleagues for ECOG and the North American Breast Cancer Intergroup reported the results of E2197 at ASCO 2005. (Goldstein, L.J., O'Neill, A., Sparano, J.A., Perez, E.A., Schulman, L.N., Martino, S., Davidson, N.E.: E2197: Phase III AT (doxorubucin/docetaxel) vs. AC (doxorubucin/cyclophosphamide) in the Adjuvant Treatment of Node Positive and High Risk Node Negative Breast Cancer [abstract]. Proceedings of ASCO 2005)

The expression level of each of 371 genes, including five reference genes, was determined in tumor samples obtained from breast cancer patients prior to surgical resection of the tumor and treatment of the patients with either AC or AT chemotherapy. Outcome data was available for these patients so that associations between gene expression values and outcome could be established. To form the sample for this project, the E2197 cohort was divided into 8 strata defined by hormone receptor (HR) status (estrogen receptor (ER) or progesterone receptor (PR) positive vs. both negative), axillary nodal status (positive vs. negative), and treatment arm (AT vs. AC). Within each stratum, a sub-sample was created including all recurrences with suitable tissue available and a random sample of the non-recurrences containing approximately 3.5 times as many subjects as the recurrence group.

The primary objective of the study presented in this example was to identify individual genes whose RNA expression is associated with an increased risk of recurrence of breast cancer (including all cases and controls in both AC and AT arms).

Nucleic acid from cancer cells from the patients was analyzed to measure the expression level of a test gene(s) and a reference gene(s). The expression level of the test gene(s) was then normalized to the expression level of the reference gene(s), thereby generating a normalized expression level (a "normalized expression value") of the test gene. Normalization was carried out to correct for variation in the absolute level of gene product in a cancer cell. The cycle threshold measurement (Ct) was on a log base 2 scale, thus every unit of Ct represents a two-fold difference in gene expression.

Finally, statistical correlations were made between normalized expression values of each gene and at least one measures of clinical outcome following resection and anthraeycline-based chemotherapy treatment that reflect a likelihood of (a) increased risk of recurrence of breast cancer; and (b) beneficial effect of anthracycline-based chemotherapy.

### Comparative use of AC vs. AT does not significantly affect outcome

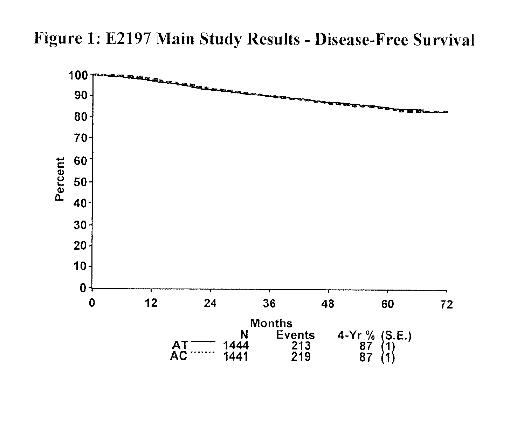

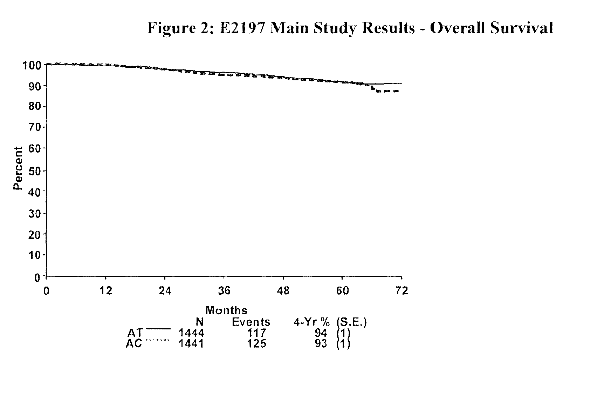

The results of the original E2197 study outlined that there is no significant difference in outcome between AC versus AT arms with regard to disease free and overall survival. See Table 1 below and Figures 1-2. Therefore, data from these treatment arms was combined for statistical analysis to identify prognostic genes.

**Table 1: Results of E2197**

| | **AC q 3wks x 4 (n=1441)** | **AT q 3wks x 4 (n=1444)** |
|---|---|---|
| 4 year DFS | 87% | 87% |
| 4 year OS | 94% | 93% |

| | | |
|---|---|---|
| Abbreviations: AC - doxorubicin 60 mg/m², cyclophosphamide 600 mg/m²; AT - doxorubicin 60 mg/m², docetaxel 60 mg/m²; DFS - disease free survival; OSO - overall survival | | |

### Genes associated with clinical outcome

Methods to predict the likelihood of recurrence in patients with invasive breast cancer treated with non-anthracycline-based treatment (e.g., tamoxifen) can be found, for example, in U.S. Patent No. 7,056,674 and U.S. Application Publication No. 20060286565, published December 21. 2006, the entire disclosures of which are expressly incorporated by reference herein.

### Inclusion and exclusion criteria

Samples were obtained from a subset of patients enrolled in clinical trial E2197 conducted by the East Coast Oncology Cooperative (ECOG). Goldstein and colleagues for the Eastern Cooperative Oncology Group (ECOG) and the North American Breast Cancer Intergroup reported the results of E2197 at ASCO 2005 (Goldstein, L.J., O'Neill, A., Sparano, J.A.. Perez, E.A., Schulman, L.N., Martino, S., Davidson, N.E.: E2197: Phase III AT (doxorubucin/docetaxel) vs. AC (doxorubucin/cyclophosphamide) in the Adjuvant Treatment of Node Positive and High Risk Node Negative Breast Cancer [abstract]. Proceedings of ASCO 2005. Abstract 512.). Genomic data was collected from 776 patients from the E2197 trial. Inclusion and exclusion criteria for the studies presented herein were as follows:

### Inclusion Criteria

■ Tumor samples from patients enrolled on E2197 and who meet the other eligibility criteria specific below.
■ Adequate tumor material available in ECOG Pathology Coordinating Center.
■ Patient previously consented to future cancer-related research.
■ Meet criteria for case and control selection outlined in statistical section.

### Exclusion Criteria

■ A patient that was not enrolled in E2197.
■ No patient sample available in the ECOG Pathology Archive
■ Insufficient RNA (<642 ng ) for the RT-PCR analysis.
■ Average non-normalized C_{T} for the 5 reference genes > 35.

### Probes and primers

For each sample included in the study, the expression level for each gene listed in Table 1 was assayed by qRT-PCR as previously described in Paik et al. N. Engl. J. Med. 351: 2817-2826 (2004). Probe and primer sequences utilized in qRT-PCR assays are also provided in Table 1. Sequences for the amplicons that result from the use of the primers given in Table 2 are listed in Table 3.

### Identification of genes that are indicators of clinical outcome

Statistical analyses were carried out using tumor samples from patients enrolled in the E2197 study who met the inclusion criteria. The patient samples were classified based on estrogen receptor (ER) expression (positive, negative), progesterone receptor (PR) expression (positive, negative), and human epidermal growth factor receptor 2 (HER2) expression (negative [0, 1+], weakly positive [2+], or positive [3+]) (Herceptest™, Dako USA, Carpintcria). The cut points for ER, PR, and HER2 positivity were 6.5, 5.5 and 11.5, respectively. For example, samples having a normalized ER expression of >6.5Ct were classified as ER+. These quantitative RT-PCR (e.g., qRT-PCR as described in U.S. Application Publ. No. 20050095634) cut points were established in reference to three independent prior determinations of ER, PR and HER2 expression as determined by immunohistochemistry. Tumors testing positive for either ER or PR were classified as hormone receptor positive (HR+). Because there was no significant difference between the two chemotherapy treatments (AC, AT) in the E2197 study, data from these two treatment arms were combined for this statistical analysis.

Recurrence Free Interval is defined as the time from study entry to the first evidence of breast cancer recurrence, defined as invasive breast cancer in local, regional or distant sites, including the ipsilateral breast, but excluding new primary breast cancers in the opposite breast. Follow-up for recurrence was censored at the time of death without recurrence, new primary cancer in the opposite breast, or at the time of the patient was last evaluated for recurrence.

Raw expression data expressed as C_{T} values were normalized using GAPDH, GUS, TFRC, Beta-actin, and RPLP0 as reference genes. Further analysis to identify statistically meaningful associations between expression levels of particular genes or gene sets and particular clinical outcomes was carried out using the normalized expression values.

### Example Analysis 1

A statistical analysis was performed using Univariate Cox Regression models (SAS version 9.1.3). When examining the relationship between Recurrence-Free Interval and the expression level of individual genes, the expression levels were treated as continuous variables. Follow-up for recurrence was censored at the time of death without recurrence, new primary cancer in the opposite breast, or at the time of the patient was last evaluated for recurrence. All hypothesis tests were reported using two-sided p-values, and p-values of < 0.05 was considered statistically significant.

To form the sample for this project, the E2197 cohort was divided into 8 strata defined hormone status (ER or PR positive vs. both negative) using local IIIC, axillary nodal status (positive vs. negative) and treatment arm (AT vs. AC). Within each stratum, a sub-sample was created including all recurrences with suitable tissue available and a random sample of the non-recurrences containing approximately 3.5 times as many subjects as the recurrence groups.

Sampling weights for each of the 16 groups in the case-control sample are defined by the number of patients in the E2197 study in that group divided by the number in the sample. In the weighted analyses, contributions to estimators and other quantities, such as partial likelihoods, are multiplied by these weights. If the patients included in the case-control sample are a random subset of the corresponding group from E2197, then the weighted estimators give consistent estimates of the corresponding quantities from the full E2197 sample. The weighted partial likelihood computed in this fashion is used for estimating hazard ratios and testing effects. This essentially gives the weighted pseudo-likelihood estimator of Chen and Lo. (K. Chen, S.H. Lo, Biometrika, 86:755-764 (1999)) The primary test for the effect of gene expression on recurrence risk was pre-specified as the weighted partial likelihood Wald test. The variance of the partial likelihood estimators is estimated using the general approach of Lin (D.Y. Lin, Biometrika, 87:37-47 (2000)), which leads to a generalization of the variance estimator from Borgan et. al. to allow subsampling of cases. (Borgan et al., Lifetime Data Analysis, 6:39-58 (2000)).

### Example Analysis 2

Statistical analyses were performed by Univariate Cox proportional hazards regression models, using stratum-specific sampling weights to calculate weighted partial likelihoods, to estimate hazard ratios, and an adjusted variance estimate was used to calculate confidence intervals and perform hypothesis tests. When examining the relationship between Recurrence-Free Interval and the expression level of individual genes, the expression levels were treated as continuous variables. All hypothesis tests were reported using the approach of Korn et al. that is used to address the multiple testing issue within each population providing strong control of the number of false discoveries. (E. L. Korn, et al., Journal of Statistical Planning and Inference, Vol. 124(2):379-398 (Sept. 2004)) The adjusted p-values give the level of confidence that the false discovery proportion (FDP) is less than or equal to 10%, in the sense that the p-value is the proportion of experiments where the true FDP is expected to exceed the stated rate. If genes with adjusted p-values < α are selected as significant, then the chance (in an average sense over replicate experiments) that the number of false discoveries is greater than the specified number is < α. In this algorithm, 500 permutations are used. For each permutation, the subject label of the gene expression levels is randomly permuted relative to the other data.

Sampling weights for each of the 16 groups in the case-control sample are defined by the number of patients in E2197 study in that group divided by the number in the sample. In the weighted analyses, contributions to estimators and other quantities, such as partial likelihoods are multiplied by these weights. (R. Gray, Lifetime Data Analysis, 9:123-138 (2003)). If the patients included in the case-control sample are a random subset of the corresponding group from E2197, then the weighted estimators give consistent estimates of the corresponding quantities from the full E2197 sample. The weighted partial likelihood computed in this fashion is used for estimating hazard ratios and testing effects. This essentially gives the weighted pseudo-likelihood estimator of Chen and Lo. (K. Chen, S.H. Lo, Biometrika, 86:755-764 (1999))

Weighted Kaplan-Meier estimators are used to estimate unadjusted survival plots and unadjusted event-free rates. The Cox proportional hazards regression model may be used to estimate covariate-adjusted survival plots and event-free rates. The empirical cumulative hazard estimate of survival, rather than the Kaplan-Meier product limit estimate, may be employed for these analyses with the Cox model.

Weighted averages, with proportions estimated using weighted averages of indicator variables, may also be used for estimating the distribution of factors and for comparing the distributions between the overall E2197 study population and the genomic sample. Tests comparing factor distributions are based on asymptotic normality of the difference in weighted averages.

### Example Analysis 3

Recurrence risk was examined in the combined HR+ population (without and with adjustment for Recurrence Score [RS]), in the HR+, HER2- population, in the combined HR-population, and in the HR-, HER2- population. (Recurrence Score is described in detail in copending US Application Serial No. 10/883,303 and in S. Paik, et al., N. Engl. J. Med., 351: 2817-2826 (2004).) Since the finite population sub-sampling in the genomic data set produces some dependence among observations within a stratum, the following procedure was used to generate *K* independent sets for cross-validation. First, the subjects within each stratum in the 776-patient genomic data set are randomly divided into *K* subsets (with as close to equal numbers in each group as possible), without regard to outcome (recurrence) status. Then subjects within each stratum in the 2952-patient E2197 cohort who are not in the genomic sample are randomly divided into *K* subsets. For each of the *K* subsets, sampling weights (the inverse of the sampling fraction in each of the stratum-recurrence status combinations) are recomputed using just the data in that subset. These weights are used for the sampling weights in the validation analyses. For each of the *K* subsets, a set of sampling weights is recomputed using the complementary *(K-1)*/*K* portion of the data. These are used as the sampling weights in the training set analyses (with different weights when each of the *K* subsets is omitted).

The supervised principal components procedure (SPC) is described in detail in Bair et al (Bair E, et al., J. Amer. Stat. Assoc., 101:119-137 (2006)). In this procedure, variables (genes and other factors, if considered) are ranked in terms of their significance for the outcome of interest when considered individually. The ranking here is done using Cox model Wald statistics using the adjusted variance computed using the general theory in Lin. (D.Y. Lin, Biometrika, 87:37-47 (2000)) Univariate analysis of Hazard Ratios for each single gene are calculated (no exclusions) to assess which genes are associated with higher or lower risk of recurrence. The singular value decomposition (SVD) is then applied to the design matrix formed using the *m* most significant of the variables. In the design matrix, each variable is first centered to have mean 0. The leading left singular vector from this decomposition (also called the leading principal component) is then used as the continuous predictor of the outcome of interest. This continuous predictor can then be analyzed as a continuous variable or grouped to form prognostic or predictive classes. The contributions (factor loadings) of the individual variables to the predictor can also be examined, and those variables with loadings smaller in magnitude than a specified threshold could be eliminated to obtain a more parsimonious predictor.

The supervised principal components procedure has several possible tuning parameters. Most important is the number m of most individually significant variables to include. The threshold for elimination of variables with low contributions is another potential tuning parameter.

A nested cross-validation approach is used. At the top level, the subjects are randomly divided into *K* disjoint subsets (*K* = 5 is used in the analyses). First, the first subset is omitted. The supervised principal components procedure described below is then applied to develop a predictor or classifier using the remaining *(K-1)*/*K* portion of the data. This predictor or classifier is then applied to the omitted *1*/*K* portion of the data to evaluate how well it predicts or classifies in an independent set (that is, the omitted *1*/*K* portion is used as a validation sample). This process is repeated with each of the *K* subsets omitted in turn. The predictor /classifier developed is different for each omitted subset, but the results from the validation analyses can be aggregated to give an overall estimate of the accuracy of the procedure when applied to the full data set.

A nested cross-validation procedure is used to attempt to optimize the tuning parameters. In this procedure, *K*-fold cross-validation is applied to the training sample at each step of the top level cross-validation procedure. The *K* subsets of the training sample are generated as indicated above, except that the top-level coefficient of variation (CV) training subset (both the subjects in the genomic sample and those from E2197 not in the genomic sample) take the role of the full E2197 cohort. Within this second level of cross validation, the SPC procedure is applied to each training sample for a sequence of tuning parameter values, and the parameters are chosen to optimize some measure of performance (such as the value of the pseudo-likelihood or a Wald statistic) averaged over the validation samples. For the pseudo-likelihood, values are scaled by subtracting the log of the null model likelihood from the log pseudo likelihood for each model. The SPC procedure with these optimized tuning parameters is then applied to the full top-level CV training sample to generate the continuous predictor to evaluate on the omitted top level validation sample. Within this procedure, different optimized tuning parameters are therefore used for each step in the top-level CV procedure. Generally below, only the number of genes m is optimized in this fashion.

The primary analyses focus on the endpoint of recurrence, with follow-up censored at the time last known free of recurrence for patients without recurrence reported (including at death without recurrence). For analyses developing a prognostic classifier on the combined treatment arms, two analyses are performed on the validation sample. First, the continuous predictor is fit on the validation sample using the proportional hazards model (maximizing the weighted pseudo partial likelihood). This gives an estimated coefficient, standard error and p-value for each validation set. The average coefficient and approximate standard error over the validation sets are also computed. Second, three prognostic groups are defined using tertiles of the continuous predictor (defined on the training set), and each subject in the validation set is assigned to a prognostic group on the basis of this classifier. The weighted Kaplan-Meier estimates of the event-free probabilities are then computed within each prognostic group (within each validation set). These estimates from each tertile are then averaged over the validation sets to obtain an overall average estimate of performance. All analyses were run on 764 patients.

### Handling outlying gene expression values

To avoid problems with excessive influence from outlying gene expression values, substitution methods may be used for each gene. For example, two different methods were used in the above-described analyses. Specifically, for Analysis 1, the minimum value of gene expression was replaced by the 2^{nd} smallest value if the inter-quartile range (IQR) was higher than 0.3 and the difference between the two smallest values was more than 2 x the IQR. Since some genes have little variation, if the IQR were less than 0.3, the minimum was replaced by the 2^{nd} smallest value if the difference between the two smallest values was more than 2 x 0.3. Similarly, if the largest value was more than 2 x max {0.3, IQR} above the 2^{nd} largest, then the largest value was set to the same as the 2^{nd} largest. The same criteria were used to assess whether the second most extreme value had to be replaced.

For Analyses 2 and 3, if the minimum value for a gene was more than 2 x max {0.3, IQR} for the gene below the 2^{nd} smallest value, then the minimum was replaced by a missing value. Similarly, if the largest value was more than 2 x max {0.3, IQR} above the 2^{nd} largest, then the largest value was set to missing. Missing values then were replaced by the mean of the non-missing values for that gene.

### Example: Summary of Results

The results of these exemplar analyses are listed in Tables 3A-8B, below. The endpoint measured was Recurrence Free Interval. As used in these tables, "HR" means hazard ratio per standard deviation of gene expression. The hazard ratio is used to assess each gene's influence on the recurrence rate. If HR > 1, then elevated expression of a particular gene transcript or its expression product is associated with a higher recurrence rate and a negative clinical outcome. Similarly, if IIR < 1, then elevated expression of a particular gene transcript or its expression product is associated with a lower recurrence rate and a beneficial clinical outcome.

**Table 4A: (Hormone Receptor Positive (HR+), Any HER2) Genes with higher risk of recurrence with higher expression**

| **gene** | **Analysis 1 (Unadjusted)** | | **Analysis 2 (Adjusted)** | | **Analysis 3 (SPC predictor of recurrence, adj. for RS)** |
|---|---|---|---|---|---|
| | **HR** | **p value** | **HR** | **Korn adj. p value** | **HR** |
| NUSAP1 | 1.587 | 3.442E-07 | 1.5872 | 0.000 | 1.5872 |
| DEPDC1 | 1.672 | 2.063E-06 | 1.6720 | 0.000 | -- |
| TOP2A | 1.476 | 9.244E-06 | 1.4722 | 0.000 | -- |
| AURKB | 1.498 | 0.0000384 | 1.4978 | 0.002 | -- |
| BIRC5 | 1.422 | 0.0000422 | 1.3951 | 0.002 | -- |
| GAPDH | 2.350 | 0.0000516 | 2.3467 | 0.002 | 2.3467 |
| PTTG1 | 1.568 | 0.0000788 | 1.5683 | 0.002 | -- |
| CDC2 | 1.437 | 0.0001058 | 1.4376 | 0.002 | -- |
| KIFC1 | 1.501 | 0.0001395 | 1.5008 | 0.004 | -- |
| MKI67 | 1.527 | 0.0001604 | 1.4963 | 0.004 | -- |
| BUB1B | 7.128 | 0.0002369 | 7.1278 | 0.002 | -- |
| PLK1 | 1.414 | 0.0003211 | 1.4134 | 0.004 | -- |
| BUB1 | 1.464 | 0.0003938 | 1.4637 | 0.004 | -- |
| MAD2L1 | 1.513 | 0.0004665 | 1.5129 | 0.004 | -- |
| TACC3 | 1.609 | 0.0005893 | 1.6080 | 0.006 | -- |
| CENPF | 1.411 | 0.0006091 | 1.4106 | 0.006 | -- |
| NEK2 | 1.437 | 0.0008261 | 1.4376 | 0.010 | -- |
| CDC20 | 1.352 | 0.0011946 | 1.3512 | 0.016 | -- |
| TYMS | 1.493 | 0.0013813 | 1.4933 | 0.020 | -- |
| TTK | 1.418 | 0.0017844 | 1.4176 | 0.024 | -- |
| CENPA | 1.411 | 0.0017901 | 1.4106 | 0.030 | -- |
| FOXM1 | 1.418 | 0.0019025 | 1.4176 | 0.042 | -- |
| TPX2 | 1.348 | 0.0020794 | -- | ** | -- |
| CDCA8 | 1.420 | 0.0023514 | 1.4205 | 0.034 | -- |
| MYBL2 | 1.299 | 0.0030240 | -- | ** | -- |
| CCNB1 | 1.595 | 0.0051888 | -- | ** | -- |
| KIF11 | 1.371 | 0.0052941 | -- | ** | -- |
| ZWILCH | 1.634 | 0.0057525 | -- | ** | -- |
| GPR56 | 1.532 | 0.0060626 | -- | ** | -- |
| ZWINT | 1.358 | 0.0081847 | -- | ** | -- |
| KIF2C | 1.336 | 0.0101481 | -- | ** | -- |
| ESPL1 | 1.287 | 0.0111571 | -- | ** | -- |
| GRB7 | 1.259 | 0.0120361 | -- | ** | -- |
| HSP90AA1 | 1.627 | 0.0129320 | -- | ** | -- |
| CHGA | 1.159 | 0.0153291 | -- | ** | 1.1584 |
| PGK1 | 1.646 | 0.0158863 | -- | ** | -- |
| MMP12 | 1.271 | 0.0164373 | -- | ** | -- |
| MAGEA2 | 1.369 | 0.0173455 | -- | ** | -- |
| SLC7A5 | 1.250 | 0.0183518 | -- | ** | -- |
| CCND1 | 1.233 | 0.0202102 | -- | ** | 1.2324 |
| BRCA2 | 1.549 | 0.0276566 | -- | ** | -- |
| AURKA | 1.394 | 0.0402357 | -- | ** | -- |
| RAD54L | 1.302 | 0.0450509 | -- | ** | -- |
| ERBB2 | 1.199 | 0.0470906 | -- | ** | -- |

| | | | | | |
|---|---|---|---|---|---|
| **Korn adj. p value > 0.05 | | | | | |

**Table 4B: (Hormone Receptor Positive (HR+), Any HER2) Genes with lower risk of recurrence with higher expression**

| **gene** | **Analysis 1 (Unadjusted)** | | **Analysis 2 (Adjusted)** | | **Analysis 3 (SPC predictor of recurrence, adj. for RS)** |
|---|---|---|---|---|---|
| | **HR** | **p value** | **HR** | **Korn Adj p value** | **HR** |
| PFDN5 | 0.601 | 2.014E-07 | -- | ** | -- |
| STK11 | 0.399 | 4.404E-07 | 0.3985 | 0.000 | 0.3985 |
| SCUBE2 | 0.805 | 9.808E-06 | 0.8138 | 0.002 | -- |
| ZW10 | 0.430 | 0.0000102 | 0.4304 | 0.002 | -- |
| RASSF1 | 0.464 | 0.0000536 | 0.4639 | 0.002 | 0.4639 |
| ID1 | 0.583 | 0.0000757 | 0.5827 | 0.004 | -- |
| ABCA9 | 0.702 | 0.0001145 | 0.7026 | 0.002 | -- |
| GSTM1 | 0.713 | 0.0001248 | 0.7218 | 0.004 | -- |
| PGR | 0.815 | 0.0001459 | 0.8138 | 0.004 | -- |
| PRDM2 | 0.620 | 0.0001680 | 0.6206 | 0.004 | -- |
| RELA | 0.496 | 0.0002484 | 0.4956 | 0.004 | 0.4956 |
| FHIT | 0.661 | 0.0002685 | 0.6610 | 0.004 | -- |
| ERCC1 | 0.497 | 0.0002786 | 0.4971 | 0.004 | -- |
| ESR1 | 0.814 | 0.0004879 | 0.8245 | 0.032 | -- |
| AKT3 | 0.629 | 0.0007087 | 0.6288 | 0.006 | -- |
| SLC1A3 | 0.614 | 0.0011054 | 0.6139 | 0.016 | 0.6139 |
| CSF1 | 0.559 | 0.0011623 | 0.5593 | 0.012 | -- |
| AKT2 | 0.491 | 0.0013291 | 0.4916 | 0.016 | -- |
| PECAM1 | 0.614 | 0.0014795 | 0.6145 | 0.022 | -- |
| PIK3C2A | 0.533 | 0.0015982 | 0.5331 | 0.022 | -- |
| MAPT | 0.822 | 0.0016329 | 0.8220 | 0.032 | -- |
| MRE11A | 0.585 | 0.0018207 | 0.5851 | 0.030 | -- |
| MYH11 | 0.788 | 0.0018833 | 0.7882 | 0.022 | -- |
| NPC2 | 0.524 | 0.0019133 | 0.5241 | 0.024 | -- |
| GADD45B | 0.614 | 0.0019389 | 0.6145 | 0.022 | -- |
| PTPN21 | 0.706 | 0.0019855 | 0.7061 | 0.032 | -- |
| COL1A1 | 0.741 | 0.0020877 | 0.7408 | 0.034 | -- |
| ROCK1 | 0.550 | 0.0025041 | 0.5499 | 0.034 | -- |
| ABAT | 0.793 | 0.0025380 | 0.7937 | 0.034 | -- |
| COL1A2 | 0.769 | 0.0028633 | 0.7408 | 0.034 | -- |
| PIM2 | 0.713 | 0.0029396 | 0.7132 | 0.032 | 0.7132 |
| CDKN1C | 0.697 | 0.0031276 | 0.6970 | 0.044 | -- |
| SEMA3F | 0.720 | 0.0032523 | -- | ** | -- |
| PMS2 | 0.538 | 0.0035689 | 0.5379 | 0.050 | -- |
| MGC52057 | 0.720 | 0.0037128 | 0.7204 | 0.024 | -- |
| FAS | 0.674 | 0.0037460 | 0.6744 | 0.050 | -- |
| ELP3 | 0.553 | 0.0040295 | -- | ** | -- |
| BAX | 0.506 | 0.0046591 | -- | ** | -- |
| PRKCH | 0.637 | 0.0050308 | -- | ** | -- |
| CD247 | 0.735 | 0.0052363 | -- | ** | 0.7349 |
| NME6 | 0.615 | 0.0053468 | -- | ** | -- |
| GGPS1 | 0.621 | 0.0056877 | -- | ** | -- |
| ACTR2 | 0.459 | 0.0057060 | -- | ** | 0.4593 |
| STAT3 | 0.715 | 0.0058238 | 0.7153 | 0.008 | -- |
| BIRC3 | 0.756 | 0.0065975 | -- | ** | 0.7558 |
| ABCB1 | 0.581 | 0.0066902 | -- | ** | -- |
| RPLPO | 0.439 | 0.0067008 | -- | ** | -- |
| CLU | 0.771 | 0.0068700 | -- | ** | -- |
| FYN | 0.652 | 0.0068877 | -- | ** | -- |
| MAP4 | 0.512 | 0.0076104 | -- | ** | -- |
| IGFBP2 | 0.776 | 0.0081400 | -- | ** | -- |
| RELB | 0.695 | 0.0081769 | -- | ** | -- |
| WNT5A | 0.700 | 0.0084988 | -- | ** | -- |
| LIMK1 | 0.634 | 0.0088995 | -- | ** | -- |
| CYP1B1 | 0.727 | 0.0105903 | -- | ** | -- |
| LILRB1 | 0.721 | 0.0106359 | -- | ** | -- |
| PPP2CA | 0.559 | 0.0111439 | -- | ** | -- |
| ABCG2 | 0.660 | 0.0115255 | -- | ** | -- |
| EGFR | 0.754 | 0.0124036 | -- | ** | -- |
| BBC3 | 0.719 | 0.0139470 | -- | ** | -- |
| TNFRSF10B | 0.700 | 0.0144998 | -- | ** | -- |
| CYP2C8 | 0.483 | 0.0145393 | -- | ** | -- |
| CTNNB1 | 0.611 | 0.0166914 | -- | ** | -- |
| SGK3 | 0.757 | 0.0168533 | -- | ** | -- |
| BIRC4 | 0.625 | 0.0172627 | -- | ** | -- |
| MAPK3 | 0.710 | 0.0202294 | -- | ** | -- |
| ARAF | 0.657 | 0.0202552 | -- | ** | -- |
| IRS1 | 0.776 | 0.0208563 | -- | ** | -- |
| APOD | 0.852 | 0.0213176 | -- | ** | -- |
| CAV1 | 0.650 | 0.0213454 | -- | ** | -- |
| MMP2 | 0.827 | 0.0217710 | -- | ** | -- |
| KNS2 | 0.659 | 0.0230028 | -- | ** | -- |
| PIM1 | 0.756 | 0.0235704 | -- | ** | -- |
| VCAM1 | 0.742 | 0.0237609 | -- | ** | -- |
| FASLG | 0.489 | 0.0240244 | -- | ** | 0.4892 |
| MAD1L1 | 0.667 | 0.0261089 | -- | ** | -- |
| RPL37A | 0.592 | 0.0265180 | -- | ** | -- |
| FLAD1 | 0.633 | 0.0266318 | -- | ** | -- |
| MAPK14 | 0.591 | 0.0272216 | -- | ** | -- |
| CDKN1B | 0.694 | 0.0272468 | -- | ** | -- |
| DICER1 | 0.748 | 0.0286966 | -- | ** | -- |
| PDGFRB | 0.759 | 0.028825 | -- | ** | -- |
| NFKB1 | 0.643 | 0.0309325 | -- | ** | -- |
| VEGFB | 0.757 | 0.0328536 | -- | ** | -- |
| FUS | 0.651 | 0.0363513 | -- | ** | -- |
| SNA12 | 0.771 | 0.0380711 | -- | ** | -- |
| TUBD1 | 0.749 | 0.0405564 | -- | ** | -- |
| CAPZA1 | 0.558 | 0.0407558 | -- | ** | -- |
| BCL2 | 0.782 | 0.0415340 | -- | ** | -- |
| GATA3 | 0.851 | 0.0421418 | -- | ** | -- |
| STK10 | 0.724 | 0.0436867 | -- | ** | -- |
| CNN1 | 0.816 | 0.0437974 | -- | ** | -- |
| SRI | 0.602 | 0.0438974 | -- | ** | -- |
| FOXA1 | 0.863 | 0.0440180 | -- | ** | -- |
| GBP2 | 0.741 | 0.0447335 | -- | ** | -- |
| RPN2 | 0.765 | 0.0447404 | -- | ** | -- |
| ANXA4 | 0.745 | 0.0489155 | -- | ** | -- |
| MCL1 | 0.680 | 0.0494269 | -- | ** | -- |
| GBP1 | -- | -- | -- | ** | 0.8428 |
| STAT1 | -- | -- | -- | ** | 0.8294 |
| LILRB1 | -- | -- | -- | ** | 0.7211 |
| ZW10 | -- | -- | -- | ** | 0.4304 |

| | | | | | |
|---|---|---|---|---|---|
| ** Korn adj. p value > 0.05 | | | | | |

**Table 5A: (Hormone Receptor Negative (HR-), Any HER2) Genes with higher risk of recurrence with higher expression**

| **gene** | **Analysis 1 (Unadjusted)** | | **Analysis 2 (Adjusted)** | | **Analysis 3 (SPC predictor of recurrence, adj. for RAS)** |
|---|---|---|---|---|---|
| | **HR** | **p value** | **HR** | **Korn adj. p** | **HR** |
| MYBL2 | 1.695 | 0.0019573 | -- | ** | 1.7006 |
| GPR126 | 1.380 | 0.0068126 | -- | ** | -- |
| GPR56 | 1.358 | 0.0131494 | -- | ** | -- |
| GRB7 | 1.154 | 0.0190295 | -- | ** | -- |
| CKAP1 | 1.515 | 0.0216536 | -- | ** | -- |
| N EK2 | 1.331 | 0.0219334 | -- | ** | -- |
| L1CAM | 1.184 | 0.0231607 | -- | ** | -- |
| TUBA3 | 1.383 | 0.0294187 | -- | ** | -- |
| LAPTM4B | 1.300 | 0.0381478 | -- | ** | -- |
| TBCE | 1.468 | 0.0401742 | -- | ** | -- |

| | | | | | |
|---|---|---|---|---|---|
| ** Korn adj. p value > 0.05 | | | | | |

**Table 5B: (Hormone Receptor Negative (HR-), Any HER2) Genes with lower risk of recurrence with higher expression**

| **gene** | **Analysis 1 (Unadjusted)** | | **Analysis 2 (Adjusted)** | | **Analysis 3 (SPC predictor of recurrence, adj. for RS)** |
|---|---|---|---|---|---|
| | **HR** | **p value** | **HR** | **Korn adj. p value** | **HR** |
| CD68 | 0.652 | 0.0000543 | -- | ** | 0.6525 |
| ACTR2 | 0.695 | 0.0000610 | -- | ** | -- |
| ESR2 | 0.142 | 0.0003262 | 0.1418 | 0.000 | 0.1418 |
| BIRC3 | 0.710 | 0.0003312 | 0.7103 | 0.008 | 0.7103 |
| PIM2 | 0.721 | 0.0003382 | 0.7211 | 0.000 | 0.7211 |
| VCAM1 | 0.716 | 0.0004912 | 0.7161 | 0.014 | 0.7161 |
| RELB | 0.572 | 0.0005896 | 0.5718 | 0.014 | 0.5718 |
| IL7 | 0.606 | 0.0007567 | 0.6053 | 0.014 | 0.6053 |
| APOC1 | 0.726 | 0.0011094 | 0.7254 | 0.042 | 0.7254 |
| XIST | 0.730 | 0.0013534 | -- | ** | 0.7298 |
| CST7 | 0.727 | 0.0020814 | -- | ** | 0.7276 |
| GBP2 | 0.695 | 0.0022400 | -- | ** | 0.6956 |
| PRKCH | 0.602 | 0.0022573 | -- | ** | 0.6023 |
| LILRB1 | 0.706 | 0.0029297 | -- | ** | 0.07061 |
| FASLG | 0.458 | 0.0041478 | 0.4584 | 0.022 | 0.4584 |
| CSF1 | 0.676 | 0.0042618 | -- | ** | 0.6757 |
| CD247 | 0.734 | 0.0042817 | -- | ** | 0.7334 |
| BIN1 | 0.711 | 0.0043244 | -- | ** | 0.7103 |
| WNT5A | 0.483 | 0.0045915 | -- | ** | -- |
| PRKCA | 0.730 | 0.0051254 | -- | ** | 0.7298 |
| STAT1 | 0.723 | 0.0061824 | -- | ** | 0.7233 |
| PGR | 0.604 | 0.0068937 | -- | ** | 0.6169 |
| IRAK2 | 0.634 | 0.0073992 | -- | ** | 0.6338 |
| CYBA | 0.711 | 0.0077397 | -- | ** | 0.7103 |
| SCUBE2 | 0.783 | 0.0087744 | -- | ** | 0.7851 |
| ERCC1 | 0.505 | 0.0089315 -- | | ** | -- |
| CAPZA1 | 0.574 | 0.0091684 | -- | ** | 0.5735 |
| IL2RA | 0.634 | 0.0098419 | -- | ** | 0.6338 |
| GBP1 | 0.779 | 0.0104451 | -- | ** | 0.7788 |
| PECAM1 | 0.694 | 0.0130612 | -- | ** | 0.6942 |
| CCL2 | 0.729 | 0.0136238 | -- | ** | 0.7291 |
| STAT3 | 0.530 | 0.0152545 | -- | ** | 0.5305 |
| NFKB1 | 0.596 | 0.0161377 | -- | ** | 0.5963 |
| CD14 | 0.692 | 0.0161533 | -- | ** | 0.6921 |
| TNFSF10 | 0.782 | 0.0167007 | -- | ** | 0.7819 |
| TFF1 | 0.811 | 0.0197258 | -- | ** | -- |
| GADD45A | 0.720 | 0.0228062 | -- | ** | -- |
| SLC1A3 | 0.769 | 0.0228194 | -- | ** | -- |
| BAD | 0.645 | 0.0230521 | -- | ** | -- |
| FYN | 0.745 | 0.0245100 | -- | ** | 0.7453 |
| CTSL | 0.722 | 0.0247385 | -- | ** | -- |
| DIAPH1 | 0.623 | 0.0251948 | -- | ** | -- |
| ABAT | 0.737 | 0.0277218 | -- | ** | -- |
| ABCG2 | 0.544 | 0.0300971 | -- | ** | -- |
| PRKCG | 0.349 | 0.0314412 | -- | ** | -- |
| PLD3 | 0.654 | 0.0332019 | -- | ** | -- |
| KNTC1 | 0.742 | 0.0335689 | -- | ** | -- |
| GSR | 0.712 | 0.0345107 | -- | ** | -- |
| CSAG2 | 0.840 | 0.0350118 | -- | ** | -- |
| CHFR | 0.671 | 0.0380636 | -- | ** | -- |
| MSH3 | 0.700 | 0.0460279 | -- | ** | -- |
| TPT1 | 0.713 | 0.0483077 | -- | ** | -- |
| BAX | 0.601 | 0.0488665 | -- | ** | -- |
| CLU | 0.855 | 0.0492894 | -- | ** | -- |
| ABCA9 | 0.809 | 0.0494329 | -- | ** | -- |
| STK10 | 0.737 | 0.0498826 | -- | ** | -- |
| APOE | -- | -- | -- | ** | 0.8353 |

| | | | | | |
|---|---|---|---|---|---|
| ** Korn adj. p value> 0.05 | | | | | |

**Table 6A: (Hormone Receptor Positive (HR+), HER2 Negative (HER2-)) Genes with higher risk of recurrence with higher expression**

| **gene** | **Analysis 1 (Unadjusted)** | | **Analysis 2 (Adjusted)** | |
|---|---|---|---|---|
| | **HR** | **p value** | **HR** | **Korn adj. p value** |
| NUSAP1 | 1.640 | 5.151E-07 | 1.6703 | 0.000 |
| DEPDC1 | 1.671 | 9.82E-06 | 1.6703 | 0.000 |
| TOP2A | 1.554 | 0.0000134 | 1.5543 | 0.000 |
| AURKB | 1.591 | 0.0000153 | 1.5904 | 0.000 |
| GAPDH | 2.726 | 0.0000175 | 2.5498 | 0.004 |
| KIFC1 | 1.586 | 0.0000699 | 1.5857 | 0.004 |
| BIRC5 | 1.420 | 0.0002009 | 1.3979 | 0.008 |
| PLK1 | 1.446 | 0.0003978 | 1.4463 | 0.008 |
| TYMS | 1.588 | 0.0004860 | 1.5872 | 0.008 |
| PTTG1 | 1.543 | 0.0006240 | 1.5434 | 0.008 |
| CENPF | 1.453 | 0.0007597 | 1.4521 | 0.010 |
| MKI67 | 1.522 | 0.0007619 | 1.4933 | 0.032 |
| CDC2 | 1.405 | 0.0008394 | 1.4049 | 0.016 |
| BUB1B | 6.708 | 0.0008669 | 6.6993 | 0.006 |
| FOXM1 | 1.477 | 0.0012756 | -- | ** |
| ESPL1 | 1.418 | 0.0013859 | 1.4191 | 0.030 |
| TACC3 | 1.605 | 0.0014749 | 1.6048 | 0.032 |
| NEK2 | 1.448 | 0.0018204 | 1.4477 | 0.040 |
| MAD2L1 | 1.480 | 0.0023430 | 1.4799 | 0.042 |
| TTK | 1.442 | 0.0023457 | -- | ** |
| BUB1 | 1.426 | 0.0024859 | 1.4262 | 0.044 |
| MYBL2 | 1.344 | 0.0033909 | -- | ** |
| TPX2 | 1.361 | 0.0037780 | -- | ** |
| CENPA | 1.407 | 0.0047243 | -- | ** |
| CDC20 | 1.335 | 0.0055217 | -- | ** |
| CDCA8 | 1.404 | 0.0060377 | -- | ** |
| CCND1 | 1.323 | 0.0063660 | -- | ** |
| ZWINT | 1.414 | 0.0084107 | -- | ** |
| CCNB1 | 1.639 | 0.0085470 | -- | ** |
| ZWILCH | 1.649 | 0.0106632 | -- | ** |
| CENPE | 1.715 | 0.0106842 | -- | ** |
| KIF11 | 1.371 | 0.0113708 | -- | ** |
| BRCA1 | 1.430 | 0.0150194 | -- | ** |
| CHGA | 1.157 | 0.0257914 | -- | ** |
| HSPA5 | 1.982 | 0.0264599 | -- | ** |
| MAGEA2 | 1.394 | 0.0268474 | -- | ** |
| KIF2C | 1.304 | 0.0315403 | -- | ** |
| RAD54L | 1.346 | 0.0368698 | -- | ** |
| CA9 | 1.213 | 0.0420931 | -- | ** |

| | | | | |
|---|---|---|---|---|
| ** Korn adj. p value > 0.05 | | | | |

**Table 6B: (Hormone Receptor Positive (HR+), HER2 Negative (HER2-)) Genes with lower risk of recurrence with higher expression**

| **gene** | **Analysis 1 (Unadjusted)** | | **Analysis 2 (Adjusted)** | |
|---|---|---|---|---|
| | **HR** | **p value** | **HR** | **Korn adj. p value** |
| STK11 | 0.407 | 9.027E-06 | 0.4070 | 0.002 |
| ACTR2 | 0.283 | 0.0000563 | 0.2825 | 0.004 |
| ZW10 | 0.446 | 0.0000654 | 0.4466 | 0.002 |
| RASSF1 | 0.451 | 0.0000826 | 0.4507 | 0.004 |
| ID1 | 0.586 | 0.0001968 | 0.5863 | 0.008 |
| MMP2 | 0.719 | 0.0002211 | 0.7189 | 0.010 |
| NPC2 | 0.435 | 0.0003018 | -- | ** |
| GADD45B | 0.530 | 0.0003473 | 0.5305 | 0.006 |
| COL1A2 | 0.728 | 0.0004219 | 0.7283 | 0.016 |
| SLC1A3 | 0.568 | 0.0006126 | 0.5684 | 0.014 |
| SCUBE2 | 0.829 | 0.0006362 | -- | ** |
| RELA A | 0.486 | 0.0006912 | 0.4863 | 0.020 |
| PTPN21 | 0.658 | 0.0007339 | 0.6577 | 0.016 |
| GSTM1 | 0.713 | 0.0009258 | 0.7225 | 0.040 |
| COL1A1 | 0.710 | 0.0009907 | 0.7096 | 0.026 |
| PRDM2 | 0.625 | 0.0011046 | 0.6250 | 0.018 |
| AKT3 | 0.612 | 0.0011152 | 0.6114 | 0.026 |
| CSF1 | 0.507 | 0.0012382 | 0.5071 | 0.020 |
| FAS | 0.615 | 0.0012471 | 0.6145 | 0.020 |
| ABCA9 | 0.726 | 0.0012999 | 0.7261 | 0.028 |
| ROCK1 | 0.474 | 0.0018953 | 0.4738 | 0.044 |
| VCAM1 | 0.650 | 0.0022313 | -- | ** |
| PIM2 | 0.686 | 0.0023819 | 0.6859 | 0.040 |
| CD247 | 0.685 | 0.0024093 | 0.6845 | 0.036 |
| PECAM1 | 0.605 | 0.0024170 | -- | ** |
| PIK3C2A | 0.501 | 0.0026879 | -- | ** |
| FYN | 0.597 | 0.0034648 | -- | ** |
| CYP2C8 | 0.353 | 0.0034744 | -- | ** |
| MAP4 | 0.456 | 0.0036702 | -- | ** |
| PPP2CA | 0.478 | 0.0039174 | -- | ** |
| CDKN1C | 0.677 | 0.0039353 | -- | ** |
| PRKCH | 0.621 | 0.0043849 | -- | ** |
| ERCC1 | 0.546 | 0.0048268 | -- | ** |
| BAX | 0.471 | 0.0050208 | -- | ** |
| PDGFRB | 0.692 | 0.0053268 | -- | ** |
| STK10 | 0.545 | 0.0054089 | -- | ** |
| CXCR4 | 0.670 | 0.0072433 | -- | ** |
| FHIT | 0.714 | 0.0073859 | -- | ** |
| ELP3 | 0.544 | 0.0076927 | -- | ** |
| ITGB1 | 0.447 | 0.0086595 | -- | ** |
| PGR | 0.853 | 0.0088435 | -- | ** |
| BIRC3 | 0.742 | 0.0090302 | -- | ** |
| RPN2 | 0.734 | 0.0091664 | -- | ** |
| MYH11 | 0.799 | 0.0093865 | -- | ** |
| NME6 | 0.623 | 0.0095259 | -- | ** |
| GGPS1 | 0.620 | 0.0099960 | -- | ** |
| CAPZA1 | 0.444 | 0.0105944 | -- | ** |
| MRE11A | 0.622 | 0.0112118 | -- | ** |
| BIRC4 | 0.581 | 0.0114118 | -- | ** |
| ABAT | 0.814 | 0.0117097 | -- | ** |
| TNFRSF10B | 0.669 | 0.0118729 | -- | ** |
| ACTB | 0.490 | 0.0119353 | -- | ** |
| SEMA3F | 0.733 | 0.0122670 | -- | ** |
| WNT5A | 0.683 | 0.0124795 | -- | ** |
| EGFR | 0.728 | 0.0128218 | -- | ** |
| PIM1 | 0.713 | 0.0130874 | -- | ** |
| REB | 0.698 | 0.0151985 | -- | ** |
| LILRB1 | 0.712 | 0.0153494 | -- | ** |
| S100A10 | 0.638 | 0.0154250 | -- | ** |
| MAD1L1 | 0.613 | 0.0162166 | -- | ** |
| LIMK1 | 0.640 | 0.0166726 | -- | ** |
| SNAI2 | 0.725 | 0.0179736 | -- | ** |
| CYP1B1 | 0.728 | 0.0181752 | -- | ** |
| CTNNB1 | 0.586 | 0.0187559 | -- | ** |
| KNS2 | 0.617 | 0.0191798 | -- | ** |
| STAT3 | 0.741 | 0.0208400 | -- | ** |
| ESR1 | 0.851 | 0.0220104 | -- | ** |
| CCL2 | 0.734 | 0.0229520 | -- | ** |
| BBC3 | 0.713 | 0.0235615 | -- | ** |
| AKT2 | 0.577 | 0.0243569 | -- | ** |
| MAPK14 | 0.522 | 0.0245184 | -- | ** |
| CALD1 | 0.666 | 0.0256356 | -- | ** |
| FASLG | 0.408 | 0.0256649 | -- | ** |
| ABCG2 | 0.668 | 0.0265022 | -- | ** |
| CAV1 | 0.623 | 0.0276134 | -- | ** |
| ABCB1 | 0.620 | 0.0276165 | -- | ** |
| HIFIA | 0.620 | 0.0284583 | -- | ** |
| MAPK3 | 0.698 | 0.0284801 | -- | ** |
| GBP1 | 0.773 | 0.0300251 | -- | ** |
| PMS2 | 0.601 | 0.0302953 | -- | ** |
| RHOC | 0.668 | 0.0324165 | -- | ** |
| PRKCD | 0.642 | 0.0340220 | -- | ** |
| ANXA4 | 0.719 | 0.0353797 | -- | ** |
| GBP2 | 0.700 | 0.0356809 | -- | ** |
| CLU | 0.805 | 0.0376120 | -- | ** |
| IL7 | 0.725 | 0.0390326 | -- | ** |
| COL6A3 | 0.826 | 0.0436450 | -- | ** |
| HSPA1L | 0.276 | 0.0478052 | -- | ** |
| MGC52057 | 0.791 | 0.0478901 | -- | ** |

| | | | | |
|---|---|---|---|---|
| ** Korn adj. p value > 0.05 | | | | |

**Table 7A: (Hormone Receptor Negative (HR-), HER2 Negative (HER2-)) Genes with higher risk of recurrence with higher expression**

| **gene** | **Analysis 1 (Unadjusted)** | | **Analysis 2 (Adjusted)** | | **Analysis 3 (SPC predictor of recurrence, adj. for RS)** |
|---|---|---|---|---|---|
| | **HR** | **p value** | **HR** | **Korn adj. p value** | **HR** |
| GRB7 | 1.906 | 0.0000224 | 1.8908 | 0.000 | 1.8908 |
| GAGE1 | 1.648 | 0.0043470 | -- | ** | 1.6487 |
| GPR126 | 1.442 | 0.0055425 | -- | ** | -- |
| CYP2C8 | 2.363 | 0.0083138 | -- | ** | -- |
| NEK2 | 1.460 | 0.0091325 | -- | ** | -- |
| KRT19 | 1.354 | 0.0156629 | -- | ** | -- |
| MYBL2 | 1.604 | 0.01946199 | -- | ** | -- |
| MYC | 1.379 | 0.0299718 | -- | ** | -- |
| CKAP1 | 1.560 | 0.0304028 | -- | ** | -- |
| TUBA3 | 1.423 | 0.0311994 | -- | ** | -- |
| L1CAM | 1.197 | 0.0331190 | -- | ** | -- |
| ERBB2 | 1.381 | 0.0362432 | -- | ** | -- |
| CCND1 | 1.259 | 0.0499983 | -- | ** | -- |

| | | | | | |
|---|---|---|---|---|---|
| ** Korn adj. p value > 0.05 | | | | | |

**Table 7B: (Hormone Receptor Negative (HR-), HER2 Negative (HER2-)) Genes with lower risk of recurrence with higher expression**

| **gene** | **Analysis 1 (Unadjusted)** | | **Analysis 2 (Adjusted)** | | **Analysis 3 (SPC predictor of recurrence, adj. for RS)** |
|---|---|---|---|---|---|
| | **HR** | **p value** | **HR** | **Korn adj. p value** | |
| CD68 | 0.592 | 2.116E-07 | 0.5945 | 0.002 | 0.5945 |
| ACTR2 | 0.656 | 1.36E-06 | -- | ** | -- |
| XIST | 0.654 | 0.0000296 | 0.6544 | 0.022 | 0.6544 |
| APOC1 | 0.637 | 0.0000523 | 0.6364 | 0.000 | 0.6364 |
| BIRC3 | 0.662 | 0.0001202 | 0.6623 | 0.002 | 0.6623 |
| ESR2 | 0.084 | 0.0001243 | 0.0840 | 0.000 | 0.0840 |
| PIM2 | 0.671 | 0.0001318 | 0.6710 | 0.002 | 0.6710 |
| SLC1A3 | 0.620 | 0.0002156 | 0.6200 | 0.014 | 0.6200 |
| BIN1 | 0.626 | 0.0002500 | 0.6256 | 0.012 | 0.6256 |
| PRKCH | 0.502 | 0.0004783 | 0.5021 | 0.014 | 0.5021 |
| LILRB1 | 0.639 | 0.0006606 | 0.6395 | 0.012 | 0.6395 |
| CST7 | 0.671 | 0.0006776 | 0.6710 | 0.018 | 0.6710 |
| RELB | 0.526 | 0.0007769 | 0.5262 | 0.020 | 0.5262 |
| VCAM1 | 0.700 | 0.0009248 | 0.6998 | 0.026 | 0.6998 |
| CAPZA1 | 0.449 | 0.0011732 | -- | ** | 0.4489 |
| GBP2 | 0.635 | 0.0011762 | 0.6351 | 0.034 | 0.6351 |
| PLD3 | 0.523 | 0.0013142 | -- | ** | 0.5231 |
| IRAK2 | 0.512 | 0.0016339 | -- | ** | 0.5117 |
| IL7 | 0.584 | 0.0018037 | 0.5839 | 0.032 | 0.5839 |
| CTSL | 0.660 | 0.0026678 | -- | ** | -- |
| CSF1 | 0.633 | 0.0027615 | -- | ** | 0.6325 |
| CD247 | 0.688 | 0.0028163 | -- | ** | 06880 |
| FASLG | 0.356 | 0.0030677 | 0.3563 | 0.014 | 0.3563 |
| GNS | 0.483 | 0.0035073 | -- | ** | 0.4834 |
| CYBA | 0.664 | 0.0044996 | -- | ** | 0.6643 |
| NFKB1 | 0.502 | 0.0046224 | -- | ** | 0.5016 |
| DIAPH1 | 0.512 | 0.0047600 | -- | ** | 0.5117 |
| IL2RA | 0.558 | 0.0052120 | -- | ** | 0.5577 |
| STAT1 | 0.682 | 0.0053202 | -- | ** | 0.6818 |
| PECAM1 | 0.628 | 0.0056997 | -- | ** | 0.6281 |
| PLAU | 0.646 | 0.0059777 | -- | ** | 0.6466 |
| ERCC1 | 0.432 | 0.0067565 | -- | ** | 0.4321 |
| ABCC3 | 0.648 | 0.0074137 | -- | ** | 0.6479 |
| WNT5A | 0.455 | 0.0074176 | -- | ** | -- |
| CCL2 | 0.682 | 0.0074775 | -- | ** | 0.6818 |
| CD14 | 0.639 | 0.0087980 | -- | ** | -- |
| MMP9 | 0.763 | 0.0089472 | -- | ** | -- |
| BAD | 0.568 | 0.0099167 | -- | ** | -- |
| GBP1 | 0.749 | 0.0100726 | -- | ** | -- |
| GADD45A | 0.658 | 0.0108479 | -- | ** | -- |
| CDKN1A | 0.632 | 0.0110232 | -- | ** | -- |
| ECGF1 | 0.702 | 0.0111429 | -- | ** | -- |
| STK10 | 0.654 | 0.0116239 | -- | ** | -- |
| RKCA | 0.731 | 0.0121695 | -- | ** | -- |
| MMP2 | 0.738 | 0.0129347 | -- | ** | -- |
| GSR | 0.625 | 0.0164580 | -- | ** | -- |
| PLAUR | 0.656 | 0.0194483 | -- | ** | -- |
| BAX | 0.482 | 0.0221901 | -- | ** | -- |
| PRKCG | 0.263 | 0.0223421 | -- | ** | -- |
| FYN | 0.725 | 0.0227879 | -- | ** | 0.7254 |
| APOE | 0.799 | 0.0229649 | -- | ** | 0.7993 |
| ACTB | 0.502 | 0.0241365 | -- | ** | -- |
| GLRX | 0.271 | 0.0256879 | -- | ** | -- |
| TYRO3 | 0.627 | 0.0270209 | -- | ** | -- |
| SCUBE2 | 0.780 | 0.0271519 | -- | ** | -- |
| STAT3 | 0.517 | 0.0281809 | -- | ** | -- |
| CLU | 0.827 | 0.0283483 | -- | ** | -- |
| PRDM2 | 0.721 | 0.0287352 | -- | ** | -- |
| KALPHAL | 0.549 | 0.0345194 | -- | ** | -- |
| RELA | 0.591 | 0.0372553 | -- | ** | -- |
| KNS2 | 0.634 | 0.0391500 | -- | ** | -- |
| COL1A1 | 0.791 | 0.0405529 | -- | ** | -- |
| MET | 0.714 | 0.0415376 | -- | ** | -- |
| NPC2 | 0.632 | 0.0415918 | -- | ** | -- |
| SNA12 | 0.734 | 0.0420155 | -- | ** | -- |
| ABCG2 | 0.529 | 0.0456976 | -- | ** | -- |
| GPX1 | 0.614 | 0.0459149 | -- | ** | -- |
| PGR | 0.632 | 0.0459791 | -- | ** | -- |
| IGFBP3 | 0.744 | 0.0465884 | -- | ** | -- |
| TNFSF10 | 0.793 | 0.0486299 | -- | ** | -- |

| | | | | | |
|---|---|---|---|---|---|
| ** Korn adj. p value > 0.05 | | | | | |

**Table 8A: (Hormone Receptor Positive (HR+), HER2 Positive (HER2+)) Genes with higher risk of recurrence with higher expression**

| **gene** | **Analysis 1 (Unadjusted)** | | **Analysis 2 (Adjusted)** | |
|---|---|---|---|---|
| | **HR** | **p value** | **HR** | **Korn adj. p value** |
| ERBB2 | 1.864 | 0.0014895 | 1.8814 | 0.00 |
| TUBB3 | 1.779 | 0.0017456 | -- | ** |
| VEGFC | 2.909 | 0.0034593 | -- | ** |
| GRB7 | 1.702 | 0.0042453 | 1.6955 | 0.044 |
| GPR56 | 2.997 | 0.0048843 | -- | ** |
| PGK1 | 4.246 | 0.0051870 | -- | ** |
| SLC7A5 | 1.935 | 0.0058417 | -- | ** |
| CDH1 | 2.213 | 0.0132978 | -- | ** |
| PLAUR | 2.141 | 0.0155687 | -- | ** |
| THUS1 | 2.203 | 0.0189764 | -- | ** |
| APRT | 3.447 | 0.0206994 | -- | ** |
| VIM | 2.361 | 0.0238545 | -- | ** |
| SL | 1.916 | 0.0248133 | -- | ** |
| MMP12 | 1.614 | 0.0251326 | -- | ** |
| HSP90AA1 | 2.783 | 0.0267250 | -- | ** |
| PLAU | 1.885 | 0.0342672 | -- | ** |
| ABCC3 | 1.635 | 0.0368664 | -- | ** |
| C140RF10 | 2.140 | 0.0399352 | -- | ** |
| PTTG1 | 1.624 | 0.0493965 | -- | ** |

| | | | | |
|---|---|---|---|---|
| ** Korn adj. p value > 0.05 | | | | |

**Table 8B: (Hormone Receptor Positive (HR+), HER2 Positive (HER2+)) Genes with lower risk of recurrence with higher expression**

| **gene** | **Analysis 1 (Unadjusted)** | | **Analysis 2 (Adjusted)** | |
|---|---|---|---|---|
| | **HR** | **p value** | **HR** | **Korn adj. p value** |
| PFDN5 | 0.636 | 9.018E-06 | -- | ** |
| RPLP0 | 0.081 | 0.0001399 | 0.0711 | 0.034 |
| MAPT | 0.612 | 0.0005146 | 0.6126 | 0.00 |
| ESR1 | 0.721 | 0.0019943 | -- | ** |
| APOD | 0.658 | 0.0032732 | -- | ** |
| IGFBP2 | 0.632 | 0.0035894 | -- | ** |
| SGK3 | 0.418 | 0.0048934 | -- | ** |
| SCUBE2 | 0.692 | 0.0056279 | -- | ** |
| PGR | 0.712 | 0.0059421 | 0.6663 | 0.036 |
| IRS1 | 0.528 | 0.0065563 | -- | ** |
| KLK10 | 0.174 | 0.0094446 | -- | ** |
| CHEK2 | 0.294 | 0.0141015 | -- | ** |
| MGC52057 | 0.381 | 0.0142698 | -- | ** |
| FHIT | 0.523 | 0.0157057 | -- | ** |
| AKT2 | 0.260 | 0.0220461 | -- | ** |
| FASN | 0.609 | 0.0284812 | -- | ** |
| ERCC1 | 0.345 | 0.0347430 | -- | ** |
| ABCA9 | 0.611 | 0.0360546 | -- | ** |
| GATA3 | 0.728 | 0.0374971 | -- | ** |
| STK11 | 0.379 | 0.0395275 | -- | ** |
| TUBD1 | 0.552 | 0.0414193 | -- | ** |

| | | | | |
|---|---|---|---|---|
| ** Korn adj. p value > 0.05 | | | | |

**Table 9A: (Hormone Receptor Negative (HR-), HER2 Positive (HER2+)) Genes with higher risk of recurrence with higher expression**

| **gene** | **Analysis 1 (Unadjusted)** | | **Analysis 2 (Adjusted)** | |
|---|---|---|---|---|
| | **HR** | **p value** | **HR** | **Korn adj. p value** |
| MYBL2 | 2.4606679 | 0.0088333 | -- | ** |
| AURKB | 2.1954949 | 0.0070310 | -- | ** |
| BRCA2 | 1.9594455 | 0.0255585 | -- | ** |
| PTTG | 1.9428582 | 0.0110666 | -- | ** |
| KIFC1 | 1.8397539 | 0.0323052 | -- | ** |
| CDC20 | 1.7849698 | 0.0190490 | -- | ** |
| ESPL1 | 1.7654602 | 0.0254649 | -- | ** |
| DEPDC1 | 1.6955687 | 0.0089039 | -- | ** |
| EGFR | 1.6497619 | 0.0366391 | -- | ** |
| LAPTM4B | 1.5456666 | 0.0397772 | -- | ** |
| MMP12 | 1.463091 | 0.0376501 | -- | ** |

| | | | | |
|---|---|---|---|---|
| ** Korn adj. p value > 0.05 | | | | |

**Table 9B: (Hormone Receptor Negative (HR-), HER2 Positive (HER2+)) Genes with lower risk of recurrence with higher expression**

| **genes** | **Analysis 1 (Unadjusted)** | | **Analysis 2 (Adjusted)** | |
|---|---|---|---|---|
| | **HR** | **p value** | **HR** | **Korn adj. p value** |
| APOD | 0.7736676 | 0.0435824 | -- | ** |
| MUC1 | 0.7606705 | 0.0346312 | -- | ** |
| FOXA1 | 0.7438023 | 0.0130209 | -- | ** |
| GRB7 | 0.7054072 | 0.0039900 | -- | ** |
| SCUBE2 | 0.682751 | 0.0195569 | -- | ** |
| ERBB2 | 0.6675413 | 0.0191915 | -- | ** |
| TFF1 | 0.6380236 | 0.0039543 | 0.6383 | 0.00 |
| TPT1 | 0.6367527 | 0.0027398 | -- | ** |
| SEMA3F | 0.6309245 | 0.0472318 | -- | ** |
| GATA3 | 0.6225757 | 0.0295526 | -- | ** |
| ERBB4 | 0.6097751 | 0.0215173 | -- | ** |
| RAB27B | 0.6055422 | 0.0064456 | -- | ** |
| RHOB | 0.6008914 | 0.0436872 | -- | ** |
| TNFSF10 | 0.5863233 | 0.0011459 | -- | ** |
| KRT19 | 0.5577157 | 0.0000444 | -- | ** |
| PGR | 0.4937589 | 0.0303120 | -- | ** |
| TNFRSF10A | 0.4406017 | 0.0206329 | -- | ** |
| ABAT | 0.4372501 | 0.0008712 | -- | ** |
| MSH3 | 0.4368676 | 0.0143446 | -- | ** |
| ESR1 | 0.4104291 | 0.0022777 | 0.4173 | 0.000 |
| CHFR | 0.341955 | 0.0088551 | -- | ** |
| PIK3C2A | 0.3276976 | 0.0366853 | -- | ** |
| SLC25A3 | 0.246417 | 0.0168162 | -- | ** |
| CYP2C8 | 0.1471685 | 0.0237797 | -- | ** |
| HSPA1L | 0.047539 | 0.0341650 | -- | ** |

| | | | | |
|---|---|---|---|---|
| ** Korn adj. p value > 0.05 | | | | |

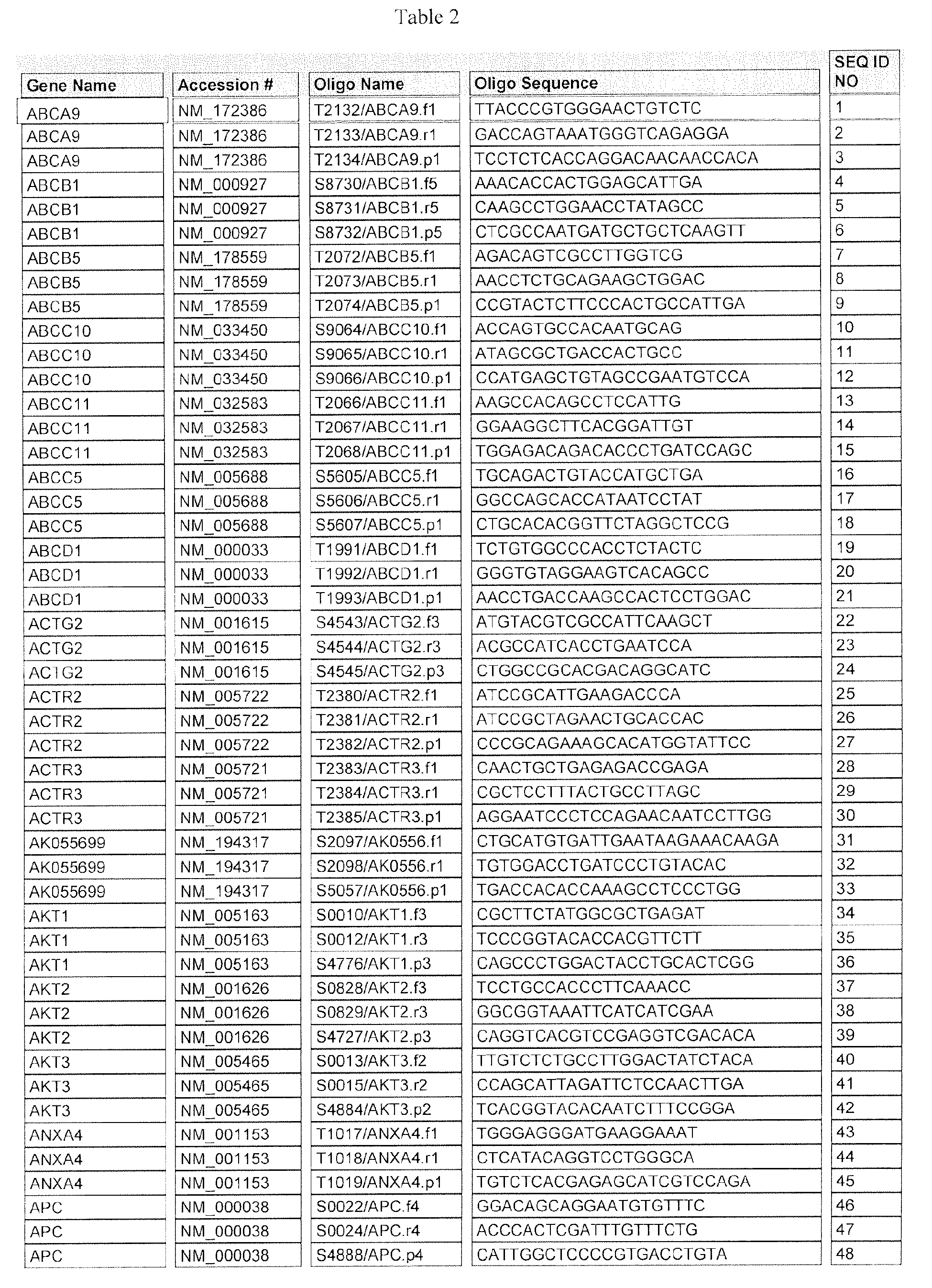

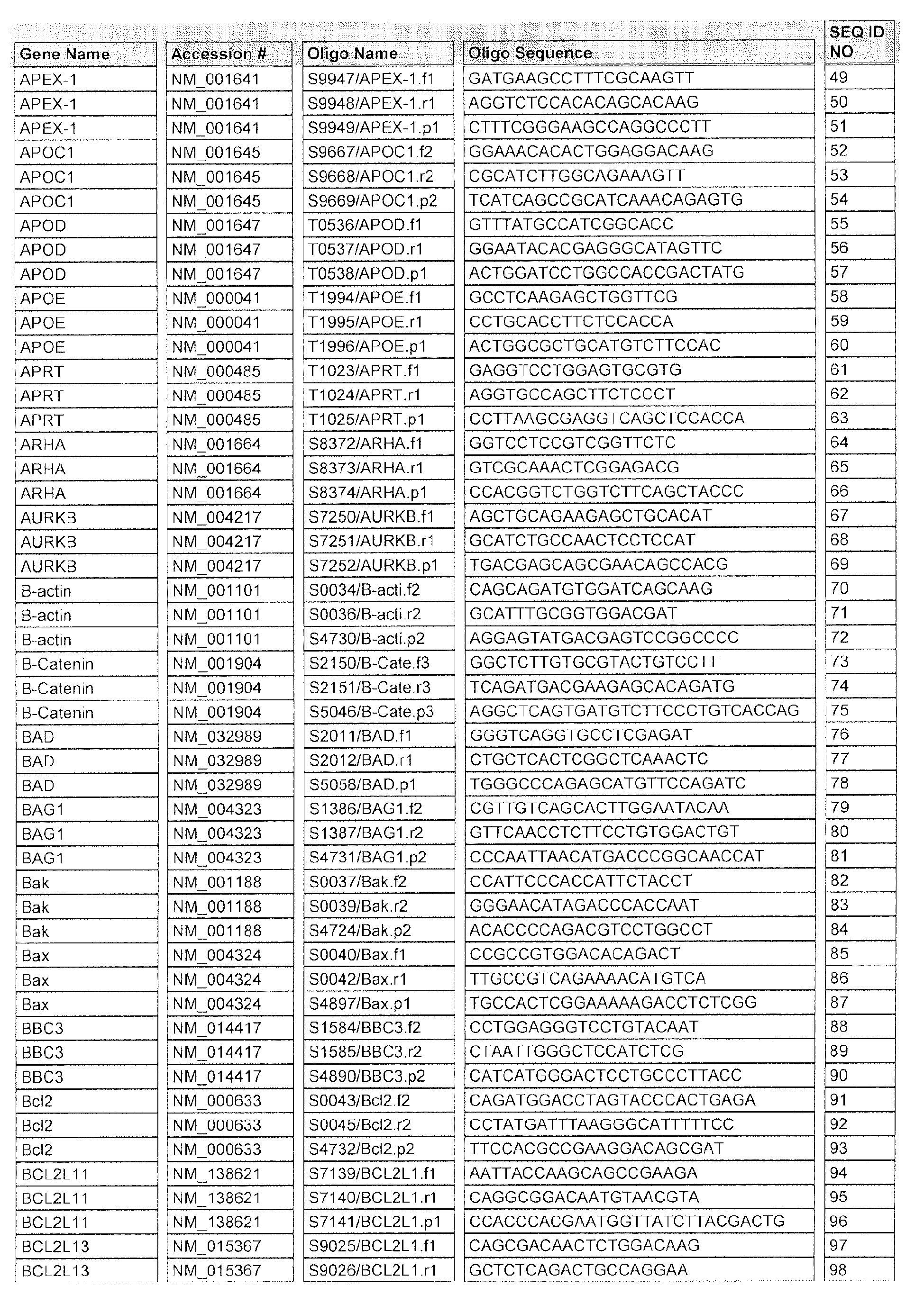

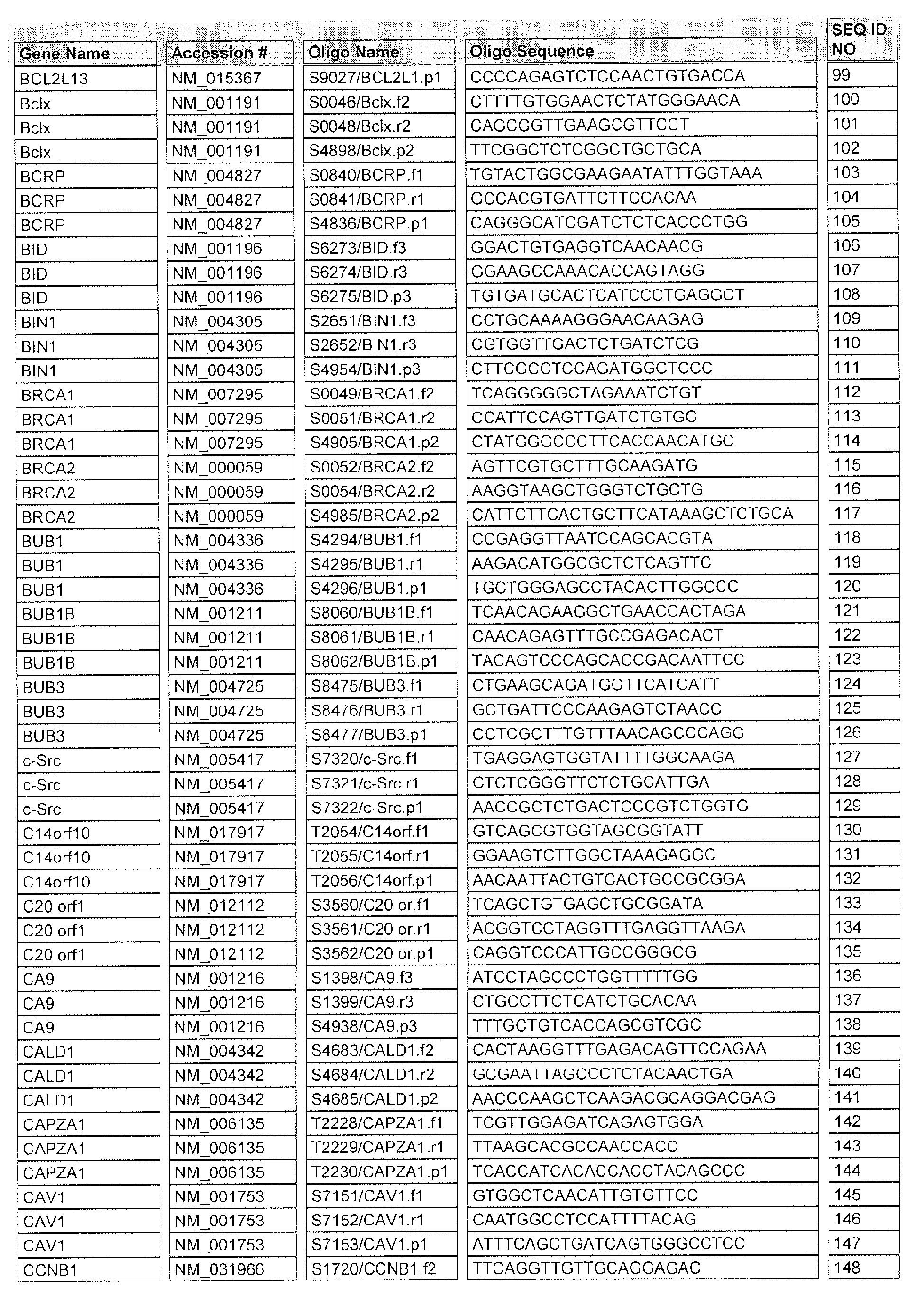

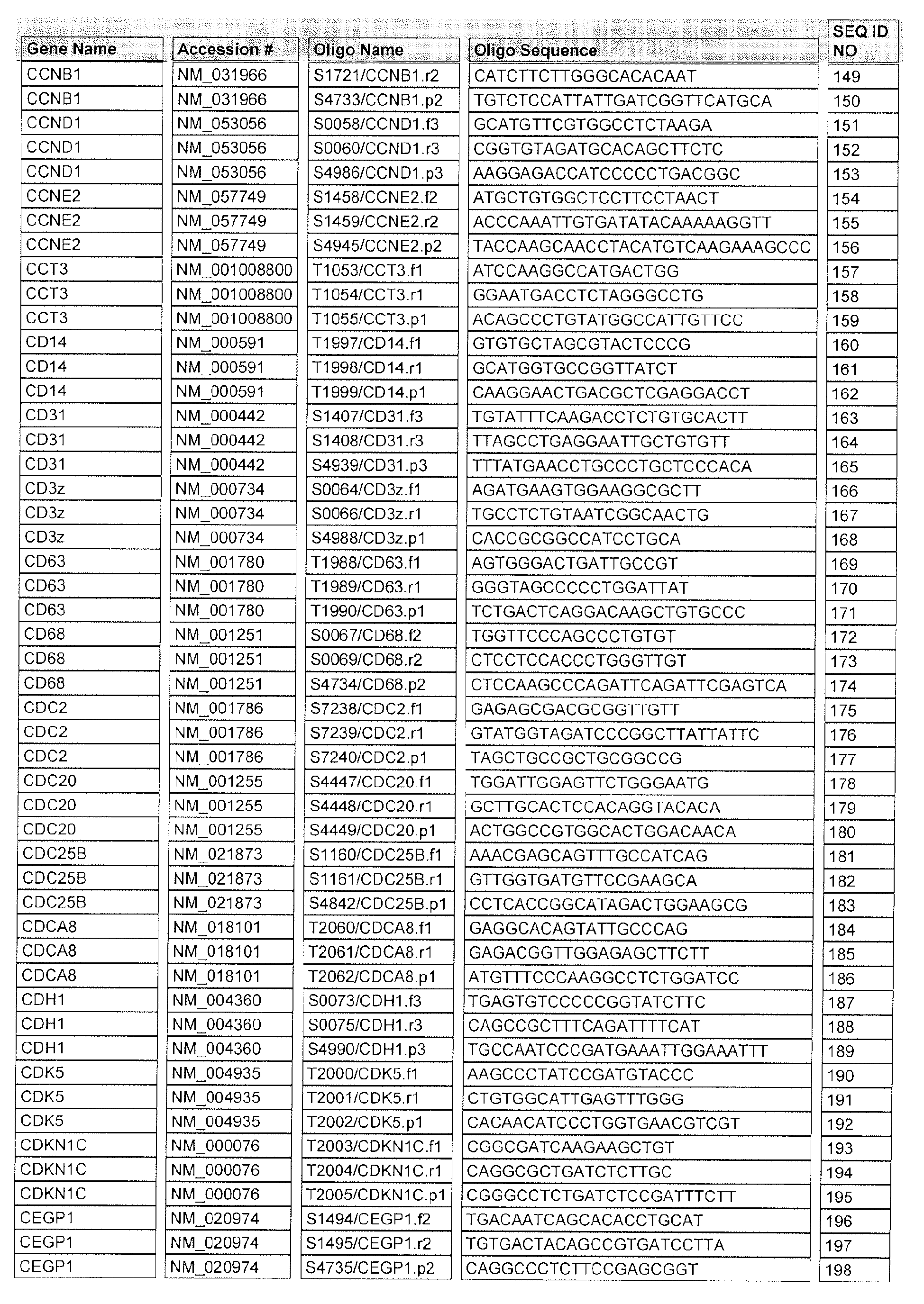

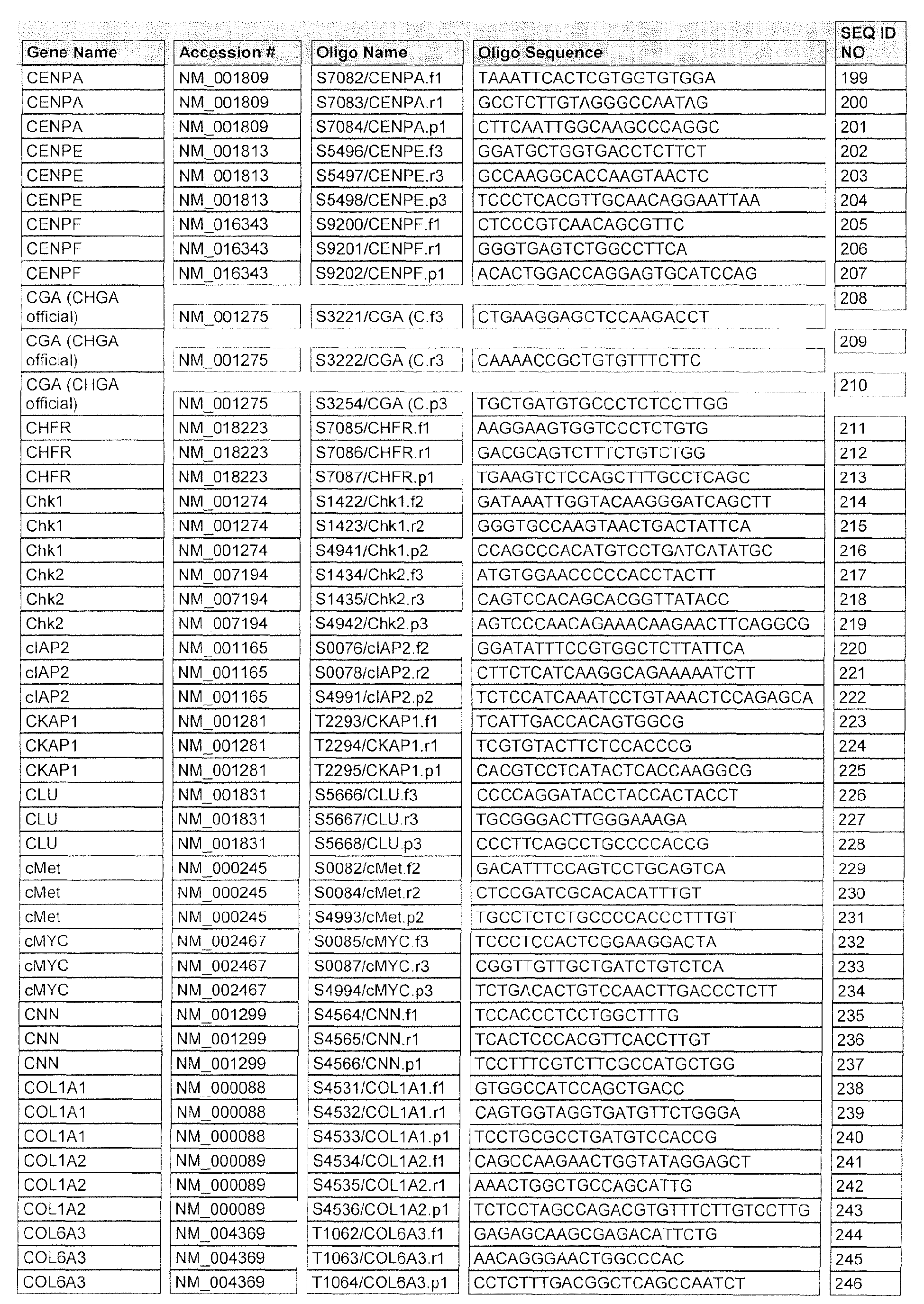

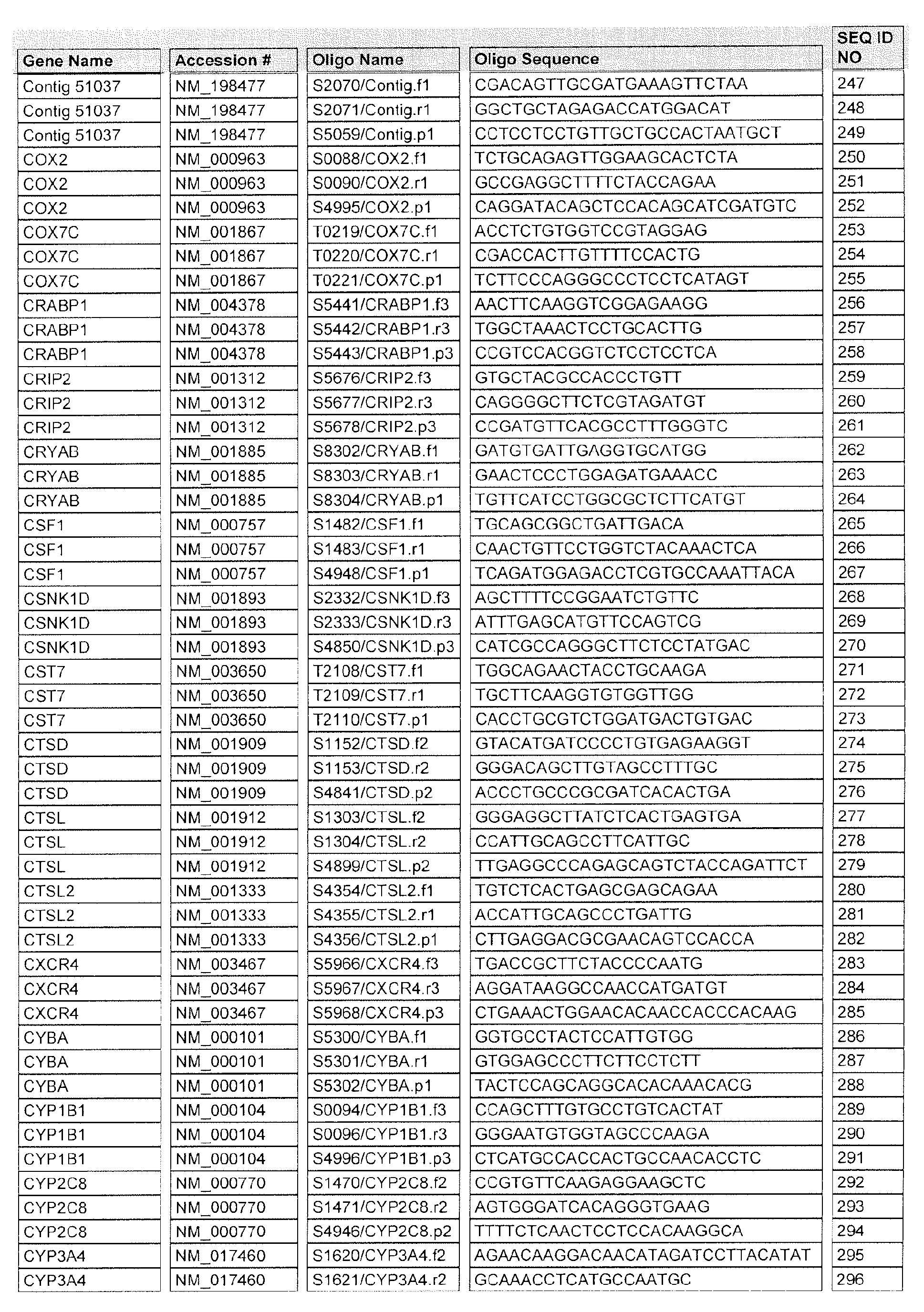

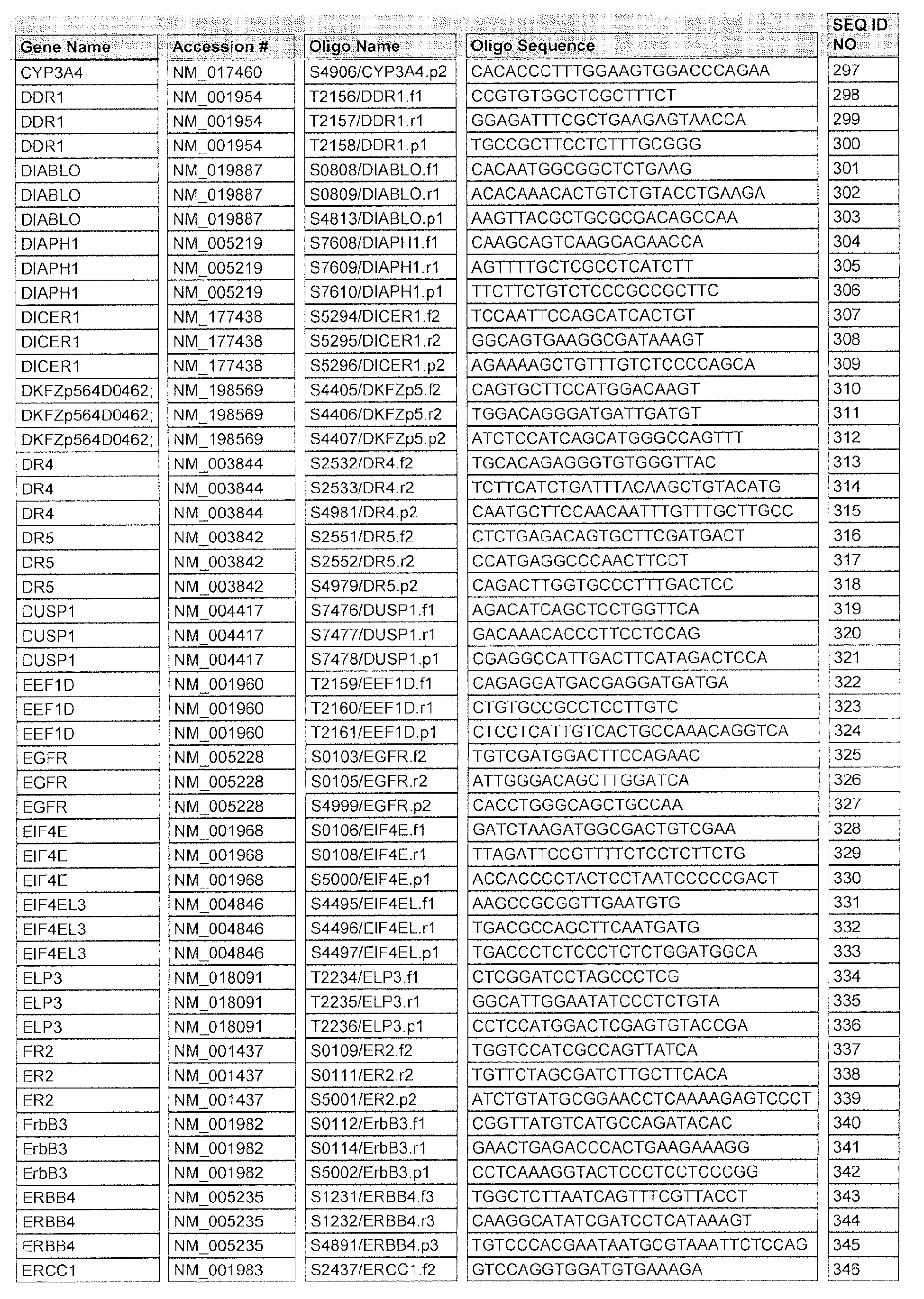

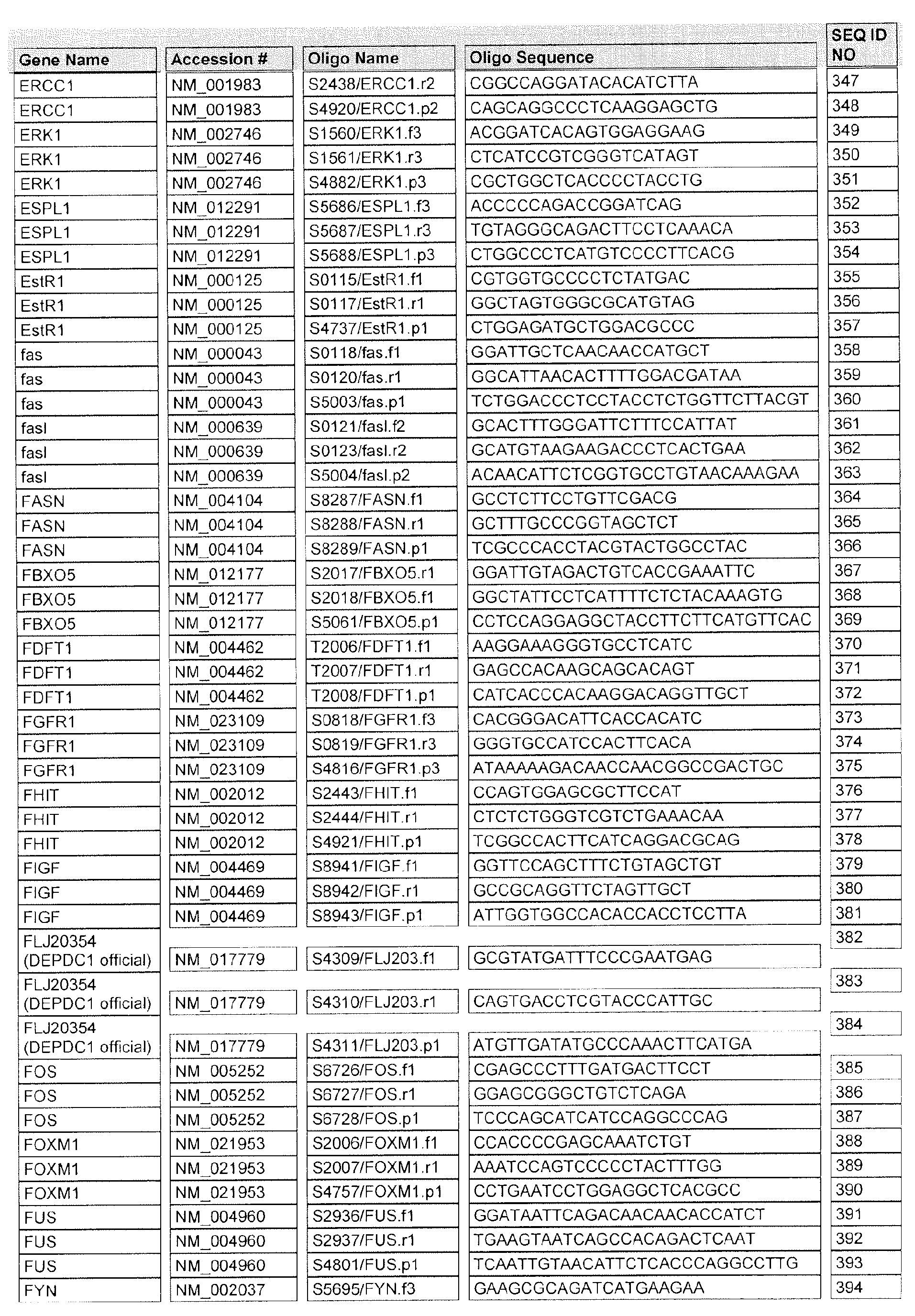

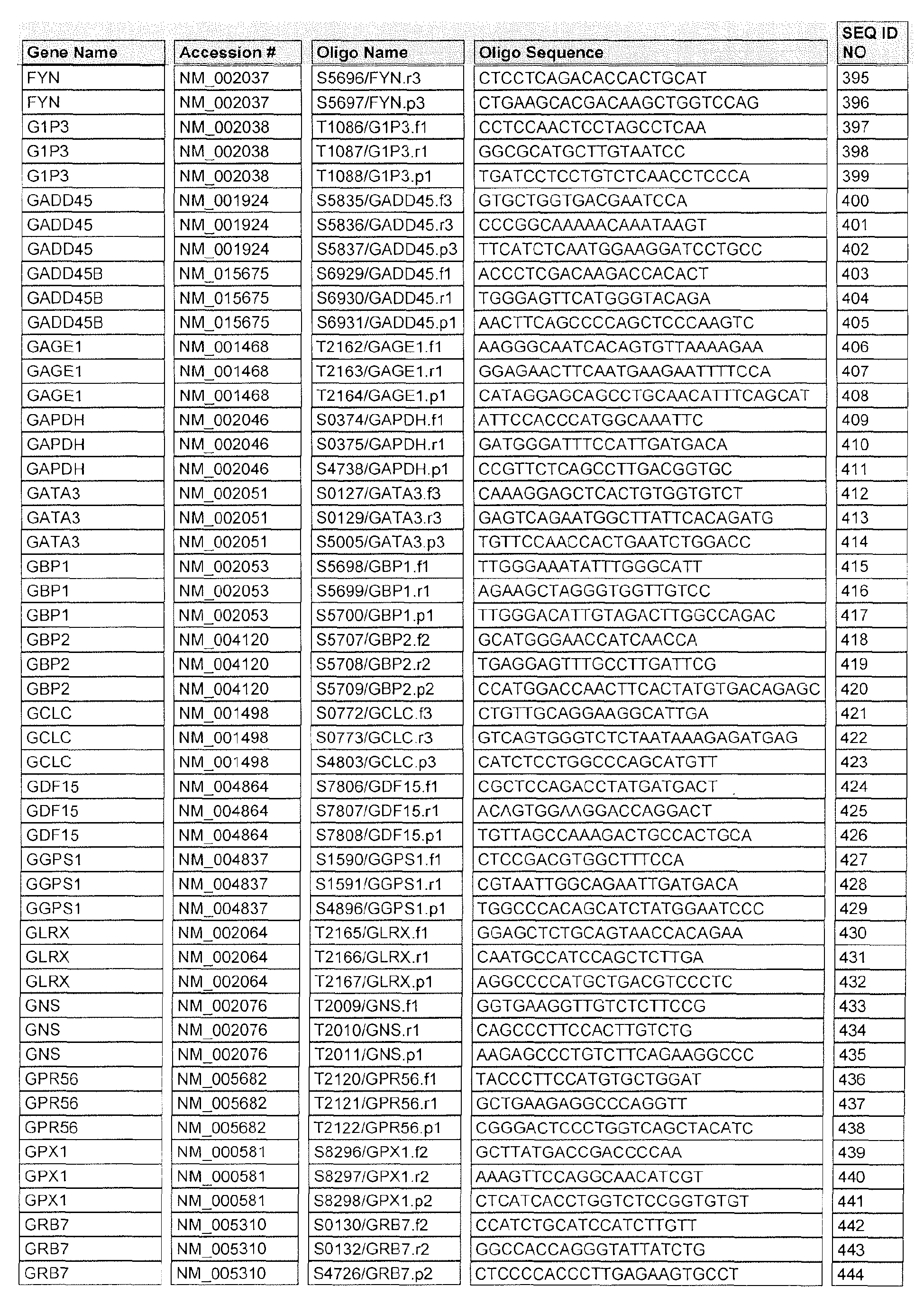

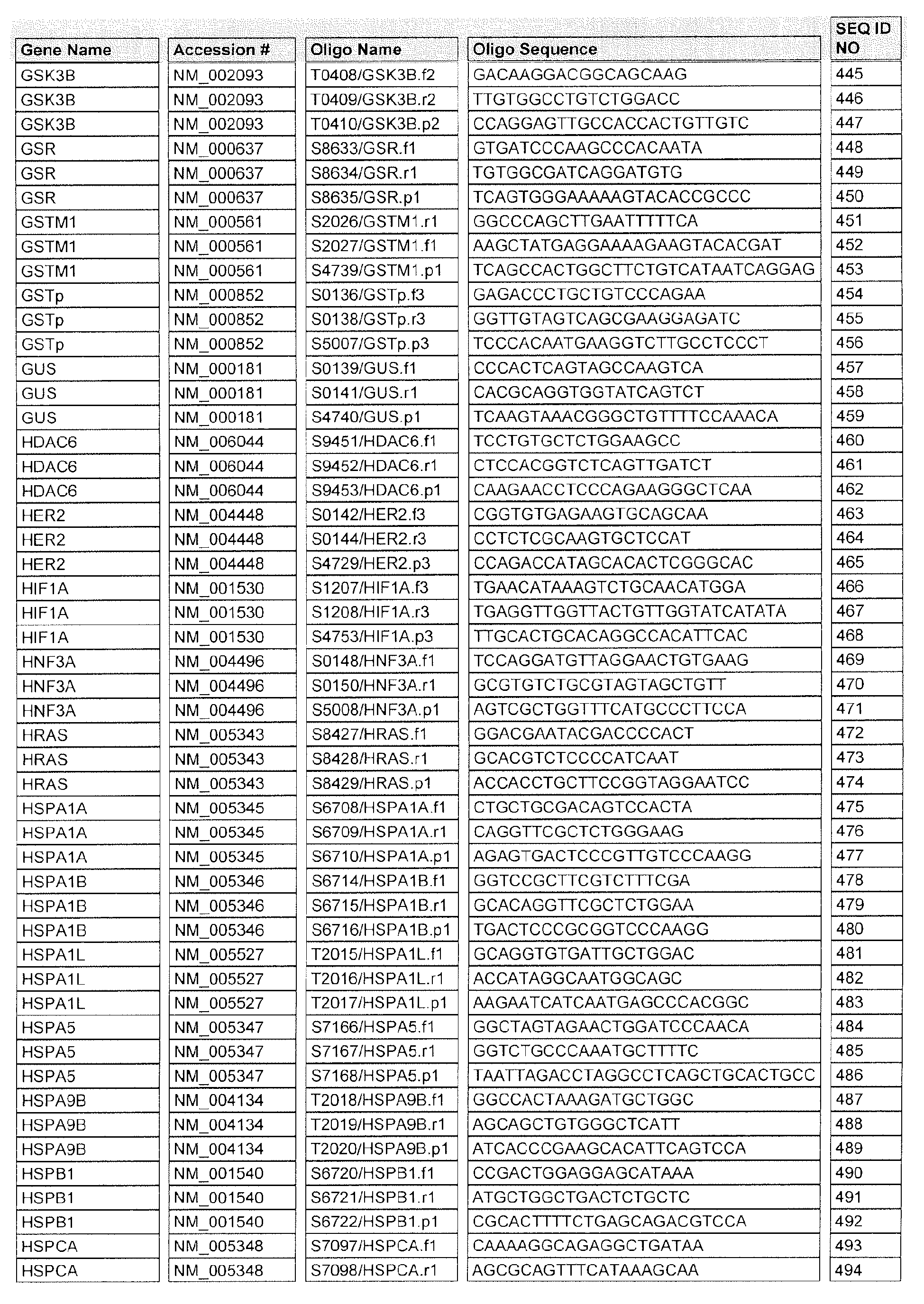

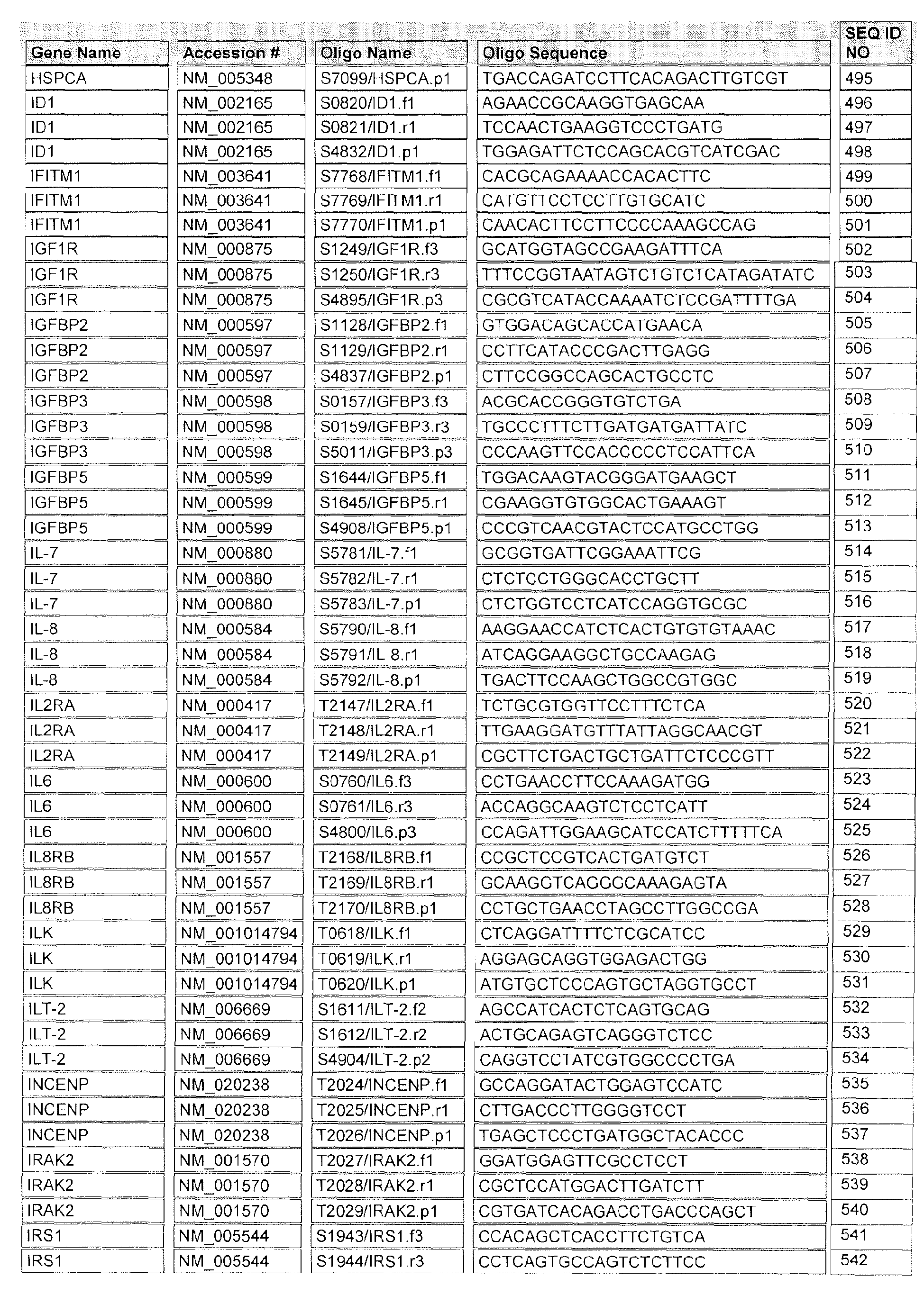

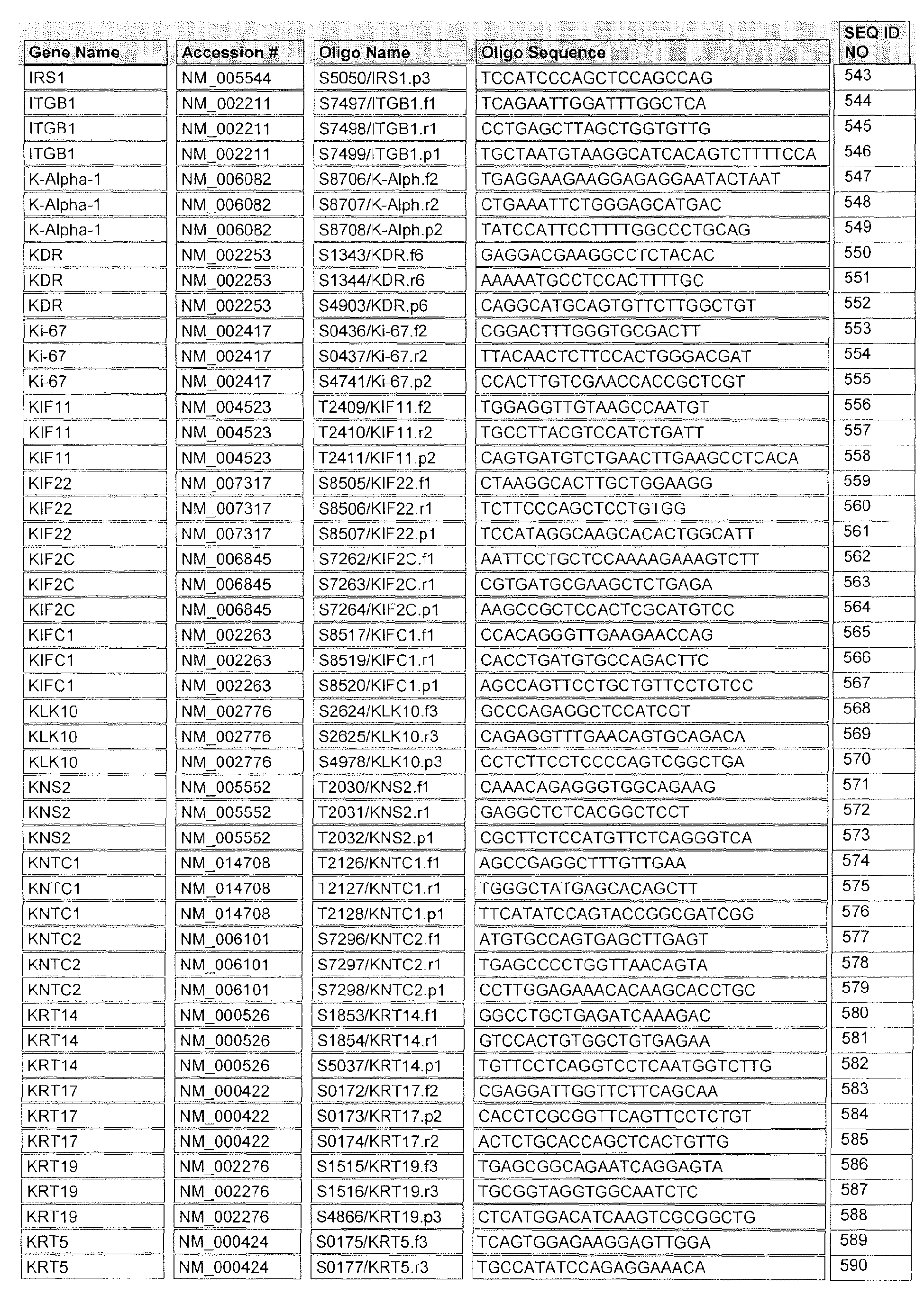

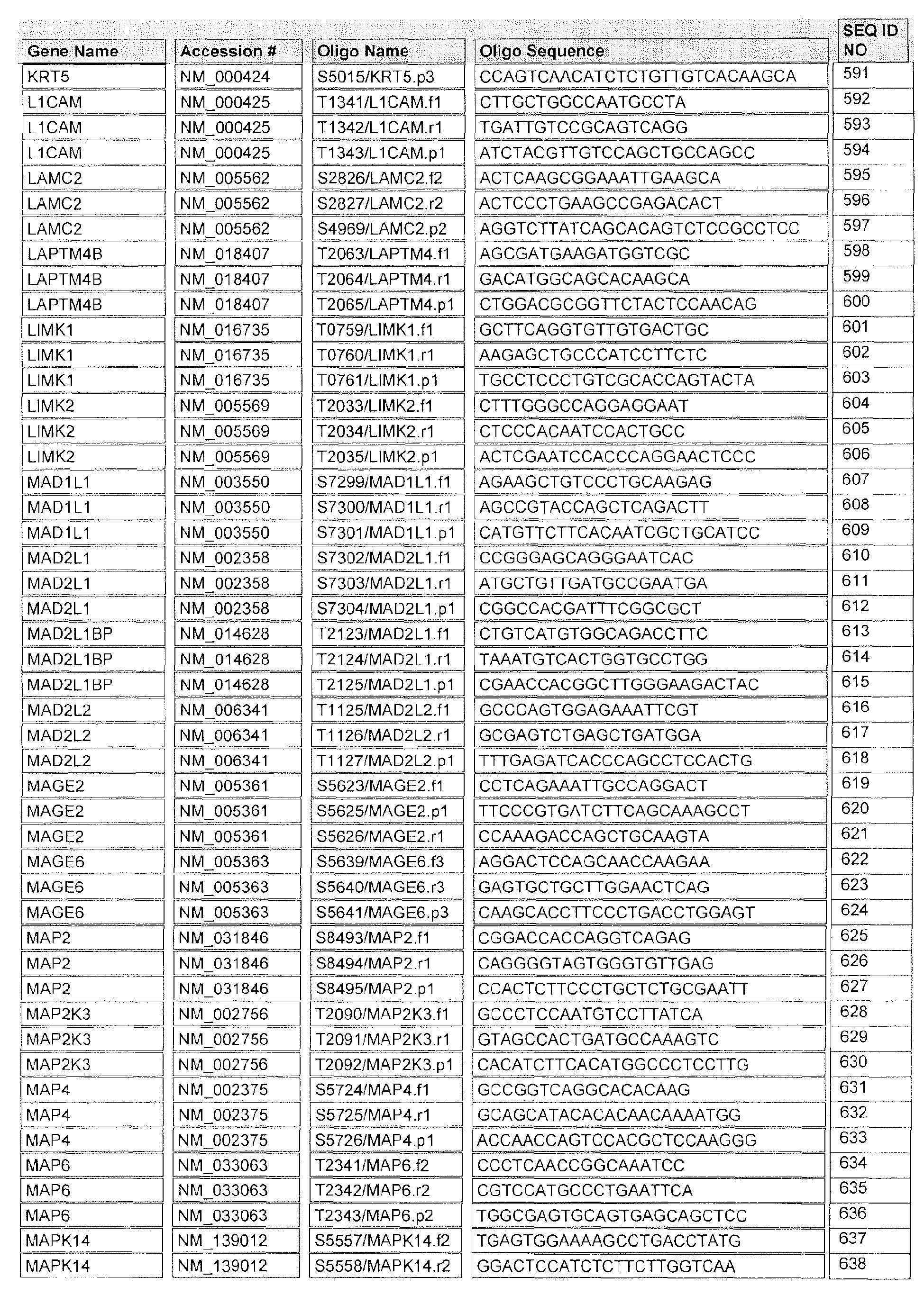

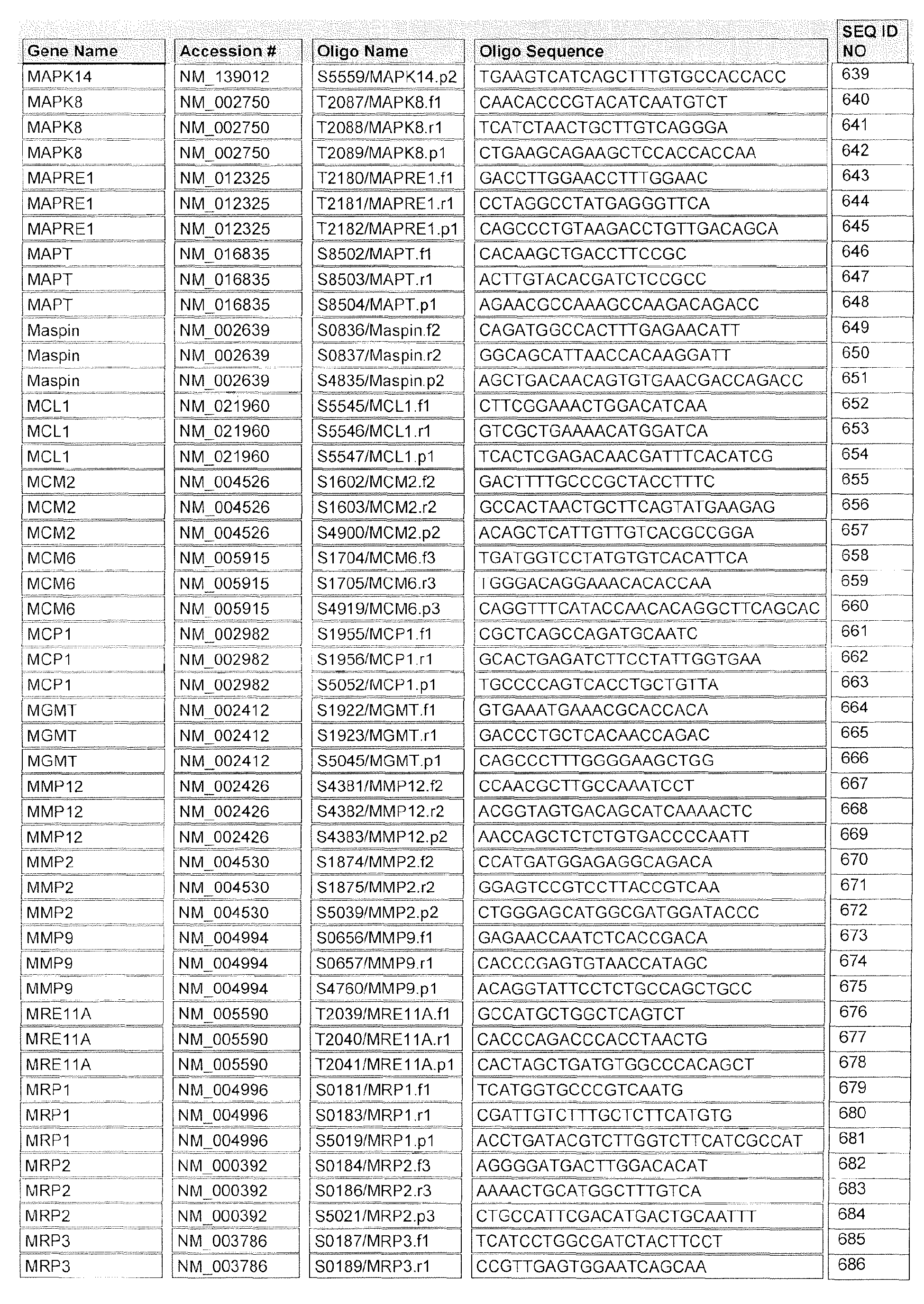

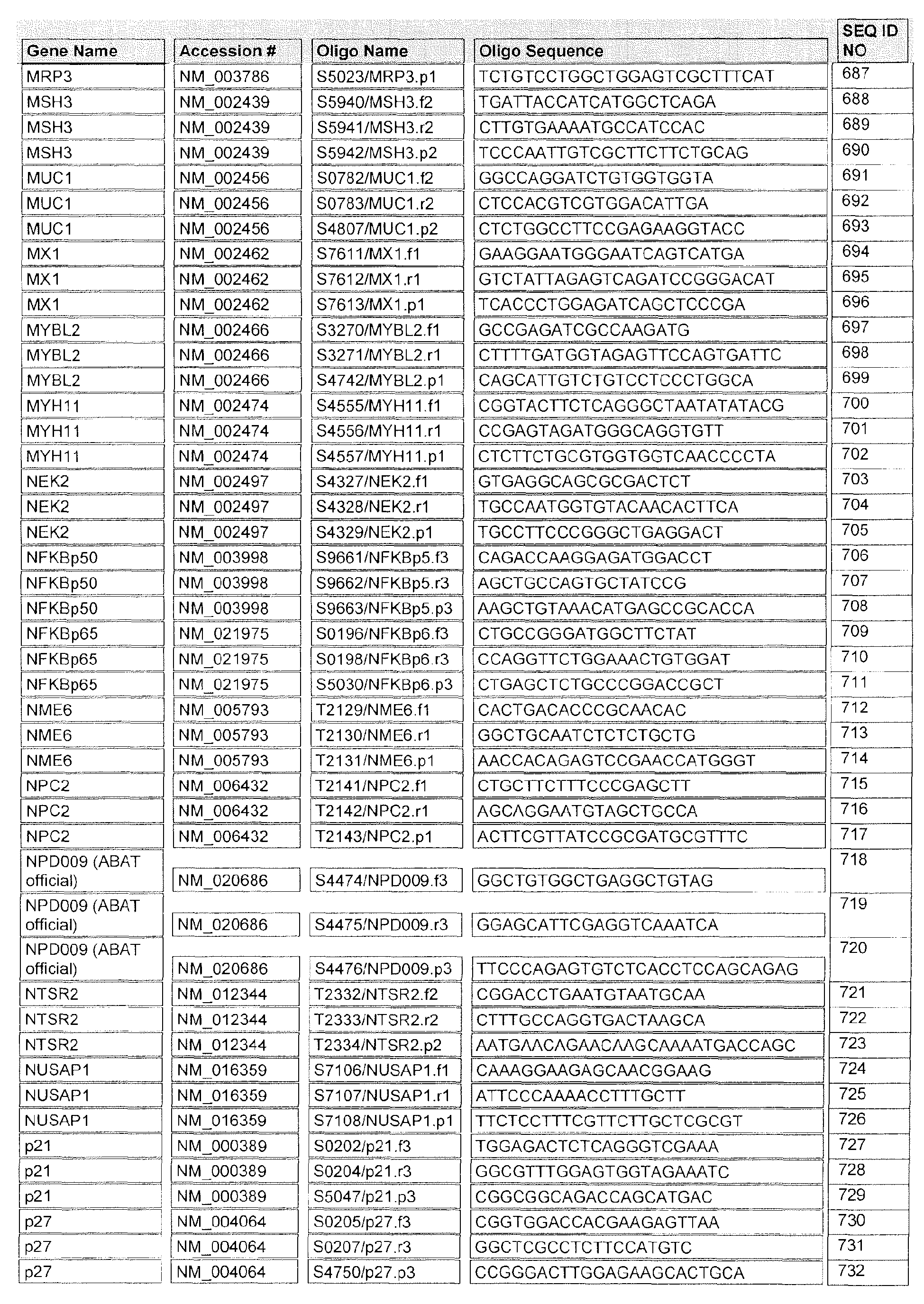

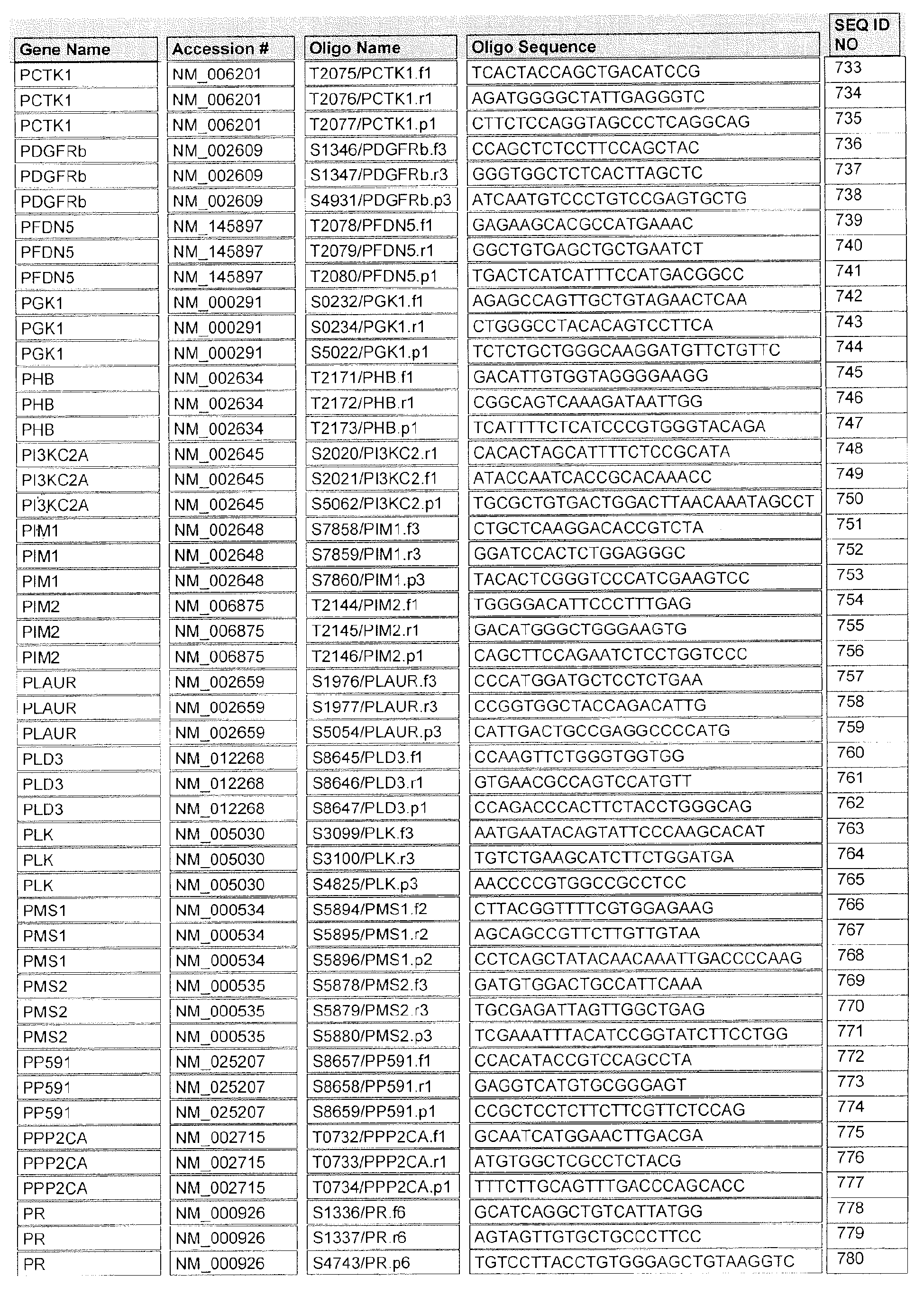

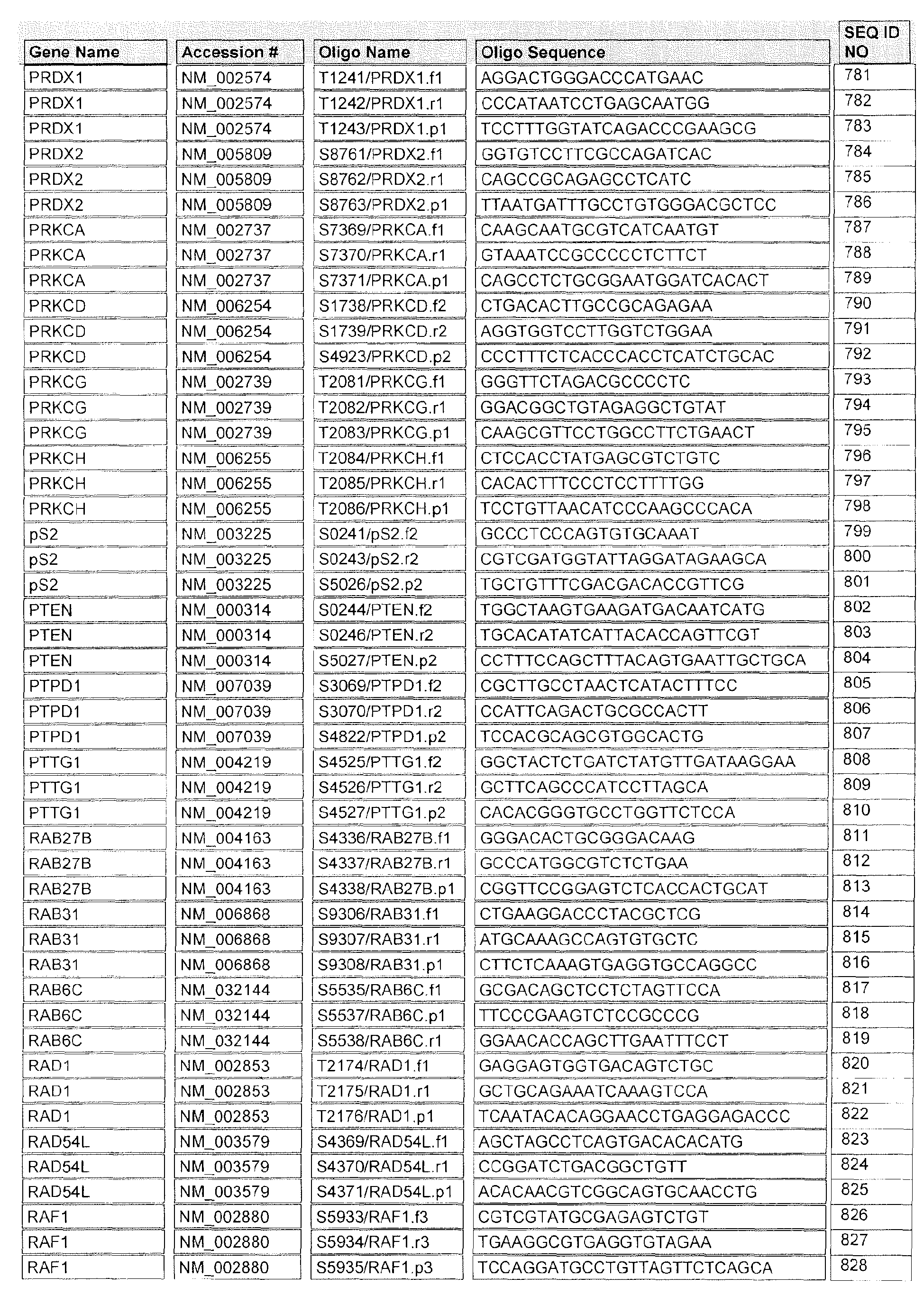

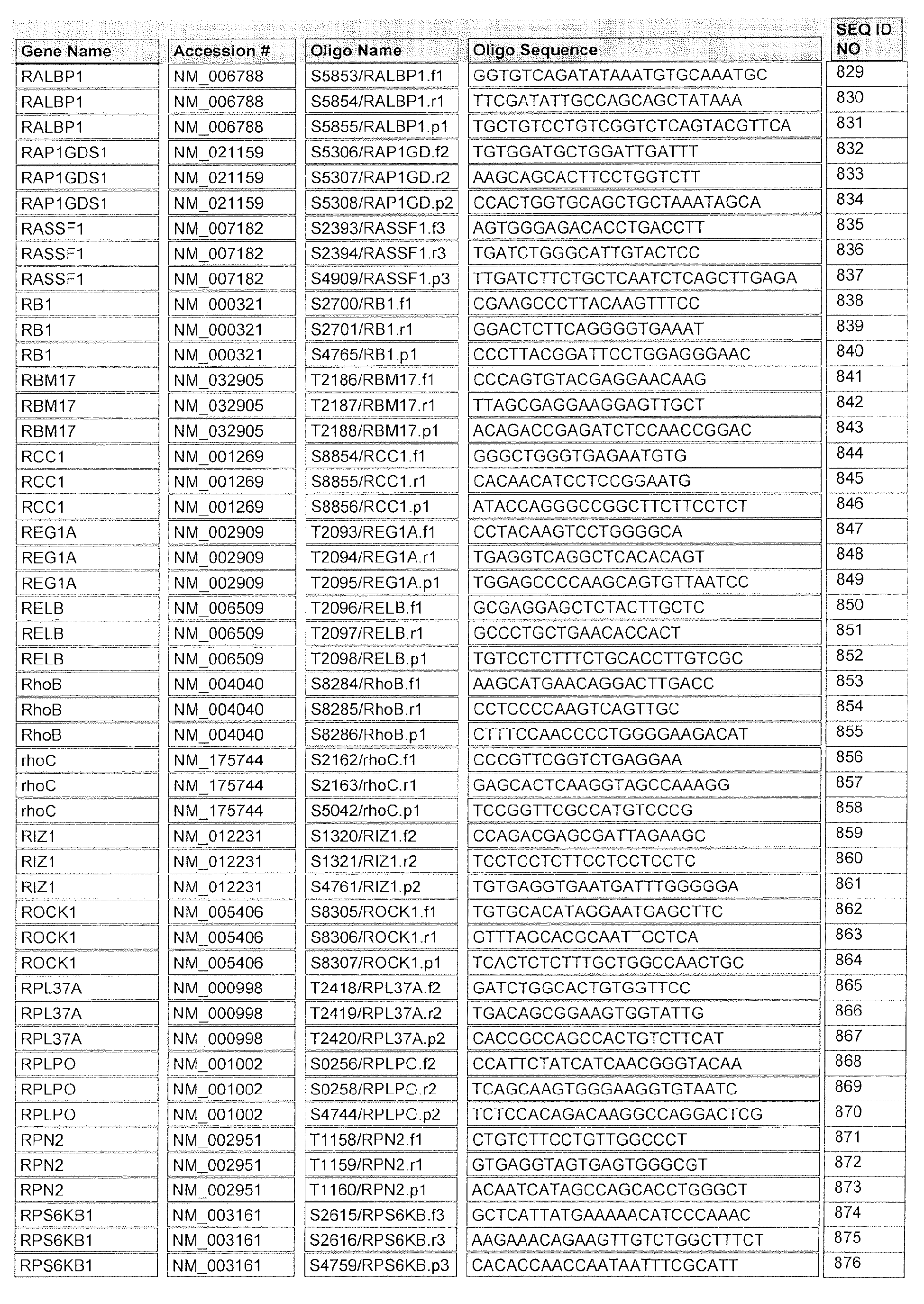

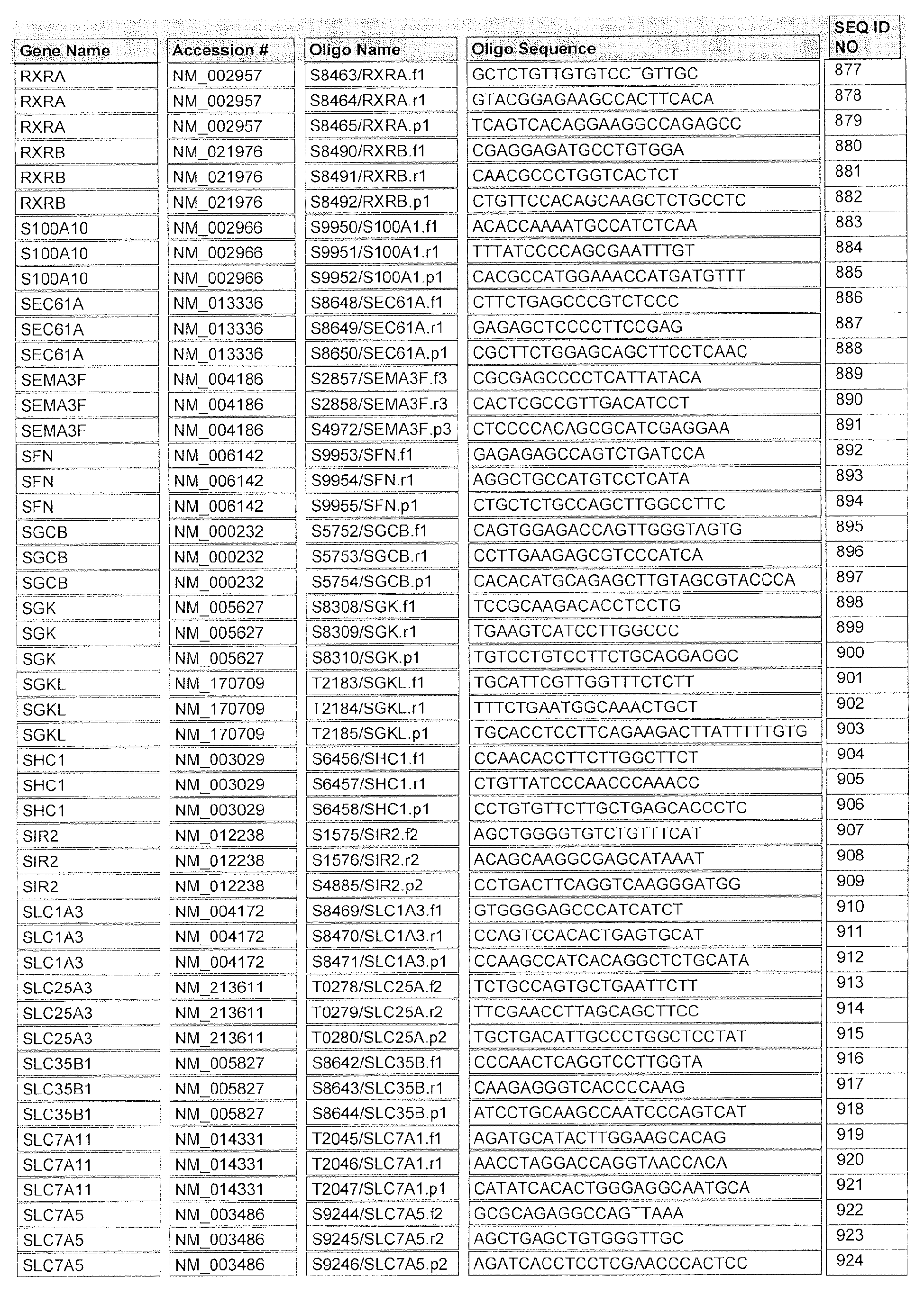

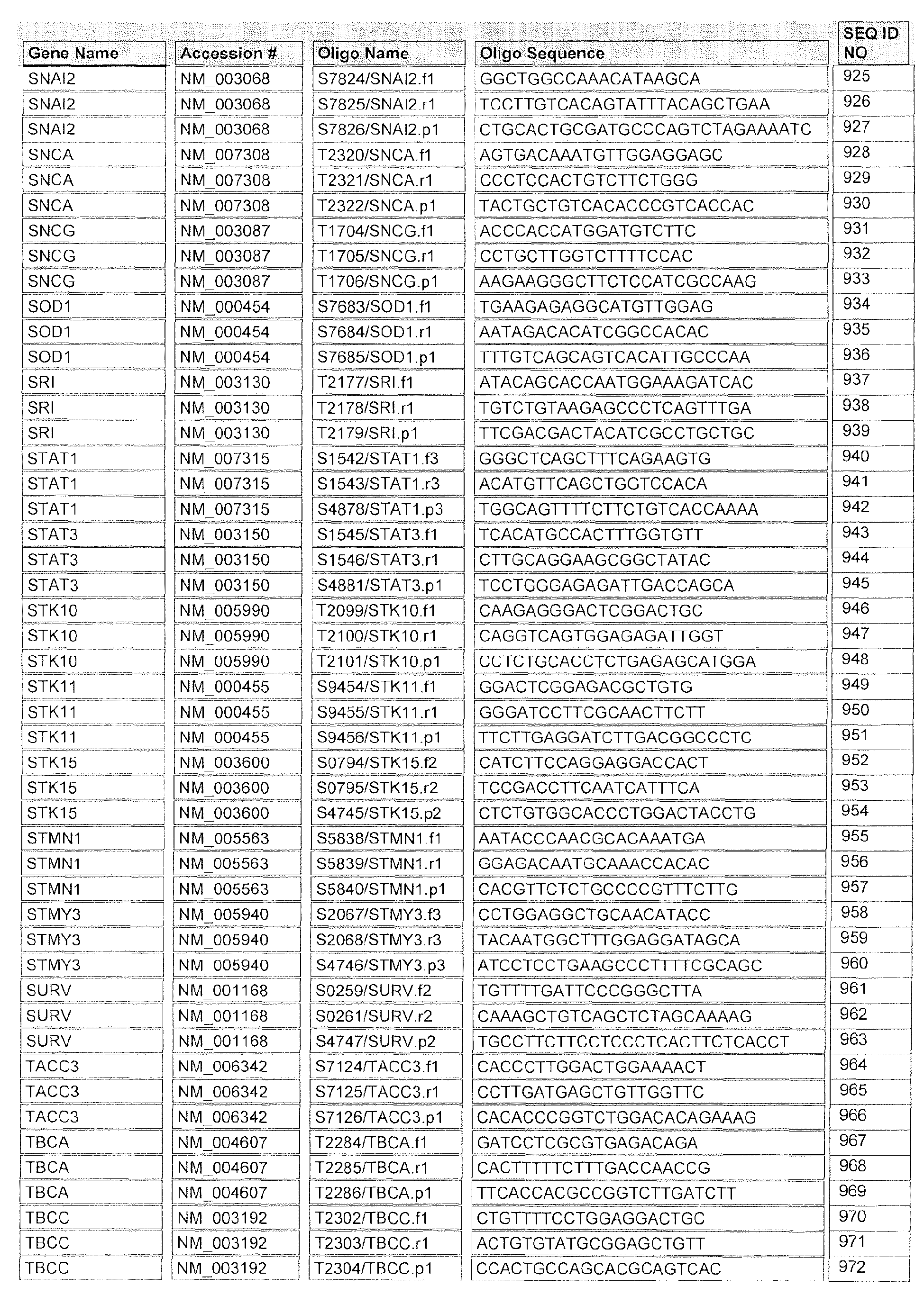

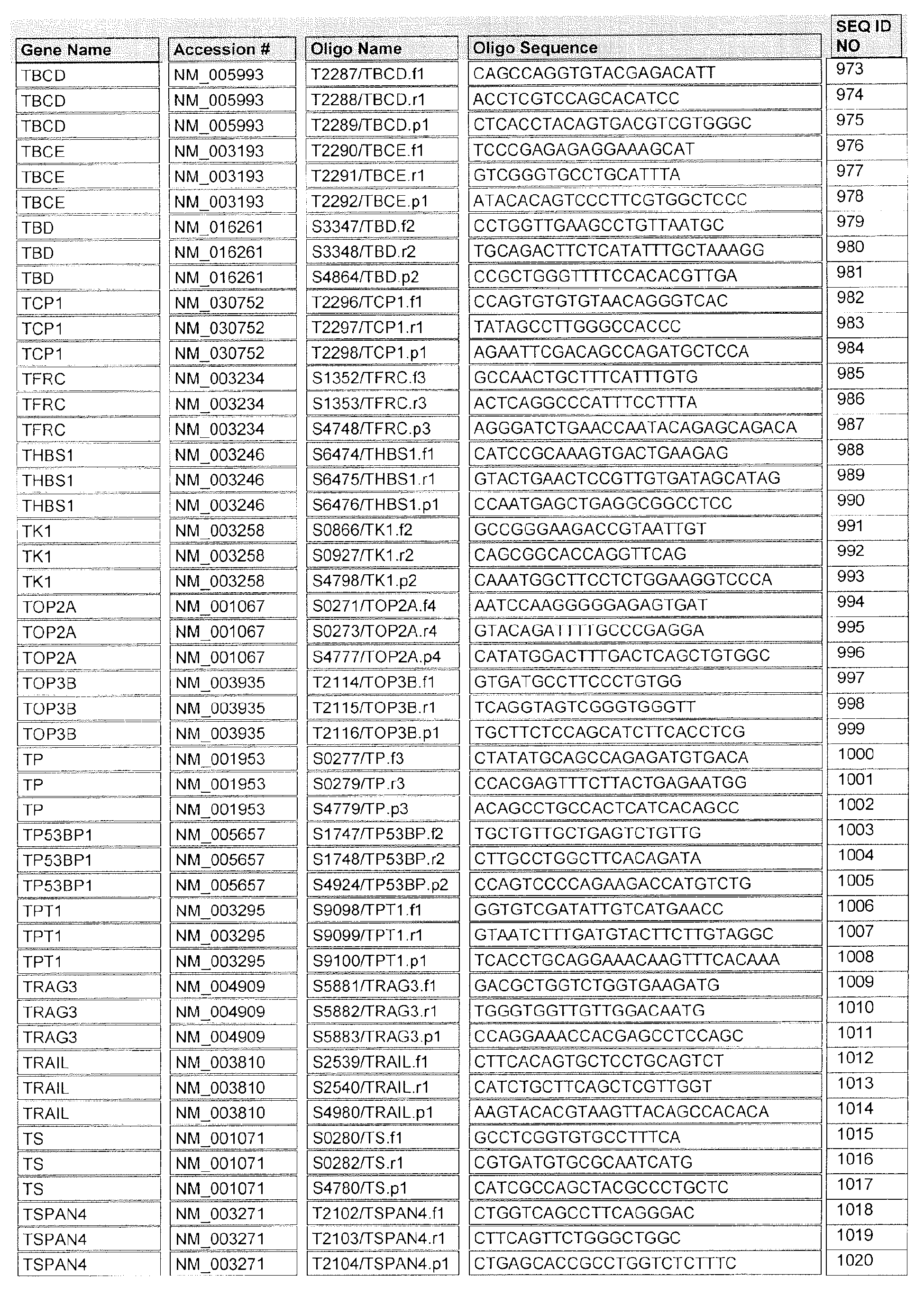

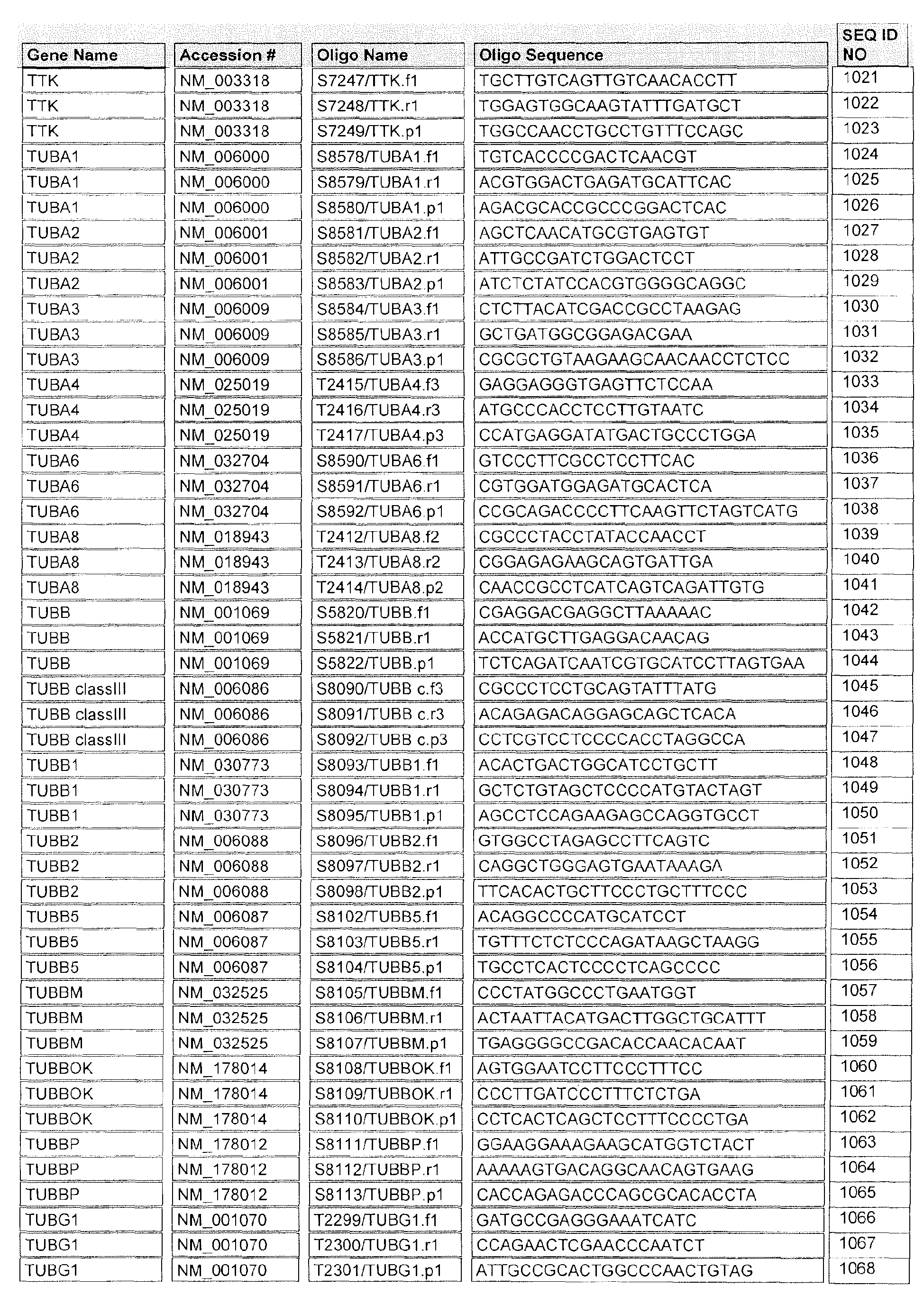

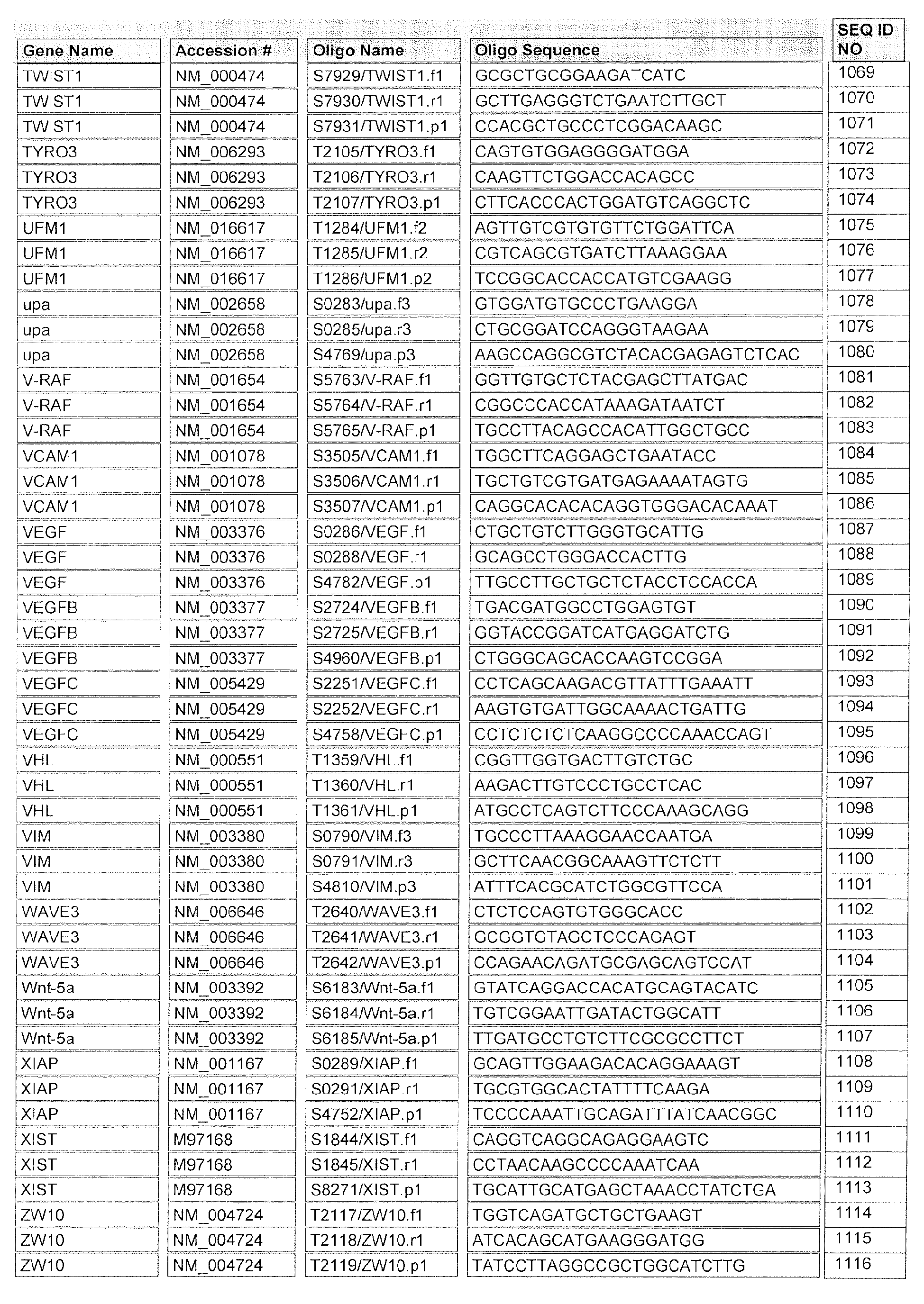

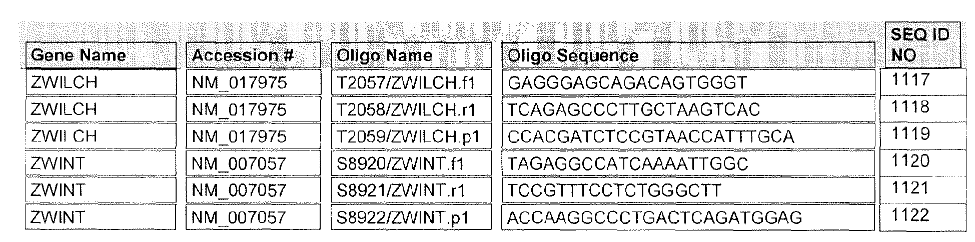

**Table 3**

| Gene Name | Access ion # | Amplicon Sequence | SEQ ID NO: |
|---|---|---|---|
| ABCA9 | NM_08 0283 | TTACCCGTGGGAACTGTCTCCAAATACATACTTCCTCTCACCAGGA CAACAACCACAGGATCCTCTGACCCATTTACTGGTC | 1123 |
| ABCB1 | NM_00 0927 | AAACACCACTGGAGCATTGACTACCAGGCTCGCCAATGATGCTGCT CAAGTTAAAGGGGCTATAGGTTCCAGGCTTG | 1124 |
| ABCB5 | NM_17 8559 | AGACAGTCGCCTTGGTCGGTCTCAATGGCAGTGGGAAGAGTACGG TAGTCCAGCTTCTGCAGAGGTT | 1125 |
| ABCC10 | NM_ 0334 50 | ACCAGTGCCACAATGCAGTGGCTGGACATTCGGCTACAGCTCATG GGGGCGGCAGTGGTCAGCGCTAT | 1126 |
| ABCC11 | NM_03 2583 | AAGCCACAGCCTCCATTGACATGGAGACAGACACCCTGATCCAGC GCACAATCCGTGAAGCCTTCC | 1127 |
| ABCC5 | NM_00 5688 | TGCAGACTGTACCATGCTGACCATTGCCCATCGCCTGCACACGGTT CTAGGCTCCGATAGGATTATGGTGCTGGCC | 1128 |
| ABCD1 | NM_00 0033 | TCTGTGGCCCACCTCTACTCCAACCTGACCAAGCCACTCCTGGAC GTGGCTGTGACTTCCTACACCC | 1129 |
| ACTG2 | NM_00 1615 | ATGTACGTCGCCATTCAAGCTGTGCTCTCCCTCTATGCCTCTGGCC GCACGACAGGCATCGTCCTGGATTCAGGTGATGGCGT | 1130 |
| ACTR2 | NM_00 5722 | ATCCGCATTGAAGACCCACCCCGCAGAAAGCACATGGTATTCCTG GGTGGTGCAGTTCTAGCGGAT | 1131 |
| ACTR3 | NM_00 5721 | CAACTGCTGAGAGACCGAGAAGTAGGAATCCCTCCAGAACAATCCT TGGAAACTGCTAAGGCAGTAAAGGAGCG | 1132 |
| AK055699 | NM_19 4317 | CTGCATGTGATTGAATAAGAAACAAGAAAGTGACCACACCAAAGCC TCCCTGGCTGGTGTACAGGGATCAGGTCCACA | 1133 |
| AKT1 | NM_00 5163 | CGCTTCTATGGCGCTGAGATTGTGTCAGCCCTGGACTACCTGCACT CGGAGAAGAACGTGGTGTACCGGGA | 1134 |
| AKT2 | NM_00 1626 | TCCTGCCACCCTTCAAACCTCAGGTCACGTCCGAGGTCGACACAA GGTACTTCGATGATGAATTTACCGCC | 1135 |
| AKT3 | NM_00 5465 | TTGTCTCTGCCTTGGACTATCTACATTCCGGAAAGATTGTGTACCGT GATCTCAAGTTGGAGAATCTAATGCTGG | 1136 |
| ANXA4 | NM_00 1153 | TGGGAGGGATGAAGGAAATTATCTGGACGATGCTCTCGTGAGACA GGATGCCCAGGACCTGTATGAG | 1137 |
| APC | NM_00 0038 | GGACAGCAGGAATGTGTTTCTCCATACAGGTCACGGGGAGCCAAT GGTTCAGAAACAAATCGAGTGGGT | 1138 |
| APEX-1 | NM_00 1641 | GATGAAGCCTTTCGCAAGTTCCTGAAGGGCCTGGCTTCCCGAAAG CCCCTTGTGCTGTGTGGAGACCT | 1139 |
| APOC1 | NM_00 1645 | GGAAACACACTGGAGGACAAGGCTCGGGAACTCATCAGCCGCATC AAACAGAGTGAACTTTCTGCCAAGATGCG | 1140 |
| APOD | NM_00 1647 | GTTTATGCCATCGGCACCGTACTGGATCCTGGCCACCGACTATGA GAACTATGCCCTCGTGTATTCC | 1141 |
| APOE | NM_00 0041 | GCCTCAAGAGCTGGTTCGAGCCCCTGGTGGAAGACATGCAGCGCC AGTGGGCCGGGCTGGTGGAGAAGGTGCAGG | 1142 |
| APRT | NM_00 0485 | GAGGTCCTGGAGTGCGTGAGCCTGGTGGAGCTGACCTCGCTTAAG GGCAGGGAGAAGCTGGCACCT | 1143 |
| ARHA | NM_00 1664 | GGTCCTCCGTCGGTTCTCTCATTAGTCCACGGTCTGGTCTTCAGCT ACCCGCCTTCGTCTCCGAGTTTGCGAC | 1144 |
| AURKB | NM_00 4217 | AGCTGCAGAAGAGCTGCACATTTGACGAGCAGCGAACAGCCACGA TCATGGAGGAGTTGGCAGATGC | 1145 |
| B-actin | NM_00 1101 | CAGCAGATGTGGATCAGCAAGCAGGAGTATGACGAGTCCGGCCCC TCCATCGTCCACCGCAAATGC | 1146 |
| BAD | NM_03 2989 | GGGTCAGGTGCCTCGAGATCGGGCTTGGGCCCAGAGCATGTTCCA GATCCCAGAGTTTGAGCCGAGTGAGCAG | 1147 |
| BAG1 | NM_00 4323 | CGTTGTCAGCACTTGGAATACAAGATGGTTGCCGGGTCATGTTAAT TGGGAAAAAGAACAGTCCACAGGAAGAGGTTGAAC | 1148 |
| Bak | NM_00 1188 | CCATTCCCACCATTCTACCTGAGGCCAGGACGTCTGGGGTGTGGG GATTGGTGGGTCTATGTTCCC | 1149 |
| Bax | NM_00 4324 | CCGCCGTGGACACAGACTCCCCCCGAGAGGTCTTTTCCGAGTGG CAGCTGACATGTTTTCTGACGGCAA | 1150 |
| BBC3 | NM_01 4417 | CCTGGAGGGTCCTGTACAATCTCATCATGGGACTCCTGCCCTTACC CAGGGGCCACAGAGCCCCCGAGATGGAGCCCAATTAG | 1151 |
| B-Catenin | NM_00 1904 | GGCTCTTGTGCGTACTGTCCTTCGGGCTGGTGACAGGGAAGACAT CACTGAGCCTGCCATCTGTGCTCTTCGTCATCTGA | 1152 |
| Bcl2 | NM_00 0633 | CAGATGGACCTAGTACCCACTGAGATTTCCACGCCGAAGGACAGC GATGGGAAAAATGCCCTTAAATCATAGG | 1153 |
| BCL2L11 | NM_13 8621 | AATTACCAAGCAGCCGAAGACCACCCACGAATGGTTATCTTACGAC TGTTACGTTACATTGTCCGCCTG | 1154 |
| BCL2L13 | NM_01 5367 | CAGCGACAACTCTGGACAAGTCAGTCCCCCAGAGTCTCCAACTGT GACCACTTCCTGGCAGTCTGAGAGC | 1155 |
| Bclx | N M_00 1191 | CTTTTGTGGAACTCTATGGGAACAATGCAGCAGCCGAGAGCCGAA AGGGCCAGGAACGCTTCAACCGCTG | 1156 |
| BCRP | NM_00 4827 | TGTACTGGCGAAGAATATTTGGTAAAGCAGGGCATCGATCTCTCAC CCTGGGGCTTGTGGAAGAATCACGTGGC | 1157 |
| BID | N M_00 1196 | GGACTGTGAGGTCAACAACGGTTCCAGCCTCAGGGATGAGTGCAT CACAAACCTACTGGTGTTTGGCTTCC | 1158 |
| BIN1 | NM_00 4305 | CCTGCAAAAGGAACAAGAGCCCTTCGCCTCCAGATGGCTCCCCT GCCGCCACCCCCGAGATCAGAGTCAACCACG | 1159 |
| BRCA1 | NM_00 7295 | TCAGGGGGCTAGAAATCTGTTGCTATGGGCCCTTCACCAACATGCC CACAGATCAACTGGAATGG | 1160 |
| BRCA2 | NM_00 0059 | AGTTCGTGCTTTGCAAGATGGTGCAGAGCTTTATGAAGCAGTGAAG AATGCAGCAGACCCAGCTTACCTT | 1161 |
| BUB1 | NM_00 4336 | CCGAGGTTAATCCAGCACGTATGGGGCCAAGTGTAGGCTCCCAGC AGGAACTGAGAGCGCCATGTCTT | 1162 |
| BUB1B | NM_00 1211 | TCAACAGAAGGCTGAACCACTAGAAAGACTACAGTCCCAGCACCG ACAATTCCAAGCTCGAGTGTCTCGGCAAACTCTGTTG | 1163 |
| BUB3 | NM_00 4725 | CTGAAGCAGATGGTTCATCATTTCCTGGGCTGTTAAACAAAGCGAG GTTAAGGTTAGACTCTTGGGAATCAGC | 1164 |
| C14orf10 | NM_01 7917 | GTCAGCGTGGTAGCGGTATTCTCCGCGGCAGTGACAGTAATTGTTT TTGCCTCTTTAGCCAAGACTTCC | 1165 |
| C20_orf1 | NM_01 2112 | TCAGCTGTGAGCTGCGGATACCGCCCGGCAATGGACCTGCTCTT AACCTCAAACCTAGGACCGT | 1166 |
| CA9 | NM_00 1216 | ATCCTAGCCCTGGTTTTTGGCCTCCTTTTTGCTGTCACCAGCGTCG CGTTCCTTGTGCAGATGAGAAGGCAG | 1167 |
| CALD | NM_00 4342 | CACTAAGGTTTGAGACAGTTCCAGAAAGAACCCAAGCTCAAGACGC AGGACGAGCTCAGTTGTAGAGGGCTAATTCGC | 1168 |
| CAPZA1 | NM_00 6135 | TCGTTGGAGATCAGAGTGGAAGTTCACCATCACACCACCTACAGCC CAGGTGGTTGGCGTGCTTAA | 1169 |
| CAV1 | N M_00 1753 | GTGGCTCAACATTGTGTTCCCATTTCAGCTGATCAGTGGGCCTCCA AGGAGGGGCTGTAAAATGGAGGCCATTG | 1170 |
| CCNB1 | N M_03 1966 | TTCAGGTTGTTGCAGGAGACCATGTACATGACTGTCTCCATTATTG ATCGGTTCATGCAGAATAATTGTGTGCCCAAGAAGATG | 1171 |
| CCND1 | NM 05 | GCATGTTCGTGGCCTCTAAGATGAAGGAGACCATCCCCCTGACGG | 1172 |
| | 3056 | CCGAGAAGCTGTGCATCTACACCG | |
| CCNE2 | NM_05 7749 | ATGCTGTGGCTCCTTCCTAACTGGGGCTTTCTTGACATGTAGGTTG CTTGGTAATAACCTTTTTGTATATCACAATTTGGGT | 1173 |
| CCT3 | N M_00 1008800 | ATCCAAGGCCATGACTGGTGTGGAACAATGGCCATACAGGGCTGT TGCCCAGGCCCTAGAGGTCATTCC | 1174 |
| CD14 | NM_00 0591 | GTGTGCTAGCGTACTCCCGCCTCAAGGAACTGACGCTCGAGGACC TAAAGATAACCGGCACCATGC | 1175 |
| CD31 | NM_00 0442 | TGTATTTCAAGACCTCTGTGCACTTATTTATGAACCTGCCCTGCTCC CACAGAACACAGCAATTCCTCAGGCTAA | 1176 |
| CD3z | NM_00 0734 | AGATGAAGTGGAAGGCGCTTTTCACCGCGGCCATCCTGCAGGCAC AGTTGCCGATTACAGAGGCA | 1177 |
| CD63 | NM_00 1780 | AGTGGGACTGATTGCCGTGGGTGTCGGGGCACAGCTTGTCCTGAG TCAGACCATAATCCAGGGGGCTACCC | 1178 |
| CD68 | NM_00 1251 | TGGTTCCCAGCCCTGTGTCCACCTCCAAGCCCAGATTCAGATTCGA GTCATGTACACAACCCAGGGTGGAGGAG | 1179 |
| CDC2 | N M_00 1786 | GAGAGCGACGCGGTTGTTGTAGCTGCCGCTGCGGCCGCCGCGGA ATAATAAGCCGGGATCTACCATAC | 1180 |
| CDC20 | NM_00 1255 | TGGATTGGAGTTCTGGGAATGTACTGGCCGTGGCACTGGACAACA GTGTGTACCTGTGGAGTGCAAGC | 1181 |
| CDC25B | N M_02 1873 | AAACGAGCAGTTTGCCATCAGACGCTTCCAGTCTATGCCGGTGAG GCTGCTGGGCCACAGCCCCGTGCTTCGGAACATCACCAAC | 1182 |
| CDCA8 | NM_01 8101 | GAGGCACAGTATTGCCCAGCTGGATCCAGAGGCCTTGGAAACAT TAAGAAGCTCTCCAACCGTCTC | 1183 |
| CDH1 | NM_00 4360 | TGAGTGTCCCCCGGTATCTTCCCCGCCCTGCCAATCCCGATGAAAT TGGAAATTTTATTGATGAAAATCTGAAAGCGGCTG | 1184 |
| CDK5 | NM_00 4935 | AAGCCCTATCCGATGTACCCGGCCACAACATCCCTGGTGAACGTC GTGCCCAAACTCAATGCCACAG | 1185 |
| CDKN1C | NM_00 0076 | CGGCGATCAAGAAGCTGTCCGGGCCTCTGATCTCCGATTTCTTCG CCAAGCGCAAGAGATCAGCGCCTG | 1186 |
| CEGP1 | NM_02 0974 | TGACAATCAGCACACCTGCATTCACCGCTCGGAAGAGGGCCTGAG CTGCATGAATAAGGATCACGGCTGTAGTCACA | 1187 |
| CENPA | NM_00 1809 | TAAATTCACTCGTGGTGTGGACTTCAATTGGCAAGCCCAGGCCCTA TTGGCCCTACAAGAGGC | 1188 |
| CENPE | NM_00 1813 | GGATGCTGGTGACCTCTTCTTCCCTCACGTTGCAACAGGAATTAAA GGCTAAAAGAAAACGAAGAGTTACTTGGTGCCTTGGC | 1189 |
| CENPF | NM_01 6343 | CTCCCGTCAACAGCGTTCTTTCCAAACACTGGACCAGGAGTGCATC CAGATGAAGGCCAGACTCACCC | 1190 |
| CGA(CHGA official) | NM_00 1275 | CTGAAGGAGCTCCAAGACCTCGCTCTCCAAGGCGCCAAGGAGAGG GCACATCAGCAGAAGAAACACAGCGGTTTTG | 1191 |
| CHFR | NM_01 8223 | AAGGAAGTGGTCCCTCTGTGGCAAGTGATGAAGTCTCCAGCTTTGC CTCAGCTCTCCCAGACAGAAAGACTGCGTC | 1192 |
| Chk1 | NM_00 1274 | GATAAATTGGTACAAGGGATCAGCTTTTCCCAGCCCACATGTCCTG ATCATATGCTTTTGAATAGTCAGTTACTTGGCACCC | 1193 |
| Chk2 | NM_00 7194 | ATGTGGAACCCCCACCTACTTGGCGCCTGAAGTTCTTGTTTCTGTT GGGACTGCTGGGTATAACCGTGCTGTGGACTG | 1194 |
| clAP2 | NM_00 1165 | GGATATTTCCGTGGCTCTTATTCAAACTCTCCATCAAATCCTGTAAA CTCCAGAGCAAATCAAGATTTTTCTGCCTTGATGAGAAG | 1195 |
| CKAP1 | NM_00 1281 | TCATTGACCACAGTGGCGCCCGCCTTGGTGAGTATGAGGACGTGT CCCGGGTGGAGAAGTACACGA | 1196 |
| CLU | NM_00 1831 | CCCCAGGATACCTACCACTACCTGCCCTTCAGCCTGCCCCACCGG AGGCCTCACTTCTTCTTTCCCAAGTCCCGCA | 1197 |
| cMet | NM_00 0245 | GACATTTCCAGTCCTGCAGTCAATGCCTCTCTGCCCCACCCTTTGT TCAGTGTGGCTGGTGCCACGACAAATGTGTGCGATCGGAG | 1198 |
| cMYC | NM_00 2467 | TCCCTCCACTCGGAAGGACTATCCTGCTGCCAAGAGGGTCAAGTT GGACAGTGTCAGAGTCCTGAGACAGATCAGCAACAACCG | 1199 |
| CNN | NM_00 | TCCACCCTCCTGGCTTTGGCCAGCATGGCGAAGACGAAAGGAAAC | 1200 |
| | 1299 | AAGGTGAACGTGGGAGTGA | |
| COL1A1 | NM_00 0088 | GTGGCCATCCAGCTGACCTTCCTGCGCCTGATGTCCACCGAGGCC TCCCAGAACATCACCTACCACTG | 1201 |
| COL1A2 | NM_00 0089 | CAGCCAAGAACTGGTATAGGAGCTCCAAGGACAAGAAACACGTCT GGCTAGGAGAAACTATCAATGCTGGCAGCCAGTTT | 1202 |
| COL6A3 | NM_00 4369 | GAGAGCAAGCGAGACATTCTGTTCCTCTTTGACGGCTCAGCCAATC TTGTGGGCCAGTTCCCTGTT | 1203 |
| Contig 51037 | NM_19 8477 | CGACAGTTGCGATGAAAGTTCTAATCTCTTCCCTCCTCCTGTTGCT GCCACTAATGCTGATGTCCATGGTCTCTAGCAGCC | 1204 |
| COX2 | NM_00 0963 | TCTGCAGAGTTGGAAGCACTCTATGGTGACATCGATGCTGTGGAG CTGTATCCTGCCCTTCTGGTAGAAAAGCCTCGGC | 1205 |
| COX7C | NM_00 1867 | ACCTCTGTGGTCCGTAGGAGCCACTATGAGGAGGGCCCTGGGAAG AATTTGCCATTTTCAGTGGAAAACAAGTGGTCG | 1206 |
| CRABP1 | NM_00 4378 | AACTTCAAGGTCGGAGAAGGCTTTGAGGAGGAGACCGTGGACGGA CGCAAGTGCAGGAGTTTAGCCA | 1207 |
| CRIP2 | NM_00 1312 | GTGCTACGCCACCCTGTTCGGACCCAAAGGCGTGAACATCGGGGG CGCGGGCTCCTACATCTACGAGAAGCCCCTG | 1208 |
| CRYAB | NM_00 1885 | GATGTGATTGAGGTGCATGGAAAACATGAAGAGCGCCAGGATGAA CATGGTTTCATCTCCAGGGAGTTC | 1209 |
| CSF1 | NM_00 0757 | TGCAGCGGCTGATTGACAGTCAGATGGAGACCTCGTGCCAAATTA CATTTGAGTTTGTAGACCAGGAACAGTTG | 1210 |
| CSNK1 D | NM_00 1893 | AGCTTTTCCGGAATCTGTTCCATCGCCAGGGCTTCTCCTATGACTA CGTGTTCGACTGGAACATGCTCAAAT | 1211 |
| CST7 | NM_00 3650 | TGGCAGAACTACCTGCAAGAAAAACCAGCACCTGCGTCTGGATGA CTGTGACTTCCAAACCAACCACACCTTGAAGCA | 1212 |
| CTSD | NM_00 1909 | GTACATGATCCCCTGTGAGAAGGTGTCCACCCTGCCCGCGATCAC ACTGAAGCTGGGAGGCAAAGGCTACAAGCTGTCCC | 1213 |
| CTSL | NM_00 1912 | GGGAGGCTTATCTCACTGAGTGAGCAGAATCTGGTAGACTGCTCT GGGCCTCAAGGCAATGAAGGCTGCAATGG | 1214 |
| CTSL2 | NM_00 1333 | TGTCTCACTGAGCGAGCAGAATCTGGTGGACTGTTCGCGTCCTCAA GGCAATCAGGGCTGCAATGGT | 1215 |
| CXCR4 | NM_00 3467 | TGACCGCTTCTACCCCAATGACTTGTGGGTGGTTGTGTTCCAGTTT CAGCACATCATGGTTGGCCTTATCCT | 1216 |
| CYBA | NM_00 0101 | GGTGCCTACTCCATTGTGGCGGGCGTGTTTGTGTGCCTGCTGGAG TACCCCCGGGGGAAGAGGAAGAAGGGCTCCAC | 1217 |
| CYP1B1 | NM_00 0104 | CCAGCTTTGTGCCTGTCACTATTCCTCATGCCACCACTGCCAACAC CTCTGTCTTGGGCTACCACATTCCC | 1218 |
| CYP2C8 | NM_00 0770 | CCGTGTTCAAGAGGAAGCTCACTGCCTTGTGGAGGAGTTGAGAAA AACCAAGGCTTCACCCTGTGATCCCACT | 1219 |
| CYP3A4 | NM_01 7460 | AGAACAAGGACAACATAGATCCTTACATATACACACCCTTTGGAAG TGGACCCAGAAACTGCATTGGCATGAGGTTTGC | 1220 |
| DDR1 | NM_00 1954 | CCGTGTGGCTCGCTTTCTGCAGTGCCGCTTCCTCTTTGCGGGGCC CTGGTTACTCTTCAGCGAAATCTCC | 1221 |
| DIABLO | NM_01 9887 | CACAATGGCGGCTCTGAAGAGTTGGCTGTCGCGCAGCGTAACTTC ATTCTTCAGGTACAGACAGTGTTTGTGT | 1222 |
| DIAPH1 | NM_00 5219 | CAAGCAGTCAAGGAGAACCAGAAGCGGCGGGAGACAGAAGAAAA GATGAGGCGAGCAAAACT | 1223 |
| DICER1 | NM_17 7438 | TCCAATTCCAGCATCACTGTGGAGAAAAGCTGTTTGTCTCCCCAGC ATACTTTATCGCCTTCACTGCC | 1224 |
| DKFZp564D 0462; | NM_19 8569 | CAGTGCTTCCATGGACAAGTCCTTGTCAAAACTGGCCCATGCTGAT GGAGATCAAACATCAATCATCCCTGTCCA | 1225 |
| DR4 | NM_00 3844 | TGCACAGAGGGTGTGGGTTACACCAATGCTTCCAACAATTTGTTTG CTTGCCTCCCATGTACAGCTTGTAAATCAGATGAAGA | 1226 |
| DR5 | NM_00 3842 | CTCTGAGACAGTGCTTCGATGACTTTGCAGACTTGGTGCCCTTTGA CTCCTGGGAGCCGCTCATGAGGAAGTTGGGCCTCATGG | 1227 |
| DUSP1 | NM_00 4417 | AGACATCAGCTCCTGGTTCAACGAGGCCATTGACTTCATAGACTCC ATCAAGAATGCTGGAGGAAGGGTGTTTGTC | 1228 |
| EEF1D | NM_00 1960 | CAGAGGATGACGAGGATGATGACATTGACCTGTTTGGCAGTGACA ATGAGGAGGAGGACAAGGAGGCGGCACAG | 1229 |
| EGFR | NM_00 5228 | TGTCGATGGACTTCCAGAACCACCTGGGCAGCTGCCAAAAGTGTG ATCCAAGCTGTCCCAAT | 1230 |
| EIF4E | NM_00 1968 | GATCTAAGATGGCGACTGTCGAACCGGAAACCACCCCTACTCCTAA TCCCCCGACTACAGAAGAGGAGAAAACGGAATCTAA | 1231 |
| EIF4EL3 | NM_00 4846 | AAGCCGCGGTTGAATGTGCCATGACCCTCTCCCTCTCTGGATGGC ACCATCATTGAAGCTGGCGTCA | 1232 |
| ELP3 | NM_01 8091 | CTCGGATCCTAGCCCTCGTGCCTCCATGGACTCGAGTGTACCGAG TACAGAGGGATATTCCAATGCC | 1233 |
| ER2 | NM_00 1437 | TGGTCCATCGCCAGTTATCACATCTGTATGCGGAACCTCAAAAGAG TCCCTGGTGTGAAGCAAGATCGCTAGAACA | 1234 |
| ErbB3 | NM_00 1982 | CGGTTATGTCATGCCAGATACACACCTCAAAGGTACTCCCTCCTCC CGGGAAGGCACCCTTTCTTCAGTGGGTCTCAGTTC | 1235 |
| ERBB4 | NM_00 5235 | TGGCTCTTAATCAGTTTCGTTACCTGCCTCTGGAGAATTTACGCATT ATTCGTGGGACAAAACTTTATGAGGATCGATATGCCTTG | 1236 |
| ERCC1 | NM_00 1983 | GTCCAGGTGGATGTGAAAGATCCCCAGCAGGCCCTCAAGGAGCTG GCTAAGATGTGTATCCTGGCCG | 1237 |
| ERK1 | NM_00 2746 | ACGGATCACAGTGGAGGAAGCGCTGGCTCACCCCTACCTGGAGCA GTACTATGACCCGACGGATGAG | 1238 |
| ESPL1 | NM_01 2291 | ACCCCCAGACCGGATCAGGCAAGCTGGCCCTCATGTCCCCTTCAC GGTGTTTGAGGAAGTCTGCCCTACA | 1239 |
| EstR1 | NM_00 0125 | CGTGGTGCCCCTCTATGACCTGCTGCTGGAGATGCTGGACGCCCA CCGCCTACATGCGCCCACTAGCC | 1240 |
| fas 0043 | NM_00 | GGATTGCTCAACAACCATGCTGGGCATCTGGACCCTCCTACCTCTG GTTCTTACGTCTGTTGCTAGATTATCGTCCAAAAGTGTTAATGCC | 1241 |
| fasl | NM_00 0639 | GCACTTTGGGATTCTTTCCATTATGATTCTTGTTACAGGCACCGAG AATGTTGTATTCAGTGAGGGTCTTCTTACATGC | 1242 |
| FASN | NM_00 4104 | GCCTCTTCCTGTTCGACGGCTCGCCCACCTACGTACTGGCCTACA CCCAGAGCTACCGGGCAAAGC | 1243 |
| FBXO5 | NM_01 2177 | GGCTATTCCTCATTTTCTCTACAAAGTGGCCTCAGTGAACATGAAG AAGGTAGCCTCCTGGAGGAGAATTTCGGTGACAGTCTACAATCC | 1244 |
| FDFT1 | NM_00 4462 | AAGGAAAGGGTGCCTCATCCCAGCAACCTGTCCTTGTGGGTGATG ATCACTGTGCTGCTTGTGGCTC | 1245 |
| FGFR1 | NM_02 3109 | CACGGGACATTCACCACATCGACTACTATAAAAAGACAACCAACGG CCGACTGCCTGTGAAGTGGATGGCACCC | 1246 |
| FHIT | NM_00 2012 | CCAGTGGAGCGCTTCCATGACCTGCGTCCTGATGAAGTGGCCGAT TTGTTTCAGACGACCCAGAGAG | 1247 |
| FIGF | NM_00 4469 | GGTTCCAGCTTTCTGTAGCTGTAAGCATTGGTGGCCACACCACCTC CTTACAAAGCAACTAGAACCTGCGGC | 1248 |
| FLJ20354 (DEPDC1 official) | NM_01 7779 | GCGTATGATTTCCCGAATGAGTCAAAATGTTGATATGCCCAAACTTC ATGATGCAATGGGTACGAGGTCACTG | 1249 |
| FOS | NM_00 5252 | CGAGCCCTTTGATGACTTCCTGTTCCCAGCATCATCCAGGCCCAGT GGCTCTGAGACAGCCCGCTCC | 1250 |
| FOXM1 | NM_02 1953 | CCACCCCGAGCAAATCTGTCCTCCCCAGAACCCCTGAATCCTGGA GGCTCACGCCCCCAGCCAAAGTAGGGGGACTGGATTT | 1251 |
| FUS | NM_00 4960 | GGATAATTCAGACAACAACACCATCTTTGTGCAAGGCCTGGGTGAG AATGTTACAATTGAGTCTGTGGCTGATTACTTCA | 1252 |
| FYN | N M_00 2037 | GAAGCGCAGATCATGAAGAAGCTGAAGCACGACAAGCTGGTCCAG CTCTATGCAGTGGTGTCTGAGGAG | 1253 |
| G 1 P3 | NM_00 2038 | CCTCCAACTCCTAGCCTCAAGTGATCCTCCTGTCTCAACCTCCCAA GTAGGATTACAAGCATGCGCC | 1254 |
| GADD45 | NM_00 1924 | GTGCTGGTGACGAATCCACATTCATCTCAATGGAAGGATCCTGCCT TAAGTCAACTTATTTGTTTTTGCCGGG | 1255 |
| GADD45B | NM_01 5675 | ACCCTCGACAAGACCACACTTTGGGACTTGGGAGCTGGGGCTGAA GTTGCTCTGTACCCATGAACTCCCA | 1256 |
| GAGE1 | NM_00 1468 | AAGGGCAATCACAGTGTTAAAAGAAGACATGCTGAAATGTTGCAGG CTGCTCCTATGTTGGAAAATTCTTCATTGAAGTTCTCC | 1257 |
| GAPDH | NM_00 2046 | ATTCCACCCATGGCAAATTCCATGGCACCGTCAAGGCTGAGAACG GGAAGCTTGTCATCAATGGAAATCCCATC | 1258 |
| GATA3 | NM_00 2051 | CAAAGGAGCTCACTGTGGTGTCTGTGTTCCAACCACTGAATCTGGA CCCCATCTGTGAATAAGCCATTCTGACTC | 1259 |
| GBP1 | NM_00 2053 | TTGGGAAATATTTGGGCATTGGTCTGGCCAAGTCTACAATGTCCCA ATATCAAGGACAACCACCCTAGCTTCT | 1260 |
| GBP2 | NM_00 4120 | GCATGGGAACCATCAACCAGCAGGCCATGGACCAACTTCACTATGT GACAGAGCTGACAGATCGAATCAAGGCAAACTCCTCA | 1261 |
| GCLC | NM_00 1498 | CTGTTGCAGGAAGGCATTGATCATCTCCTGGCCCAGCATGTTGCTC ATCTCTTTATTAGAGACCCACTGAC | 1262 |
| GDF15 | NM_00 4864 | CGCTCCAGACCTATGATGACTTGTTAGCCAAAGACTGCCACTGCAT ATGAGCAGTCCTGGTCCTTCCACTGT | 1263 |
| GGPS1 | NM_00 4837 | CTCCGACGTGGCTTTCCAGTGGCCCACAGCATCTATGGAATCCCAT CTGTCATCAATTCTGCCAATTACG | 1264 |
| GLRX | NM_00 2064 | GGAGCTCTGCAGTAACCACAGAACAGGCCCCATGCTGACGTCCCT CCTCAAGAGCTGGATGGCATTG | 1265 |
| GNS | NM_00 2076 | GGTGAAGGTTGTCTCTTCCGAGGGCCTTCTGAAGACAGGGCTCTT GAACAGACAAGTGGAAGGGCTG | 1266 |
| GPR56 | NM_00 5682 | TACCCTTCCATGTGCTGGATCCGGGACTCCCTGGTCAGCTACATCA CCAACCTGGGCCTCTTCAGC | 1267 |
| GPX1 | NM_00 0581 | GCTTATGACCGACCCCAAGCTCATCACCTGGTCTCCGGTGTGTCG CAACGATGTTGCCTGGAACTTT | 1268 |
| GRB7 | NM_00 5310 | CCATCTGCATCCATCTTGTTTGGGCTCCCCACCCTTGAGAAGTGCC TCAGATAATACCCTGGTGGCC | 1269 |
| GSK3B | NM_00 2093 | GACAAGGACGGCAGCAAGGTGACAACAGTGGTGGCAACTCCTGG GCAGGGTCCAGACAGGCCACAA | 1270 |
| GSR | NM_00 0637 | GTGATCCCAAGCCCACAATAGAGGTCAGTGGGAAAAAGTACACCG CCCCACACATCCTGATCGCCACA | 1271 |
| GSTM1 | NM_00 0561 | AAGCTATGAGGAAAAGAAGTACACGATGGGGGACGCTCCTGATTAT GACAGAAGCCAGTGGCTGAATGAAAAATTCAAGCTGGGCC | 1272 |
| GSTp | NM_00 0852 | GAGACCCTGCTGTCCCAGAACCAGGGAGGCAAGACCTTCATTGTG GGAGACCAGATCTCCTTCGCTGACTACAACC | 1273 |
| GUS | NM_00 0181 | CCCACTCAGTAGCCAAGTCACAATGTTTGGAAAACAGCCCGTTTAC TTGAGCAAGACTGATACCACCTGCGTG | 1274 |
| HDAC6 | NM_00 6044 | TCCTGTGCTCTGGAAGCCCTTGAGCCCTTCTGGGAGGTTCTTGTGA GATCAACTGAGACCGTGGAG | 1275 |
| HER2 | NM_00 4448 | CGGTGTGAGAAGTGCAGCAAGCCCTGTGCCCGAGTGTGCTATGGT CTGGGCATGGAGCACTTGCGAGAGG | 1276 |
| HIF1A | NM_00 1530 | TGAACATAAAGTCTGCAACATGGAAGGTATTGCACTGCACAGGCCA CATTCACGTATATGATACCAACAGTAACCAACCTCA | 1277 |
| HNF3A | NM_00 4496 | TCCAGGATGTTAGGAACTGTGAAGATGGAAGGGCATGAAACCAGC GACTGGAACAGCTACTACGCAGACACGC | 1278 |
| HRAS | NM_00 5343 | GGACGAATACGACCCCACTATAGAGGATTCCTACCGGAAGCAGGT GGTCATTGATGGGGAGACGTGC | 1279 |
| HSPA1A | NM_00 5345 | CTGCTGCGACAGTCCACTACCTTTTTCGAGAGTGACTCCCGTTGTC CCAAGGCTTCCCAGAGCGAACCTG | 1280 |
| HSPA1B | NM_00 5346 | GGTCCGCTTCGTCTTTCGAGAGTGACTCCCGCGGTCCCAAGGCTT TCCAGAGCGAACCTGTGC | 1281 |
| HSPA1L | NM_00 5527 | GCAGGTGTGATTGCTGGACTTAATGTGCTAAGAATCATCAATGAGC CCACGGCTGCTGCCATTGCCTATGGT | 1282 |
| HSPA5 | NM_00 5347 | GGCTAGTAGAACTGGATCCCAACACCAAACTCTTAATTAGACCTAG GCCTCAGCTGCACTGCCCGAAAAGCATTTGGGCAGACC | 1283 |
| HSPA9B | NM_00 4134 | GGCCACTAAAGATGCTGGCCAGATATCTGGACTGAATGTGCTTCG GGTGATTAATGAGCCCACAGCTGCT | 1284 |
| HSPB1 | NM_ 00 1540 | CCGACTGGAGGAGCATAAAAGCGCAGCCGAGCCCAGCGCCCCGC ACTTTTCTGAGCAGACGTCCAGAGCAGAGTCAGCCAGCAT | 1285 |
| HSPCA | NM_00 5348 | [?] CAAAAGGCAGAGGCTGATAAGAACGACAAGTCTGTGAAGGATCTG GTCATCTTGCTTTATGAAACTGCGCT | 1286 |
| ID1 | NM_00 2165 | AGAACCGCAAGGTGAGCAAGGTGGAGATTCTCCAGCACGTCATCG ACTACATCAGGGACCTTCAGTTGGA | 1287 |
| IFITM1 | NM_00 3641 | CACGCAGAAAACCACACTTCTCAAACCTTCACTCAACACTTCCTTCC CCAAAGCCAGAAGATGCACAAGGAGGAACATG | 1288 |
| IGF1R | NM_00 0875 | GCATGGTAGCCGAAGATTTCACAGTCAAAATCGGAGATTTTGGTAT GACGCGAGATATCTATGAGACAGACTATTACCGGAAA | 1289 |
| IGFBP2 | NM_00 0597 | GTGGACAGCACCATGAACATGTTGGGCGGGGGAGGCAGTGCTGG CCGGAAGCCCCTCAAGTCGGGTATGAAGG | 1290 |
| IGFBP3 | NM_00 0598 | ACGCACCGGGTGTCTGATCCCAAGTTCCACCCCCTCCATTCAAAGA TAATCATCATCAAGAAAGGGCA | 1291 |
| IGFBP5 | NM_00 0599 | TGGACAAGTACGGGATGAAGCTGCCAGGCATGGAGTACGTTGACG GGGACTTTCAGTGCCACACCTTCG | 1292 |
| IL2RA | NM_00 0417 | TCTGCGTGGTTCCTTTCTCAGCCGCTTCTGACTGCTGATTCTCCCG TTCACGTTGCCTAATAAACATCCTTCAA | 1293 |
| IL6 | NM_00 0600 | CCTGAACCTTCCAAAGATGGCTGAAAAGATGGATGCTTCCAATCT GGATTCAATGAGGAGACTTGCCTGGT | 1294 |
| IL-7 | NM_00 0880 | GCGGTGATTCGGAAATTCGCGAATTCCTCTGGTCCTCATCCAGGTG CGCGGGAAGCAGGTGCCCAGGAGAG | 1295 |
| IL-8 | NM_00 0584 | AAGGAACCATCTCACTGTGTGTAAACATGACTTCCAAGCTGGCCGT GGCTCTCTTGGCAGCCTTCCTGAT | 1296 |
| IL8RB | NM_00 1557 | CCGCTCCGTCACTGATGTCTACCTGCTGAACCTAGCCTTGGCCGA CCTACTCTTTGCCCTGACCTTGC | 1297 |
| ILK | NM_00 101479 4 | CTCAGGATTTTCTCGCATCCAAATGTGCTCCCAGTGCTAGGTGCCT GCCAGTCTCCACCTGCTCCT | 1298 |
| ILT-2 | NM_00 6669 | AGCCATCACTCTCAGTGCAGCCAGGTCCTATCGTGGCCCCTGAGG AGACCCTGACTCTGCAGT | 1299 |
| INCENP | NM_02 0238 | GCCAGGATACTGGAGTCCATCACAGTGAGCTCCCTGATGGCTACA CCCCAGGACCCCAAGGGTCAAG | 1300 |
| IRAK2 | NM_00 1570 | GGATGGAGTTCGCCTCCTACGTGATCACAGACCTGACCCAGCTGC GGAAGATCAAGTCCATGGAGCG | 1301 |
| IRS1 | NM_00 5544 | CCACAGCTCACCTTCTGTCAGGTGTCCATCCCAGCTCCAGCCAGCT CCCAGAGAGGAAGAGACTGGCACTGAGG | 1302 |
| ITGB1 | NM_00 2211 | TCAGAATTGGATTTGGCTCATTTGTGGAAAAGACTGTGATGCCTTA CATTAGCACAACACCAGCTAAGCTCAGG | 1303 |
| K-Alpha-1 | NM_00 6082 | TGAGGAAGAAGGAGAGGAATACTAATTATCCATTCCTTTTGGCCCT GCAGCATGTCATGCTCCCAGAATTTCAG | 1304 |
| KDR | NM_00 2253 | GAGGACGAAGGCCTCTACACCTGCCAGGCATGCAGTGTTCTTGGC TGTGCAAAAGTGGAGGCATTTTT | 1305 |
| Ki-67 | NM_00 2417 | CGGACTTTGGGTGCGACTTGACGAGCGGTGGTTCGACAAGTGGCC TTGCGGGCCGGATCGTCCCAGTGGAAGAGTTGTAA | 1306 |
| KIF11 | NM_00 4523 | TGGAGGTTGTAAGCCAATGTTGTGAGGCTTCAAGTTCAGACATCAC TGAGAAATCAGATGGACGTAAGGCA | 1307 |
| KIF22 | NM_00 7317 | CTAAGGCACTTGCTGGAAGGGCAGAATGCCAGTGTGCTTGCCTAT GGACCCACAGGAGCTGGGAAGA | 1308 |
| KIF2C | NM_00 6845 | AATTCCTGCTCCAAAAGAAAGTCTTCGAAGCCGCTCCACTCGCATG TCCACTGTCTCAGAGCTTCGCATCACG | 1309 |
| KIFC1 | NM_00 2263 | CCACAGGGTTGAAGAACCAGAAGCCAGTTCCTGCTGTTCCTGTCCA GAAGTCTGGCACATCAGGTG | 1310 |
| KLK10 | NM_00 2776 | GCCCAGAGGCTCCATCGTCCATCCTCTTCCTCCCCAGTCGGCTGA ACTCTCCCCTTGTCTGCACTGTTCAAACCTCTG | 1311 |
| KNS2 | NM_00 5552 | CAAACAGAGGGTGGCAGAAGTGCTCAATGACCCTGAGAACATGGA GAAGCGCAGGAGCCGTGAGAGCCTC | 1312 |
| KNTC1 | NM_01 4708 | AGCCGAGGCTTTGTTGAAGAAGCTTCATATCCAGTACCGGCGATCG GGCACAGAAGCTGTGCTCATAGCCCA | 1313 |
| KNTC2 | NM_00 6101 | ATGTGCCAGTGAGCTTGAGTCCTTGGAGAAACACAAGCACCTGCTA GAAAGTACTGTTAACCAGGGGCTCA | 1314 |
| KRT14 | NM_00 0526 | GGCCTGCTGAGATCAAAGACTACAGTCCCTACTTCAAGACCATTGA GGACCTGAGGAACAAGATTCTCACAGCCACAGTGGAC | 1315 |
| KRT17 | NM_00 0422 | CGAGGATTGGTTCTTCAGCAAGACAGAGGAACTGAACCGCGAGGT GGCCACCAACAGTGAGCTGGTGCAGAGT | 1316 |
| KRT19 | NM_00 2276 | TGAGCGGCAGAATCAGGAGTACCAGCGGCTCATGGACATCAAGTC GCGGCTGGAGCAGGAGATTGCCACCTACCGCA | 1317 |
| KRT5 | NM_00 0424 | TCAGTGGAGAAGGAGTTGGACCAGTCAACATCTCTGTTGTCACAAG CAGTGTTTCCTCTGGATATGGCA | 1318 |
| L1CAM | NM_00 0425 | CTTGCTGGCCAATGCCTACATCTACGTTGTCCAGCTGCCAGCCAAG ATCCTGACTGCGGACAATCA | 1319 |
| LAMC2 | NM_00 5562 | ACTCAAGCGGAAATTGAAGCAGATAGGTCTTATCAGCACAGTCTCC GCCTCCTGGATTCAGTGTCTCGGCTTCAGGGAGT | 1320 |
| LAPTM4B | NM_01 8407 | AGCGATGAAGATGGTCGCGCCCTGGACGCGGTTCTACTCCAACAG CTGCTGCTTGTGCTGCCATGTC | 1321 |
| LIMK1 | NM_01 6735 | GCTTCAGGTGTTGTGACTGCAGTGCCTCCCTGTCGCACCAGTACTA TGAGAAGGATGGGCAGCTCTT | 1322 |
| LIMK2 | NM_00 5569 | CTTTGGGCCAGGAGGAATCTGTTACTCGAATCCACCCAGGAACTCC CTGGCAGTGGATTGTGGGAG | 1323 |
| MAD1L1 | NM_00 3550 | AGAAGCTGTCCCTGCAAGAGCAGGATGCAGCGATTGTGAAGAACA TGAAGTCTGAGCTGGTACGGCT | 1324 |
| MAD2L1 | NM_00 2358 | CCGGGAGCAGGGAATCACCCTGCGCGGGAGCGCCGAAATCGTGG CCGAGTTCTTCTCATTCGGCATCAACAGCAT | 1325 |
| MAD2L1BP | NM_01 4628 | CTGTCATGTGGCAGACCTTCCATCCGAACCACGGCTTGGGAAGAC TACATTTGGTTCCAGGCACCAGTGACATTTA | 1326 |
| MAD2L2 | NM_00 6341 | GCCCAGTGGAGAAATTCGTCTTTGAGATCACCCAGCCTCCACTGCT GTCCATCAGCTCAGACTCGC | 1327 |
| MAGE2 | NM_00 5361 | CCTCAGAAATTGCCAGGACTTCTTTCCCGTGATCTTCAGCAAAGCC TCCGAGTACTTGCAGCTGGTCTTTGG | 1328 |
| MAGE6 | NM_00 5363 | AGGACTCCAGCAACCAAGAAGAGGAGGGGCCAAGCACCTTCCCTG ACCTGGAGTCTGAGTTCCAAGCAGCACTC | 1329 |
| MAP2 | NM_00 2374 | CGGACCACCAGGTCAGAGCCAATTCGCAGAGCAGGGAAGAGTGGT ACCTCAACACCCACTACCCCTG | 1330 |
| MAP2K3 | NM_00 2756 | GCCCTCCAATGTCCTTATCAACAAGGAGGGCCATGTGAAGATGTGT GACTTTGGCATCAGTGGCTAC | 1331 |
| MAP4 | NM_00 2375 | GCCGGTCAGGCACACAAGGGGCCCTTGGAGCGTGGACTGGTTGG TTTTGCCATTTTGTTGTGTGTATGCTGC | 1332 |
| MAP6 | NM_03 3063 | CCCTCAACCGGCAAATCCGCGAGGAGGTGGCGAGTGCAGTGAGC AGCTCCTACAGGAATGAATTCAGGGCATGGACG | 1333 |
| MAPK14 | NM_13 9012 | TGAGTGGAAAAGCCTGACCTATGATGAAGTCATCAGCTTTGTGCCA CCACCCCTTGACCAAGAAGAGATGGAGTCC | 1334 |
| MAPK8 | NM_00 2750 | CAACACCCGTACATCAATGTCTGGTATGATCCTTCTGAAGCAGAAG CTCCACCACCAAAGATCCCTGACAAGCAGTTAGATGA | 1335 |
| MAPRE1 | NM_01 2325 | GACCTTGGAACCTTTGGAACCTGCTGTCAACAGGTCTTACAGGGCT GCTTGAACCCTCATAGGCCTAGG | 1336 |
| MAPT | NM_01 6835 | CACAAGCTGACCTTCCGCGAGAACGCCAAAGCCAAGACAGACCAC GGGGCGGAGATCGTGTACAAGT | 1337 |
| Maspin | NM_00 2639 | CAGATGGCCACTTTGAGAACATTTTAGCTGACAACAGTGTGAACGA CCAGACCAAAATCCTTGTGGTTAATGCTGCC | 1338 |
| MCL1 | NM_02 1960 | CTTCGGAAACTGGACATCAAAAACGAAGACGATGTGAAATCGTTGT CTCGAGTGATGATCCATGTTTTCAGCGAC | 1339 |
| MCM2 | NM_00 4526 | GACTTTTGCCCGCTACCTTTCATTCCGGCGTGACAACAATGAGCTG TTGCTCTTCATACTGAAGCAGTTAGTGGC | 1340 |
| MCM6 | NM_00 5915 | TGATGGTCCTATGTGTCACATTCATCACAGGTTTCATACCAACACAG GCTTCAGCACTTCCTTTGGTGTGTTTCCTGTCCCA | 1341 |
| MCP1 | NM_00 2982 | CGCTCAGCCAGATGCAATCAATGCCCCAGTCACCTGCTGTTATAAC TTCACCAATAGGAAGATCTCAGTGC | 1342 |
| MGMT | NM_00 2412 | GTGAAATGAAACGCACCACACTGGACAGCCCTTTGGGGAAGCTGG AGCTGTCTGGTTGTGAGCAGGGTC | 1343 |
| MMP12 | NM_00 2426 | CCAACGCTTGCCAAATCCTGACAATTCAGAACCAGCTCTCTGTGAC CCCAATTTGAGTTTTGATGCTGTCACTACCGT | 1344 |
| MMP2 | NM_00 4530 | CCATGATGGAGAGGCAGACATCATGATCAACTTTGGCCGCTGGGA GCATGGCGATGGATACCCCTTTGACGGTAAGGACGGACTCC | 1345 |
| MMP9 | NM_00 4994 | GAGAACCAATCTCACCGACAGGCAGCTGGCAGAGGAATACCTGTA CCGCTATGGTTACACTCGGGTG | 1346 |
| MRE11A | NM_00 5590 | GCCATGCTGGCTCAGTCTGAGCTGTGGGCCACATCAGCTAGTGGC TCTTCTCATGCATCAGTTAGGTGGGTCTGGGTG | 1347 |
| MRP1 | N M_00 4996 | TCATGGTGCCCGTCAATGCTGTGATGGCGATGAAGACCAAGACGT ATCAGGTGGCCCACATGAAGAGCAAAGACAATCG | 1348 |
| MRP2 | NM_00 0392 | AGGGGATGACTTGGACACATCTGCCATTCGACATGACTGCAATTTT GACAAAGCCATGCAGTTTT | 1349 |
| MRP3 | NM_00 3786 | TCATCCTGGCGATCTACTTCCTCTGGCAGAACCTAGGTCCCTCTGT CCTGGCTGGAGTCGCTTTCATGGTCTTGCTGATTCCACTCAACGG | 1350 |
| MSH3 | NM_00 2439 | TGATTACCATCATGGCTCAGATTGGCTCCTATGTTCCTGCAGAAGA AGCGACAATTGGGATTGTGGATGGCATTTTCACAAG | 1351 |
| MUC1 | NM_00 2456 | GGCCAGGATCTGTGGTGGTACAATTGACTCTGGCCTTCCGAGAAG GTACCATCAATGTCCACGACGTGGAG | 1352 |
| MX1 | NM_00 2462 | GAAGGAATGGGAATCAGTCATGAGCTAATCACCCTGGAGATCAGCT CCCGAGATGTCCCGGATCTGACTCTAATAGAC | 1353 |
| MYBL2 | NM_00 2466 | GCCGAGATCGCCAAGATGTTGCCAGGGAGGACAGACAATGCTGTG AAGAATCACTGGAACTCTACCATCAAAAG | 1354 |
| MYH11 | N M_00 2474 | CGGTACTTCTCAGGGCTAATATATACGTACTCTGGCCTCTTCTGCG TGGTGGTCAACCCCTATAAACACCTGCCCATCTACTCGG | 1355 |
| NEK2 | NM_00 2497 | GTGAGGCAGCGCGACTCTGGCGACTGGCCGGCCATGCCTTCCCG GGCTGAGGACTATGAAGTGTTGTACACCATTGGCA | 1356 |
| NFKBp50 | NM_00 3998 | CAGACCAAGGAGATGGACCTCAGCGTGGTGCGGCTCATGTTTACA GCTTTTCTTCCGGATAGCACTGGCAGCT | 1357 |
| NFKBp65 | NM_02 1975 | CTGCCGGGATGGCTTCTATGAGGCTGAGCTCTGCCCGGACCGCTG CATCCACAGTTTCCAGAACCTGG | 1358 |
| NME6 | NM_00 5793 | CACTGACACCCGCAACACCACCCATGGTTCGGACTCTGTGGTTTCA GCCAGCAGAGAGATTGCAGCC | 1359 |
| NPC2 | NM_00 6432 | CTGCTTCTTTCCCGAGCTTGGAACTTCGTTATCCGCGATGCGTTTC CTGGCAGCTACATTCCTGCT | 1360 |
| NPD009 (ABAT official) | NM_02 0686 | GGCTGTGGCTGAGGCTGTAGCATCTCTGCTGGAGGTGAGACACTC TGGGAACTGATTTGACCTCGAATGCTCC | 1361 |
| NTSR2 | NM_01 2344 | CGGACCTGAATGTAATGCAAGAATGAACAGAACAAGCAAAATGACC AGCTGCTTAGTCACCTGGCAAAG | 1362 |
| NUSAP1 | NM_01 6359 | CAAAGGAAGAGCAACGGAAGAAACGCGAGCAAGAACGAAAGGAGA AGAAAGCAAAGGTTTTGGGAAT | 1363 |
| p21 | NM_00 0389 | TGGAGACTCTCAGGGTCGAAAACGGCGGCAGACCAGCATGACAGA TTTCTACCACTCCAAACGCC | 1364 |
| p27 | NM_00 4064 | CGGTGGACCACGAAGAGTTAACCCGGGACTTGGAGAAGCACTGCA GAGACATGGAAGAGGCGAGCC | 1365 |
| PCTK1 | NM_00 6201 | TCACTACCAGCTGACATCCGGCTGCCTGAGGGCTACCTGGAGAAG CTGACCCTCAATAGCCCCATCT | 1366 |
| PDGFRb | N M_00 2609 | CCAGCTCTCCTTCCAGCTACAGATCAATGTCCCTGTCCGAGTGCTG GAGCTAAGTGAGAGCCACCC | 1367 |
| PFDN5 | NM_14 5897 | GAGAAGCACGCCATGAAACAGGCCGTCATGGAAATGATGAGTCAG AAGATTCAGCAGCTCACAGCC | 1368 |
| PGK1 | NM_00 0291 | AGAGCCAGTTGCTGTAGAACTCAAATCTCTGCTGGGCAAGGATGTT CTGTTCTTGAAGGACTGTGTAGGCCCAG | 1369 |
| PHB | NM_00 2634 | GACATTGTGGTAGGGGAAGGGACTCATTTTCTCATCCCGTGGGTAC AGAAACCAATTATCTTTGACTGCCG | 1370 |
| PI3KC2A | NM_00 2645 | ATACCAATCACCGCACAAACCCAGGCTATTTGTTAAGTCCAGTCAC AGCGCAAAGAAACATATGCGGAGAAAATGCTAGTGTG | 1371 |
| PIM1 | NM_00 2648 | CTGCTCAAGGACACCGTCTACACGGACTTCGATGGGACCCGAGTG TATAGCCCTCCAGAGTGGATCC | 1372 |
| PIM2 | NM_00 6875 | TGGGGACATTCCCTTTGAGAGGGACCAGGAGATTCTGGAAGCTGA GCTCCACTTCCCAGCCCATGTC | 1373 |
| PLAUR | NM_00 2659 | CCCATGGATGCTCCTCTGAAGAGACTTTCCTCATTGACTGCCGAGG CCCCATGAATCAATGTCTGGTAGCCACCGG | 1374 |
| PLD3 | NM_01 2268 | CCAAGTTCTGGGTGGTGGACCAGACCCACTTCTACCTGGGCAGTG CCAACATGGACTGGCGTTCAC | 1375 |
| PLK | NM_00 5030 | AATGAATACAGTATTCCCAAGCACATCAACCCCGTGGCCGCCTCCC TCATCCAGAAGATGCTTCAGACA | 1376 |
| PMS1 | NM_00 0534 | CTTACGGTTTTCGTGGAGAAGCCTTGGGGTCAATTTGTTGTATAGC TGAGGTTTTAATTACAACAAGAACGGCTGCT | 1377 |
| PMS2 | NM_00 0535 | GATGTGGACTGCCATTCAAACCAGGAAGATACCGGATGTAAATTTC GAGTTTTGCCTCAGCCAACTAATCTCGCA | 1378 |
| PP591 | NM_02 5207 | CCACATACCGTCCAGCCTATCTACTGGAGAACGAAGAAGAGGAGC GGAACTCCCGCACATGACCTC | 1379 |
| PPP2CA | NM_00 2715 | GCAATCATGGAACTTGACGATACTCTAAAATACTCTTTCTTGCAGTT TGACCCAGCACCTCGTAGAGGCGAGCCACAT | 1380 |
| PR | NM_00 0926 | GCATCAGGCTGTCATTATGGTGTCCTTACCTGTGGAGCTGTAAGG TCTTCTTTAAGAGGGCAATGGAAGGGCAGCACAACTACT | 1381 |
| PRDX1 | NM_00 2574 | AGGACTGGGACCCATGAACATTCCTTTGGTATCAGACCCGAAGCG CACCATTGCTCAGGATTATGGG | 1382 |
| PRDX2 | NM_00 5809 | GGTGTCCTTCGCCAGATCACTGTTAATGATTTGCCTGTGGGACGCT CCGTGGATGAGGCTCTGCGGCTG | 1383 |
| PRKCA | NM_00 2737 | CAAGCAATGCGTCATCAATGTCCCCAGCCTCTGCGGAATGGATCAC ACTGAGAAGAGGGGGCGGATTTAC | 1384 |
| PRKCD | NM_00 6254 | CTGACACTTGCCGCAGAGAATCCCTTTCTCACCCACCTCATCTGCA CCTTCCAGACCAAGGACCACCT | 1385 |
| PRKCG | NM_00 2739 | GGGTTCTAGACGCCCCTCCCAAGCGTTCCTGGCCTTCTGAACTCC ATACAGCCTCTACAGCCGTCC | 1386 |
| PRKCH | NM_00 6255 | CTCCACCTATGAGCGTCTGTCTCTGTGGGCTTGGGATGTTAACAG[?] AGCCAAAAGGAGGGAAAGTGTG | 1387 |
| pS2 | NM_00 3225 | GCCCTCCCAGTGTGCAAATAAGGGCTGCTGTTTCGACGACACCGT TCGTGGGGTCCCCTGGTGCTTCTATCCTAATACCATCGACG | 1388 |
| PTEN | NM_00 0314 | TGGCTAAGTGAAGATGACAATCATGTTGCAGCAATTCACTGTAAAG CTGGAAAGGGACGAACTGGTGTAATGATATGTGCA | 1389 |
| PTPD1 | NM_00 7039 | CGCTTGCCTAACTCATACTTTCCCGTTGACACTTGATCCACGCAGC GTGGCACTGGGACGTAAGTGGCGCAGTCTGAATGG | 1390 |
| PTTG1 | NM_00 4219 | GGCTACTCTGATCTATGTTGATAAGGAAAATGGAGAACCAGGCACC CGTGTGGTTGCTAAGGATGGGCTGAAGC | 1391 |
| RAB27B | NM_00 4163 | GGGACACTGCGGGACAAGAGCGGTTCCGGAGTCTCACCACTGCAT TTTTCAGAGACGCCATGGGC | 1392 |
| RAB31 | NM_00 6868 | CTGAAGGACCCTACGCTCGGTGGCCTGGCACCTCACTTTGAGAAG AGTGAGCACACTGGCTTTGCAT | 1393 |
| RAB6C | NM_03 2144 | GCGACAGCTCCTCTAGTTCCACCATGTCCGCGGCGGAGACTTCG GGAATCCGCTGAGGAAATTCAAGCTGGTGTTCC | 1394 |
| RAD1 | NM_00 2853 | GAGGAGTGGTGACAGTCTGCAAAATCAATACACAGGAACCTGAGG AGACCCTGGACTTTGATTTCTGCAGC | 1395 |
| RAD54L | NM_00 3579 | AGCTAGCCTCAGTGACACACATGACAGGTTGCACTGCCGACGTTG TGTCAACAGCCGTCAGATCCGG | 1396 |
| RAF1 | NM_00 2880 | CGTCGTATGCGAGAGTCTGTTTCCAGGATGCCTGTTAGTTCTCAGC ACAGATATTCTACACCTCACGCCTTCA | 1397 |
| RALBP1 | NM 00 6788 | GGTGTCAGATATAAATGTGCAAATGCCTTCTTGCTGTCCTGTCGGT CTCAGTACGTTCACTTTATAGCTGCTGGCAATATCGAA | 1398 |
| RAP1GDS1 | NM_02 1159 | TGTGGATGCTGGATTGATTTCACCACTGGTGCAGCTGCTAAATAGC AAAGACCAGGAAGTGCTGCTT | 1399 |
| RASSF1 | NM_00 7182 | AGTGGGAGACACCTGACCTTTCTCAAGCTGAGATTGAGCAGAAGAT CAAGGAGTACAATGCCCAGATCA | 1400 |
| RB1 | NM_00 0321 | CGAAGCCCTTACAAGTTTCCTAGTTCACCCTTACGGATTCCTGGAG GGAACATCTATATTTCACCCCTGAAGAGTCC | 1401 |
| RBM17 | NM_03 2905 | CCCAGTGTACGAGGAACAAGACAGACCGAGATCTCCAACCGGACC TAGCAACTCCTTCCTCGCTAA | 1402 |
| RCC1 | NM_00 1269 | GGGCTGGGTGAGAATGTGATGGAGAGGAAGAAGCCGGCCCTGGT ATCCATTCCGGAGGATGTTGTG | 1403 |
| REG1A | NM_00 2909 | CCTACAAGTCCTGGGGCATTGGAGCCCCAAGCAGTGTTAATCCTG GCTACTGTGTGAGCCTGACCTCA | 1404 |
| RELB | NM_00 6509 | GCGAGGAGCTCTACTTGCTCTGCGACAAGGTGCAGAAAGAGGACA TATCAGTGGTGTTCAGCAGGGC | 1405 |
| RhoB | NM_00 4040 | AAGCATGAACAGGACTTGACCATCTTTCCAACCCCTGGGGAAGACA TTTGCAACTGACTTGGGGAGG | 1406 |
| rhoC | NM_17 5744 | CCCGTTCGGTCTGAGGAAGGCCGGGACATGGCGAACCGGATCAG TGCCTTTGGCTACCTTGAGTGCTC | 1407 |
| RIZ1 | NM_01 2231 | CCAGACGAGCGATTAGAAGCGGCAGCTTGTGAGGTGAATGATTTG GGGGAAGAGGAGGAGGAGGAAGAGGAGGA | 1408 |
| ROCK1 | NM_00 5406 | TGTGCACATAGGAATGAGCTTCAGATGCAGTTGGCCAGCAAAGAG AGTGATATTGAGCAATTGCGTGCTAAAC | 1409 |
| RPL37A | NM_00 0998 | GATCTGGCACTGTGGTTCCTGCATGAAGACAGTGGCTGGCGGTGC CTGGACGTACAATACCACTTCCGCTGTCA | 1410 |
| RPLPO | NM_00 1002 | CCATTCTATCATCAACGGGTACAAACGAGTCCTGGCCTTGTCTGTG GAGACGGATTACACCTTCCCACTTGCTGA | 1411 |
| RPN2 | NM_00 2951 | CTGTCTTCCTGTTGGCCCTGACAATCATAGCCAGCACCTGGGCTCT GACGCCCACTCACTACCTCAC | 1412 |
| RPS6KB1 | NM_00 3161 | GCTCATTATGAAAAACATCCCAAACTTTAAAATGCGAAATTATTGGT TGGTGTGAAGAAAGCCAGACAACTTCTGTTTCTT | 1413 |
| RXRA | NM_00 2957 | GCTCTGTTGTGTCCTGTTGCCGGCTCTGGCCTTCCTGTGACTGACT GTGAAGTGGCTTCTCCGTAC | 1414 |
| RXRB | NM_02 1976 | CGAGGAGATGCCTGTGGACAGGATCCTGGAGGCAGAGCTTGCTGT GGAACAGAAGAGTGACCAGGGCGTTG | 1415 |
| S100A10 | NM_00 2966 | ACACCAAAATGCCATCTCAAATGGAACACGCCATGGAAACCATGAT GTTTACATTTCACAAATTCGCTGGGATAAA | 1416 |
| SEC61A | NM_01 3336 | CTTCTGAGCCCGTCTCCCGGACAGGTTGAGGAAGCTGCTCCAGAA GCGCCTCGGAAGGGGAGCTCTC | 1417 |
| SEMA3F | NM_00 4186 | CGCGAGCCCCTCATTATACACTGGGCAGCCTCCCCACAGCGCATC GAGGAATGCGTGCTCTCAGGCAAGGATGTCAACGGCGAGTG | 1418 |
| SFN | NM_00 6142 | GAGAGAGCCAGTCTGATCCAGAAGGCCAAGCTGGCAGAGCAGGC CGAACGCTATGAGGACATGGCAGCCT | 1419 |
| SGCB | NM_00 0232 | CAGTGGAGACCAGTTGGGTAGTGGTGACTGGGTACGCTACAAGCT CTGCATGTGTGCTGATGGGACGCTCTTCAAGG | 1420 |
| SGK | NM_00 5627 | TCCGCAAGACACCTCCTGGAGGGCCTCCTGCAGAAGGACAGGACA AAGCGGCTCGGGGCCAAGGATGACTTCA | 1421 |
| SGKL | NM_17 0709 | TGCATTCGTTGGTTTCTCTTATGCACCTCCTTCAGAAGACTTATTTT TGTGAGCAGTTTGCCATTCAGAAA | 1422 |
| SHC1 | NM_00 3029 | CCAACACCTTCTTGGCTTCTGGGACCTGTGTTCTTGCTGAGCACCC TCTCCGGTTTGGGTTGGGATAACAG | 1423 |
| SIR2 | NM_01 2238 | AGCTGGGGTGTCTGTTTCATGTGGAATACCTGACTTCAGGTCAAGG GATGGTATTTATGCTCGCCTTGCTGT | 1424 |
| SLC1A3 | NM_00 4172 | GTGGGGAGCCCATCATCTCGCCAAGCCATCACAGGCTCTGCATAC ACATGCACTCAGTGTGGACTGG | 1425 |
| SLC25A3 | NM_21 3611 | TCTGCCAGTGCTGAATTCTTTGCTGACATTGCCCTGGCTCCTATGG AAGCTGCTAAGGTTCGAA | 1426 |
| SLC35B1 | NM_00 5827 | CCCAACTCAGGTCCTTGGTAAATCCTGCAAGCCAATCCCAGTCATG CTCCTTGGGGTGACCCTCTTG | 1427 |
| SLC7A11 | NM_01 4331 | AGATGCATACTTGGAAGCACAGTCATATCACACTGGGAGGCAATGC AATGTGGTTACCTGGTCCTAGGTT | 1428 |
| SLC7A5 | NM_00 3486 | GCGCAGAGGCCAGTTAAAGTAGATCACCTCCTCGAACCCACTCCG GTTCCCCGCAACCCACAGCTCAGCT | 1429 |
| SNAI2 | NM_00 3068 | GGCTGGCCAAACATAAGCAGCTGCACTGCGATGCCCAGTCTAGAA AATCTTTCAGCTGTAAATACTGTGACAAGGA | 1430 |
| SNCA | NM_00 7308 | AGTGACAAATGTTGGAGGAGCAGTGGTGACGGGTGTGACAGCAGT AGCCCAGAAGACAGTGGAGGG | 1431 |
| SNCG | NM_00 3087 | ACCCACCATGGATGTCTTCAAGAAGGGCTTCTCCATCGCCAAGGA GGGCGTGGTGGGTGCGGTGGAAAAGACCAAGCAGG | 1432 |
| SOD1 | NM_00 0454 | TGAAGAGAGGCATGTTGGAGACTTGGGCAATGTGACTGCTGACAA AGATGGTGTGGCCGATGTGTCTATT | 1433 |
| SRC | N M_00 5417 | TGAGGAGTGGTATTTGGCAAGATCACCAGACGGAGTCAGAGCG GTTACTGCTCAATGCAGAGAACCCGAGAG | 1434 |
| SRI | NM_00 3130 | ATACAGCACCAATGGAAAGATCACCTTCGACGACTACATCGCCTGC TGCGTCAAACTGAGGGCTCTTACAGACA | 1435 |
| STAT1 | NM_00 7315 | GGGCTCAGCTTTCAGAAGTGCTGAGTTGGCAGTTTTCTTCTGTCAC CAAAAGAGGTCTCAATGTGGACCAGCTGAACATGT | 1436 |
| STAT3 | NM_00 3150 | TCACATGCCACTTTGGTGTTTCATAATCTCCTGGGAGAGATTGACC AGCAGTATAGCCGCTTCCTGCAAG | 1437 |
| STK10 | NM_00 5990 | CAAGAGGGACTCGGACTGCAGCAGCCTCTGCACCTCTGAGAGCAT GGACTATGGTACCAATCTCTCCACTGACCTG | 1438 |
| STK11 | NM_00 0455 | GGACTCGGAGACGCTGTGCAGGAGGGCCGTCAAGATCCTCAAGAA GAAGAAGTTGCGAAGGATCCC | 1439 |
| STK15 | N M_00 3600 | CATCTTCCAGGAGGACCACTCTCTGTGGCACCCTGGACTACCTGC CCCCTGAAATGATTGAAGGTCGGA | 1440 |
| STMN1 | NM_00 5563 | AATACCCAACGCACAAATGACCGCACGTTCTCTGCCCCGTTTCTTG CCCCAGTGTGGTTTGCATTGTCTCC | 1441 |
| STMY3 | NM_00 5940 | CCTGGAGGCTGCAACATACCTCAATCCTGTCCCAGGCCGGATCCT CCTGAAGCCCTTTTCGCAGCACTGCTATCCTCCAAAGCCATTGTA | 1442 |
| SURV | NM_00 1168 | TGTTTTGATTCCCGGGCTTACCAGGTGAGAAGTGAGGAGGAAGA AGGCAGTGTCCCTTTTGCTAGAGCTGACAGCTTTG | 1443 |
| TACC3 | N M_00 6342 | CACCCTTGGACTGGAAAACTCACACCCGGTCTGGACACAGAAAGA GAACCAACAGCTCATCAAGG | 1444 |
| TBCA | NM_00 4607 | GATCCTCGCGTGAGACAGATCAAGATCAAGACCGGCGTGGTGAAG CGGTTGGTCAAAGAAAAAGTG | 1445 |
| TBCC | NM_00 3192 | CTGTTTTCCTGGAGGACTGCAGTGACTGCGTGCTGGCAGTGGCCT GCCAACAGCTCCGCATACACAGT | 1446 |
| TBCD | NM_00 5993 | CAGCCAGGTGTACGAGACATTGCTCACCTACAGTGACGTCGTGGG CGCGGATGTGCTGGACGAGGT | 1447 |
| TBCE | NM_00 3193 | TCCCGAGAGAGGAAAGCATGATGGGAGCCACGAAGGGACTGTGTA TTTTAAATGCAGGCACCCGAC | 1448 |
| TBD | NM_01 6261 | CCTGGTTGAAGCCTGTTAATGCTTTCAACGTGTGGAAAACCCAGCG GGCCTTTAGCAAATATGAGAAGTCTGCA | 1449 |
| TCP1 | NM_03 0752 | CCAGTGTGTGTAACAGGGTCACAAGAATTCGACAGCCAGATGCTC CAAGAGGGTGGCCCAAGGCTATA | 1450 |
| TFRC | NM_00 3234 | GCCAACTGCTTTCATTTGTGAGGGATCTGAACCAATACAGAGCAGA CATAAAGGAAATGGGCCTGAGT | 1451 |
| THBS1 | N M_00 3246 | CATCCGCAAAGTGACTGAAGAGAACAAAGAGTTGGCCAATGAGCT GAGGCGGCCTCCCCTATGCTATCACAACGGAGTTCAGTAC | 1452 |
| TK1 | NM_00 3258 | GCCGGGAAGACCGTAATTGTGGCTGCACTGGATGGGACCTTCCAG AGGAAGCCATTTGGGGCCATCCTGAACCTGGTGCCGCTG | 1453 |
| TOP2A | NM_00 1067 | AATCCAAGGGGGAGAGTGATGACTTCCATATGGACTTTGACTCAGC TGTGGCTCCTCGGGCAAAATCTGTAC | 1454 |
| TOP3B | NM 00 3935 | GTGATGCCTTCCCTGTGGGCGAGGTGAAGATGCTGGAGAAGCAGA CGAACCCACCCGACTACCTGA | 1455 |
| TP | NM_00 1953 | CTATATGCAGCCAGAGATGTGACAGCCACCGTGGACAGCCTGCCA CTCATCACAGCCTCCATTCTCAGTAAGAAACTCGTGG | 1456 |
| TP53BP1 | NM_00 5657 | TGCTGTTGCTGAGTCTGTTGCCAGTCCCCAGAAGACCATGTCTGTG TTGAGCTGTATCTGTGAAGCCAGGCAAG | 1457 |
| TPT1 | NM_00 3295 | GGTGTCGATATTGTCATGAACCATCACCTGCAGGAAACAAGTTTCA CAAAAGAAGCCTACAAGAAGTACATCAAAGATTAC | 1458 |
| TRAG3 | NM_00 4909 | GACGCTGGTCTGGTGAAGATGTCCAGGAAACCACGAGCCTCCAGC CCATTGTCCAACAACCACCCA | 1459 |
| TRAIL | NM_00 3810 | CTTCACAGTGCTCCTGCAGTCTCTCTGTGTGGCTGTAACTTACGTG TACTTTACCAACGAGCTGAAGCAGATG | 1460 |
| TS | NM_00 1071 | GCCTCGGTGTGCCTTTCAACATCGCCAGCTACGCCCTGCTCACGT ACATGATTGCGCACATCACG | 1461 |
| TSPAN4 | NM_00 3271 | CTGGTCAGCCTTCAGGGACCCTGAGCACCGCCTGGTCTCTTTCCT GTGGCCAGCCCAGAACTGAAG | 1462 |
| TTK | NM_00 3318 | TGCTTGTCAGTTGTCAACACCTTATGGCCAACCTGCCTGTTTCCAG CAGCAACAGCATCAAATACTTGCCACTCCA | 1463 |
| TUBA1 | NM_00 6000 | TGTCACCCCGACTCAACGTGAGACGCACCGCCCGGACTCACCATG CGTGAATGCATCTCAGTCCACGT | 1464 |
| TUBA2 | NM_00 6001 | AGCTCAACATGCGTGAGTGTATCTCTATCCACGTGGGGCAGGCAG GAGTCCAGATCGGCAAT | 1465 |
| TUBA3 | NM_00 6009 | CTCTTACATCGACCGCCTAAGAGTCGCGCTGTAAGAAGCAACAACC TCTCCTCTTCGTCTCCGCCATCAGC | 1466 |
| TUBA4 | NM_02 5019 | GAGGAGGGTGAGTTCTCCAAGGCCCATGAGGATATGACTGCCCTG GAGAAGGATTACAAGGAGGTGGGCAT | 1467 |
| TUBA6 | NM_03 2704 | GTCCCTTCGCCTCCTTCACCGCCGCAGACCCCTTCAAGTTCTAGTC ATGCGTGAGTGCATCTCCATCCACG | 1468 |
| TUBA8 | NM_01 8943 | CGCCCTACCTATACCAACCTCAACCGCCTCATCAGTCAGATTGTGT CCTCAATCACTGCTTCTCTCCG | 1469 |
| TUBB | NM_00 1069 | CGAGGACGAGGCTTAAAACTTCTCAGATCAATCGTGCATCCTTAG TGAACTTCTGTTGTCCTCAAGCATGGT | 1470 |
| TUBB classIII | NM_00 6086 | CGCCCTCCTGCAGTATTTATGGCCTCGTCCTCCCCCACCTAGGCCA CGTGTGAGCTGCTCCTGTCTCTGT | 1471 |
| TUBB1 | NM_03 0773 | ACACTGACTGGCATCCTGCTTTCCAGTGCCTGCCAGCCTCCAGAA GAGCCAGGTGCCTGACTAGTACATGGGGAGCTACAGAGC | 1472 |
| TUBB2 | NM_00 6088 | GTGGCCTAGAGCCTTCAGTCACTGGGGAAAGCAGGGAAGCAGTGT GAACTCTTTATTCACTCCCAGCCTG | 1473 |
| TUBB5 | NM_00 6087 | ACAGGCCCCATGCATCCTCCCTGCCTCACTCCCCTCAGCCCCTGC CGACCTTAGCTTATCTGGGAGAGAAACA | 1474 |
| TUBBM | NM_03 2525 | CCCTATGGCCCTGAATGGTGCACTGGTTTAATTGTGTTGGTGTCGG CCCCTCACAAATGCAGCCAAGTCATGTAATTAGT | 1475 |
| TUBBOK | NM_17 8014 | AGTGGAATCCTTCCCTTTCCAACTCTACCTCCCTCACTCAGCTCCTT TCCCCTGATCAGAGAAAGGGATCAAGGG | 1476 |
| TUBBP | NM_17 8012 | GGAAGGAAAGAAGCATGGTCTACTTTAGGTGTGCGCTGGGTCTCT GGTGCTCTTCACTGTTGCCTGTCACTTTTT | 1477 |
| TUBG1 | NM_00 1070 | GATGCCGAGGGAAATCATCACCCTACAGTTGGGCCAGTGCGGCAA TCAGATTGGGTTCGAGTTCTGG | 1478 |
| TWIST1 | NM_00 0474 | GCGCTGCGGAAGATCATCCCCACGCTGCCCTCGGACAAGCTGAGC AAGATTCAGACCCTCAAGC | 1479 |
| TYRO3 | NM_00 6293 | CAGTGTGGAGGGGATGGAGGAGCCTGACATCCAGTGGGTGAAGG ATGGGGCTGTGGTCCAGAACTTG | 1480 |
| UFM1 | NM_01 6617 | AGTTGTCGTGTGTTCTGGATTCATTCCGGCACCACCATGTCGAAGG TTTCCTTTAAGATCACGCTGACG | 1481 |
| upa | NM_00 2658 | GTGGATGTGCCCTGAAGGACAAGCCAGGCGTCTACACGAGAGTCT CACACTTCTTACCCTGGATCCGCAG | 1482 |
| VCAM1 | NM_00 1078 | TGGCTTCAGGAGCTGAATACCCTCCCAGGCACACACAGGTGGGAC ACAAATAAGGGTTTTGGAACCACTATTTCTCATCACGACAGCA | 1483 |
| VEGF | NM_00 3376 | CTGCTGTCTTGGGTGCATTGGAGCCTTGCCTTGCTGCTCTACCTCC ACCATGCCAAGTGGTCCCAGGCTGC | 1484 |
| VEGFB | NM_00 3377 | TGACGATGGCCTGGAGTGTGTGCCCACTGGGCAGCACCAAGTCCG GATGCAGATCCTCATGATCCGGTACC | 1485 |
| VEGFC | NM_00 5429 | CCTCAGCAAGACGTTATTTGAAATTACAGTGCCTCTCTCTCAAGGC CCCAAACCAGTAACAATCAGTTTTGCCAATCACACTT | 1486 |
| VHL | NM_00 0551 | CGGTTGGTGACTTGTCTGCCTCCTGCTTTGGGAAGACTGAGGCAT CCGTGAGGCAGGGACAAGTCTT | 1487 |
| VIM | NM_00 3380 | TGCCCTTAAAGGAACCAATGAGTCCCTGGAACGCCAGATGCGTGA AATGGAAGAGAACTTTGCCGTTGAAGC | 1488 |
| V-RAF | NM_00 1654 | GGTTGTGCTCTACGAGCTTATGACTGGCTCACTGCCTTACAGCCAC ATTGGCTGCCGTGACCAGATTATCTTTATGGTGGGCCG | 1489 |
| WAVE3 | NM_00 6646 | CTCTCCAGTGTGGGCACCAGCCGGCCAGAACAGATGCGAGCAGTC CATGACTCTGGGAGCTACACCGC | 1490 |
| Wnt-5a | NM_00 3392 | GTATCAGGACCACATGCAGTACATCGGAGAAGGCGCGAAGACAGG CATCAAAGAATGCCAGTATCAATTCCGACA | 1491 |
| XIAP | NM_00 1167 | GCAGTTGGAAGACACAGGAAAGTATCCCCAAATTGCAGATTTATCA ACGGCTTTTATCTTGAAAATAGTGCCACGCA | 1492 |
| XIST | NR_00 1564 | CAGGTCAGGCAGAGGAAGTCATGTGCATTGCATGAGCTAAACCTAT CTGAATGAATTGATTTGGGGCTTGTTAGG | 1493 |
| ZW10 | NM_00 4724 | TGGTCAGATGCTGCTGAAGTATATCCTTAGGCCGCTGGCATCTTGC CCATCCCTTCATGCTGTGAT | 1494 |
| ZWILCH | NM_01 7975 | GAGGGAGCAGACAGTGGGTACCACGATCTCCGTAACCATTTGCAT GTGACTTAGCAAGGGCTCTGA | 1495 |
| ZWINT | NM_00 7057 | TAGAGGCCATCAAAATTGGCCTCACCAAGGCCCTGACTCAGATGG AGGAAGCCCAGAGGAAACGGA | 1496 |

## Claims

1. A method of predicting the clinical outcome for a human patient receiving anthracycline - based chemotherapy for breast cancer, the method comprising:
a. assaying an expression level of a MYBL2 RNA transcript, or its expression product, in a biological s ample comprising breast cancer cells obtained from the human patient; and
b. determining a normalized expression level of the MYBL2 RNA transcript, or its expression product;
wherein the normalized expression level of the MYBL2 RNA transcript, or its expression product, correlates with a decreased likelihood of a positive clinical outcome.

2. The method of claim 1, wherein the biological sample is fixed, paraffin-embedded, fresh, or frozen.

3. The method of claim 1, wherein the biological sample is a biopsy specim en.

4. The method of claim 1, wherein the assaying is done by reverse transcriptase polymerase chain reaction (RT -PCR) or another PCR -based method.

5. The method of claim 1, wherein the assaying is done by immunohistochemistry.

6. The method of claim 1, wherein the anthracycline is doxorubicin.

7. The method of claim 1, wherein the anthracycline-based chemotherapy further co mprises the administration of one or more additional anti-cancer agent.

8. The method of claim 7, wherein the one or more additional anti -cancer agent is a cyclophosphamide.

9. The method of claim 7, wherein the one or more additional anti -cancer agent is a taxane.

10. The method of claim 7, wherein the one or more additional anti -cancer agent is a cyclophosphamide and a taxane.

11. The method of claim 9 or 10, wherein the taxane is docetaxel.

12. The method of claim 1, further comprising creating a report indicating the likelihood of a positive outcome for the human patient to the anthracycline -based chemotherapy.

13. The method of claim 1, wherein the human patient has a hormone receptor positive (HR+) breast cancer.

14. The method of claim 1, wherein the human patient has a hormone receptor negative (HR - ) breast cancer.
